# EUROPEAN PATENT APPLICATION

(11) **EP 2 009 020 A2**
(43) Date of publication of application: **31.12.2008**
(21) Application number: 08166553.1
(22) Date of filing: 06.06.2006
(51) Int. Cl.: C07K 14/415, C12N 15/82

(54) **Means and methods to modulate flavonoid biosynthesis in plants and plant cells**

(30) Priority: 03.06.2005 EP 05104855
(62) Divisional of application: 06763536.7
(71) Applicant: VIB VZW, 9052 Gent (BE); Universiteit Gent, 9000 Gent (BE)
(72) Inventor: Van Breusegem, Frank, 9660 Brakel (BE); Vanderauwera, Sandy, 1790 Affligem (BE)

(57) **Abstract**

The present invention provides a method for increasing the flavonoid content of plants and plant cells wherein said method comprises increasing the activity of genes implicated in the flavonoid biosynthesis pathway. The invention further relates to recombinant plant and plant cells obtainable by the process of the invention and to flavonoids made there from.

## Description

### Field of the invention

The present invention provides a method for increasing the flavonoid content of plants and plant cells wherein said method comprises increasing the activity of genes implicated in the flavonoid biosynthesis pathway. The invention further relates to recombinant plant and plant cells obtainable by the process of the invention and to flavonoids made there from.

### Background of the invention

Flavonoids are found to be ubiquitous in all vascular plants, in various parts like flowers, fruits, vegetables and seeds. These secondary metabolites form a large family of low molecular weight polyphenolic compounds and may be found under five separate headings: 1) the anthocyanins and anthochlors, which are red-to-blue and yellow flower pigments, respectively; 2) the minor flavonoids, which include flavanones, dihydro-flavonols and dihydrochalcones; 3) the flavones and flavonols, the most widely occurring and structurally variable flavonoids; 4) the isoflavonoids, a distinctive class found mainly in one plant family, the Leguminosae; and 5) the tannins, which are characterised by their affinity to bind with protein. Among the tannins are both flavonoids (the proanthocyanidins or flavolans) and simpler phenolics based on gallic acid (the gallo- and ellagi-tannins). More than 4000 flavonoids have been described, most are conjugated to sugar molecules and are commonly located in the upper epidermal layers of leaves. Reports in the prior art show that there is increasing evidence that flavonoids are potentially health-protecting components in the human diet. Indeed, several epidemiological studies suggest a direct relationship between cardioprotection and increased consumption of flavonoids, in particular flavonols of the quercetin and kaempferol type, from dietary sources such as onion, apples and tea. Flavonoids have also been reported to exhibit a wide range of biological activities *in vitro* including anti-inflammatory, anti-allergic and vasodilatory activity. Such activity has been attributed in part to their ability to act as antioxidants, capable of scavenging free radicals and preventing free radical production. Within this group of compounds, those having the most potent antioxidant activity are the flavonols. In addition, flavonoids can also inhibit the activity of key processes such as lipid peroxidation, platelet aggregation and capillary permeability. Flavanones and their glycosides are also considered important determinants of taste. For example, in contrast to many other fruit, the genus Citrus is characterised by a substantial accumulation of flavanone glycosides. It is noteworthy that in grapefruit the sour taste results mainly from the accumulation of the bitter flavanone glycoside, naringin. Another issue is that certain flavonoids have the ability to inhibit phytopathogens in several plant species. Flavonoid levels can also be manipulated in order to select particular flower colours and patterns. Moreover, increased amounts of condensed tannins in certain forage crops are useful for decreasing bloat in cattle, improving ruminal protein bypass, reducing intestinal parasites, and reducing sileage degradation by proteolysis. From the above it is clear that it would be desirable to produce plants and plant cell cultures which intrinsically posses, elevated levels of flavonoids. Health protecting compounds can for example be produced in plant cell cultures and isolated in pure compounds by extraction and purification. Although it is clear that the flavonoid biosynthetic pathway has been widely studied in a number of different plant species there are still many key genes unknown. The present invention has identified a transcriptional regulon of 69 genes, which are involved in the synthesis of flavonoids, more particularly anthocyanins. These genes can be used to modulate the levels flavonoids of in plants and plant cells.

### Aims and detailed description of the invention

The flavonoids comprise an astonishingly diverse and valuable group of more than 4500 known compounds. Among their subclasses are the anthocyanins (pigments), proanthocyanidins or condensed tannins (feeding deterrents and wood protectants), and isoflavonoids (defensive products and signaling molecules). The present invention has identified a transcriptional regulon of 69 genes, which can be used to modulate the production of flavonoids in plants and plant cells. Accordingly the invention provides in a first embodiment the use of polynucleotides consisting from the list SEQ ID NO: 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68 and/or 69 or fragments or homologues thereof to modulate the biosynthesis of flavonoids in plants or plant cells.

In yet another embodiment said polynucleotides consisting from the list SEQ ID NO: 1-69 are used to modulate the biosynthesis of anthocyanins.

In yet another embodiment the invention provides a recombinant DNA vector comprising at least one of the polynucleotide sequences selected from SEQ ID NO: 1-69.

In yet another embodiment the invention provides a transgenic plant of plant cell that is transformed with a recombinant DNA vector comprising at least one of the polynucleotide sequences selected from SEQ ID NO: 1-69.

As used herein, the word "polynucleotide" may be interpreted to mean the DNA and cDNA sequence as detailed by Yoshikai et al. (1990) Gene 87:257, with or without a promoter DNA sequence as described by Salbaum et al. (1988) EMBO J. 7(9):2807.

As used herein, "fragment" refers to a polypeptide or polynucleotide of at least about 9 amino acids or 27 base pairs, typically 50 to 75, or more amino acids or base pairs, wherein the polypeptide contains an amino acid core sequence. If desired, the fragment may be fused at either terminus to additional amino acids or base pairs, which may number from 1 to 20, typically 50 to 100, but up to 250 to 500 or more. A "functional fragment" means a polypeptide fragment possessing the biological property able to modulate the production of at least one flavonoid in an organism or cell derived thereof. In a particular embodiment said functional fragment is able to modulate the production of at least one flavonoid in a plant or plant cell derived thereof. The term 'production' includes intracellular production and secretion into the medium. The term 'modulates or modulation' refers to an increase or a decrease. Often an increase of at least one flavonoid is desired but sometimes a decrease of at least one flavonoid is wanted. Said decrease can for example refer to the decrease of an undesired intermediate product of at least one flavonoid. With an increase in the production of one or more metabolites it is understood that said production may be enhanced by at least 20%, 30%, 40%, 50%, 60%, 70%, 80%, 90% or at least 100% relative to the untransformed plant or plant cell which was used to transform with an expression vector comprising an expression cassette further comprising at least one polynucleotide or homologue or variant or fragment thereof of the invention. Conversely, a decrease in the production of the level of one or more flavonoids may be decreased by at least 20%, 30%, 40%, 50%, 60%, 70%, 80%, 90% or at least 100% relative to the untransformed plant or plant cell which was used to transform with an expression vector comprising an expression cassette further comprising at least one polynucleotide or homologue or variant or fragment thereof of the invention. The terms 'identical' or percent 'identity' in the context of two or more nucleic acids or polypeptide sequences, refer to two or more sequences or subsequences that are the same or have a specified percentage of amino acid residues or nucleotides that are the same (i.e. 70% identity over a specified region), when compared and aligned for maximum correspondence over a comparison window, or designated region as measured using sequence comparison algorithms or by manual alignment and visual inspection. Preferably, the identity exists over a region that is at least about 25 amino acids or nucleotides in length, or more preferably over a region that is 50-100 amino acids or nucleotides or even more in length. Examples of useful algorithms are PILEUP (Higgins & Sharp, CABIOS 5:151 (1989), BLAST and BLAST 2.0 (Altschul et al. J. Mol. Biol. 215: 403 (1990). Software for performing BLAST analyses is publicly available through the National Center for Biotechnology Information (http://www/ncbi.nlm.nih.gov/). In the present invention the term 'homologue' also refers to 'identity'. For example a homologue of SEQ ID NO: 1-69 has at least 60% identity to one of these sequences. According to still further features in the described preferred embodiments the polynucleotide fragment encodes a polypeptide able to modulate the flavonoid biosynthesis, which may therefore be allelic, species and/or induced variant of the amino acid sequence set forth in SEQ ID NO: 1-69. It is understood that any such variant may also be considered a homologue. The present invention accordingly provides in another embodiment a method for modulating the production of at least one flavonoid in plant or plant cells, by transformation of said plant or plant cells with an expression vector comprising an expression cassette that further comprises at least one gene comprising a fragment, variant or homologue encoded by at least one sequence selected from SEQ ID NO: 1-69.

In another embodiment the invention provides a recombinant DNA vector comprising at least one polynucleotide sequence, homologue, fragment or variant selected from at least one of the sequences comprising SEQ ID NO: 1-69. The vector may be of any suitable type including, but not limited to, a phage, virus, plasmid, phagemid, cosmid, bacmid or even an artificial chromosome. The at least one polynucleotide sequence preferably codes for at least one polypeptide that is involved in the biosynthesis and/or regulation of synthesis of at least one flavonoid (e.g. a transcription factor, a repressor, an enzyme that regulates a feed-back loop, a transporter, a chaperone). The term "recombinant DNA vector" as used herein refers to DNA sequences containing a desired coding sequence and appropriate DNA sequences necessary for the expression of the operably linked coding polynucleotide sequence in a particular host organism (e.g. plant cell). Plant cells are known to utilize promoters, polyadenylation signals and enhancers.

In yet another embodiment the invention provides a transgenic plant or derived cell thereof transformed with said recombinant DNA vector.

A recombinant DNA vector comprises at least one "Expression cassette". Expression cassettes are generally DNA constructs preferably including (5' to 3' in the direction of transcription): a promoter region, a polynucleotide sequence, homologue, variant or fragment thereof of the present invention operatively linked with the transcription initiation region, and a termination sequence including a stop signal for RNA polymerase and a polyadenylation signal. It is understood that all of these regions should be capable of operating in biological cells, such as plant cells, to be transformed. The promoter region comprising the transcription initiation region, which preferably includes the RNA polymerase binding site, and the polyadenylation signal may be native to the biological cell to be transformed or may be derived from an alternative source, where the region is functional in the biological cell.

The polynucleotide sequence, homologue, variant or fragment thereof of the invention may be expressed in for example a plant cell under the control of a promoter that directs constitutive expression or regulated expression. Regulated expression comprises temporally or spatially regulated expression and any other form of inducible or repressible expression. Temporally means that the expression is induced at a certain time point, for instance, when a certain growth rate of the plant cell culture is obtained (e.g. the promoter is induced only in the stationary phase or at a certain stage of development). Spatially means that the promoter is only active in specific organs, tissues, or cells (e.g. only in roots, leaves, epidermis, guard cells or the like). Other examples of regulated expression comprise promoters whose activity is induced or repressed by adding chemical or physical stimuli to the plant cell. In a preferred embodiment the expression is under control of environmental, hormonal, chemical, and/or developmental signals. Such promoters for plant cells include promoters that are regulated by (1) heat, (2) light, (3) hormones, such as abscisic acid and methyl jasmonate (4) wounding or (5) chemicals such as salicylic acid, chitosans or metals. Indeed, it is well known that the expression of secondary metabolites (such as flavonoids) can be boosted by the addition of for example specific chemicals, jasmonate and elicitors. In a particular embodiment the co-expression of several (more than one) polynucleotide sequence or homologue or variant or fragment thereof, in combination with the induction of secondary metabolite synthesis is beneficial for an optimal and enhanced production of flavonoids. Alternatively, the at least one polynucleotide sequence, homologue, variant or fragment thereof is placed under the control of a constitutive promoter. A constitutive promoter directs expression in a wide range of cells under a wide range of conditions. Examples of constitutive plant promoters useful for expressing heterologous polypeptides in plant cells include, but are not limited to, the cauliflower mosaic virus (CaMV) 35S promoter, which confers constitutive, high-level expression in most plant tissues including monocots; the nopaline synthase promoter and the octopine synthase promoter. The expression cassette is usually provided in a DNA or RNA construct which is typically called an "expression vector" which is any genetic element, e.g., a plasmid, a chromosome, a virus, behaving either as an autonomous unit of polynucleotide replication within a cell (i.e. capable of replication under its own control) or being rendered capable of replication by insertion into a host cell chromosome, having attached to it another polynucleotide segment, so as to bring about the replication and/or expression of the attached segment. Suitable vectors include, but are not limited to, plasmids, bacteriophages, cosmids, plant viruses and artificial chromosomes. The expression cassette may be provided in a DNA construct which also has at least one replication system. In addition to the replication system, there will frequently be at least one marker present, which may be useful in one or more hosts, or different markers for individual hosts. The markers may a) code for protection against a biocide, such as antibiotics, toxins, heavy metals, certain sugars or the like; b) provide complementation, by imparting prototrophy to an auxotrophic host: or c) provide a visible phenotype through the production of a novel compound in the plant. Exemplary genes, which may be employed, include neomycin phosphotransferase (NPTII), hygromycin phosphotransferase (HPT), chloramphenicol acetyltransferase (CAT), nitrilase, and the gentamicin resistance gene. For plant host selection, non-limiting examples of suitable markers are β-glucuronidase, providing indigo production, luciferase, providing visible light production, Green Fluorescent Protein and variants thereof, NPTII, providing kanamycin resistance or G418 resistance, HPT, providing hygromycin resistance, and the mutated aroA gene, providing glyphosate resistance.

The term "promoter activity" refers to the extent of transcription of a polynucleotide sequence, homologue, variant or fragment thereof that is operably linked to the promoter whose promoter activity is being measured. The promoter activity may be measured directly by measuring the amount of RNA transcript produced, for example by Northern blot or indirectly by measuring the product coded for by the RNA transcript, such as when a reporter gene is linked to the promoter. The term "operably linked" refers to linkage of a DNA segment to another DNA segment in such a way as to allow the segments to function in their intended manners. A DNA sequence encoding a gene product is operably linked to a regulatory sequence when it is ligated to the regulatory sequence, such as, for example a promoter, in a manner, which allows modulation of transcription of the DNA sequence, directly or indirectly. For example, a DNA sequence is operably linked to a promoter when it is ligated to the promoter downstream with respect to the transcription initiation site of the promoter and allows transcription elongation to proceed through the DNA sequence. A DNA for a signal sequence is operably linked to DNA coding for a polypeptide if it is expressed as a pre-protein that participates in the transport of the polypeptide. Linkage of DNA sequences to regulatory sequences is typically accomplished by ligation at suitable restriction sites or adapters or linkers inserted in lieu thereof using restriction endonucleases known to one of skill in the art.

In a particular embodiment the polynucleotides or homologues or variants or fragments thereof of the present invention can be introduced in plants or plant cells that are different from Arabidopsis and said polynucleotides can be used for the modulation of flavonoid synthesis in plants or plant cells.

The term "heterologous DNA" and or "heterologous RNA" refers to DNA or RNA that does not occur naturally as part of the genome or DNA or RNA sequence in which it is present, or that is found in a cell or location in the genome or DNA or RNA sequence that differs from that which is found in nature. Heterologous DNA and RNA (in contrast to homologous DNA and RNA) are not endogenous to the cell into which it is introduced, but has been obtained from another cell or synthetically or recombinantly produced. An example is a gene isolated from one plant species operably linked to a promoter isolated from another plant species. Generally, though not necessarily, such DNA encodes RNA and proteins that are not normally produced by the cell in which the DNA is transcribed or expressed. Similarly exogenous RNA encodes for proteins not normally expressed in the cell in which the exogenous RNA is present. Heterologous DNA or RNA may also refer to as foreign DNA or RNA. Any DNA or RNA that one of skill in the art would recognize as heterologous or foreign to the cell in which it is expressed is herein encompassed by the term heterologous DNA or heterologous RNA. Examples of heterologous DNA include, but are not limited to, DNA that encodes proteins, polypeptides, receptors, reporter genes, transcriptional and translational regulatory sequences, selectable or traceable marker proteins, such as a protein that confers drug resistance, RNA including mRNA and antisense RNA and ribozymes.

Accordingly, the invention provides in a further aspect a gene construct in the form of an expression cassette comprising as operably linked components in the 5'-3' direction of transcription, one or more units each comprising a suitable promoter in a plant cell, a plurality of nucleotide sequences selected from the group consisting of sequences SEQ ID NO: 1-69 for flavonoid biosynthesis and a suitable transcriptional and translational termination regulatory region.

The promoter and termination regulatory regions will be functional in the host plant cell and may be heterologous or homologous to the plant cell and the gene. Suitable promoters, which may be used, are described above.

The termination regulatory region may be derived from the 3' region of the gene from which the promoter was obtained or from another gene. Suitable termination regions, which may be used, are well known in the art and include *Agrobacterium tumefaciens* nopaline synthase terminator (Tnos), *Agrobacterium tumefaciens* mannopine synthase terminator (Tmas), the rubisco small subunit terminator (TrbcS) and the Ca 35S terminator (T35S).

The present invention can be practiced with any plant variety for which cells of the plant can be transformed with an expression cassette of the current invention and for which transformed cells can be cultured *in vitro.* Suspension culture, callus culture, hairy root culture, shoot culture or other conventional plant cell culture methods may be used (as described in: Drugs of Natural Origin, G. Samuelsson, 1999, ISBN 9186274813).

By "plant cells" it is understood any cell which is derived from a plant and can be subsequently propagated as callus, plant cells in suspension, organized tissue and organs (e.g. hairy roots). Tissue cultures derived from the plant tissue of interest can be established. Methods for establishing and maintaining plant tissue cultures are well known in the art (see, e.g. Trigiano R.N. and Gray D.J. (1999), "Plant Tissue Culture Concepts and Laboratory Exercises", ISBN: 0-8493-2029-1; Herman E.B. (2000), "Regeneration and Micropropagation: Techniques, Systems and Media 1997-1999", Agricell Report). Typically, the plant material is surface-sterilized prior to introducing it to the culture medium. Any conventional sterilization technique, such as chlorinated bleach treatment can be used. In addition, antimicrobial agents may be included in the growth medium. Under appropriate conditions plant tissue cells form callus tissue, which may be grown either as solid tissue on solidified medium or as a cell suspension in a liquid medium.

A number of suitable culture media for callus induction and subsequent growth on aqueous or solidified media are known. Exemplary media include standard growth media, many of which are commercially available (e.g., Sigma Chemical Co., St. Louis, Mo.). Examples include Schenk-Hildebrandt (SH) medium, Linsmaier-Skoog (LS) medium, Murashige and Skoog (MS) medium, Gamborg's B5 medium, Nitsch & Nitsch medium, White's medium, and other variations and supplements well known to those of skill in the art (see, e.g., Plant Cell Culture, Dixon, ed. IRL Press, Ltd. Oxford (1985) and George et al., Plant Culture Media, Vol 1, Formulations and Uses Exegetics Ltd. Wilts, UK, (1987)). For the growth of conifer cells, particularly suitable media include 1/2 MS, 1/2 L.P., DCR, Woody Plant Medium (WPM), Gamborg's B5 and its modifications, DV (Durzan and Ventimiglia, In Vitro Cell Dev. Biol. 30:219-227 (1994)), SH, and White's medium.

In a particular embodiment the current invention can be combined with other known methods to enhance the production and/or the secretion of flavonoids in plant cell cultures such as (1) by improvement of the plant cell culture conditions, (2) by the transformation of the plant cells with a transcription factor capable to induce genes involved in the pathway of flavonoid formation, (3) by the addition of specific elicitors to the plant cell culture, and 4) by the induction of organogenesis.

The term "plant" as used herein refers to vascular plants (e.g. gymnosperms and angiosperms). The method comprises transforming a plant cell with an expression cassette of the present invention and regenerating such plant cell into a transgenic plant. Such plants can be propagated vegetatively or reproductively. The transforming step may be carried out by any suitable means, including by Agrobacterium-mediated transformation and *non-Agrobacterium-*mediated transformation, as discussed in detail below. Plants can be regenerated from the transformed cell (or cells) by techniques known to those skilled in the art. Where chimeric plants are produced by the process, plants in which all cells are transformed may be regenerated from chimeric plants having transformed germ cells, as is known in the art. Methods that can be used to transform plant cells or tissue with expression vectors of the present invention include both *Agrobacterium* and *non-Agrobacterium* vectors. *Agrobacterium-*mediated gene transfer exploits the natural ability of *Agrobacterium tumefaciens* to transfer DNA into plant chromosomes and is described in detail in Gheysen, G., Angenon, G. and Van Montagu, M. 1998. Agrobacterium-mediated plant transformation: a scientifically intriguing story with significant applications. In K. Lindsey (Ed.), Transgenic Plant Research. Harwood Academic Publishers, Amsterdam, pp. 1-33 and in Stafford, H.A. (2000) Botanical Review 66: 99-118. A second group of transformation methods is the *non-Agrobacterium* mediated transformation and these methods are known as direct gene transfer methods. An overview is brought by Barcelo, P. and Lazzeri, P.A. (1998) Direct gene transfer: chemical, electrical and physical methods. In K. Lindsey (Ed.), Transgenic Plant Research, Harwood Academic Publishers, Amsterdam, pp.35-55. Hairy root cultures can be obtained by transformation with virulent strains of *Agrobacterium* rhizogenes, and they can produce high contents of secondary metabolites characteristic to the mother plant. Protocols used for establishing of hairy root cultures vary, as well as the susceptibility of plant species to infection by *Agrobacterium* (Toivounen L. (1993) Biotechnol. Prog. 9, 12; Vanhala L. et al. (1995) Plant Cell Rep. 14, 236). It is known that the *Agrobacterium* strain used for transformation has a great influence on root morphology and the degree of secondary metabolite accumulation in hairy root cultures. It is possible that by systematic clone selection e.g. via protoplasts, to find high yielding, stable, and from single cell derived-hairy root clones. This is possible because the hairy root cultures possess a great somaclonal variation. Another possibility of transformation is the use of viral vectors (Turpen TH (1999) Philos Trans R Soc Lond B Biol Sci 354(1383): 665-73).

Any plant tissue or plant cells capable of subsequent clonal propagation, whether by organogenesis or embryogenesis, may be transformed with an expression vector of the present invention. The term 'organogenesis' means a process by which shoots and roots are developed sequentially from meristematic centers; the term 'embryogenesis' means a process by which shoots and roots develop together in a concerted fashion (not sequentially), whether from somatic cells or gametes. The particular tissue chosen will vary depending on the clonal propagation systems available for, and best suited to, the particular species being transformed. Exemplary tissue targets include protoplasts, leaf disks, pollen, embryos, cotyledons, hypocotyls, megagametophytes, callus tissue, existing meristematic tissue (e.g. apical meristems, axillary buds, and root meristems), and induced meristem tissue (e.g., cotyledon meristem and hypocotyls meristem).

These plants may include, but not limited to, plants or plant cells of agronomically important crops, such as plants from the Pisum family such as peas, family of Brassicae, such as green cabbage, Brussel sprouts, cauliflower, the family of Phaseolus such as barlotti beans, green beans, kidney beans, the family of Spinacea such as spinach, the family of Solanaceae such as potato and tomato, the family of Daucus, such as carrots, family of Capsicum such as green and red pepper, and the family of Ribesiaceae such as strawberries, blackberries, raspberries, black currant and edible grasses from the family of Gramineae such as maize, and citrus fruit for example from the family of Rutaceae such as lemon, orange, tangerine, or from the apple family. Also preferred are oil producing plants such as sunflower, soybean and rape. Also preferred are plants which can form the basis of an infusion such as black tea leaves, green tea leaves, jasmine tea leaves. It is also understood that the invention may be applied to plants that produce valuable compounds. Examples of such plants include, but not limited to, *Papaver spp., Rauwolfia spp., Taxus spp., Cinchona spp., Eschscholtzia californica, Camptotheca acuminata, Hyoscyamus spp., Berberis spp., Coptis spp., Datura spp., Atropa spp., Thalictrum spp., Peganum spp.*

It may well be that increase in flavonoid content observed in plants modified according to the invention comprises an increase in a plurality of different flavonoid types depending on the nature of the plant tissue in which modified gene expression is occurring.

In yet another embodiment suitable expression cassettes comprising the nucleotide sequences of the present invention can be used for transformation into other species (different from Arabidopsis). This transformation into other species or genera can be carried out randomly or can be carried out with strategically chosen nucleotide sequences. The random combination of genetic material from one or more species of organisms can lead to the generation of novel metabolic pathways (for example through the interaction with metabolic pathways resident in the host organism or alternatively silent metabolic pathways can be unmasked) and eventually lead to the production of novel classes of compounds. This novel or reconstituted metabolic pathways can have utility in the commercial production of novel, valuable flavonoids.

Various assays within the knowledge of the person skilled in the art may be used to determine whether the plant cell shows an increase in gene expression, for example, Northern blotting or quantitative reverse transcriptase PCR (RT-PCR). Whole transgenic plants may be regenerated from the transformed cell by conventional methods. Such transgenic plants having improved flavonoid levels may be propagated and crossed to produce homozygous lines. Such plants produce seeds containing the genes for the introduced trait and can be grown to produce plants that will produce the selected phenotype.

The recombinant DNA and molecular cloning techniques applied in the below examples are all standard methods well known in the art and are e.g. described by Sambrook et al. (1989) Molecular cloning: A laboratory manual, second edition, Cold Spring Harbor Laboratory Press. Methods for tobacco cell culture and manipulation applied in the below examples are methods described in or derived from methods described in Nagata et al. (1992) Int. Rev. Cytol. 132, 1.

### Examples

### 1. Identification of 69 genes involved in flavonoid biosynthesis

Genome-wide analysis of photorespiratory hydrogen peroxide regulated gene expression in Arabidopsis reveals a high light induced transcriptional regulon involved in anthocyanin biosynthesis.

By using ATH1 Affymetrix microarrays, expression profiles were compared between control and catalase-deficient *Arabidopsis thaliana* plants. Reduced catalase levels already provoked differences in nuclear gene expression under ambient growth conditions and these effects are amplified by high light exposure in a sun simulator for 3 and 8 h. Genome-wide expression analysis allowed the characterization of complete pathways and functional categories during H₂O₂ stress. In addition by analyzing transcriptome data sets obtained from a combination of different perturbations it becomes possible to identify more robustly co-regulated genes over a wide range of stresses, which are to be part of the same regulon and, therefore, to be considered as "brothers in arms" within the studied biological process. From such a "guilt by association" analysis the function of hitherto unknown genes can be predicted with more certainty. Through the analysis of transcriptomic changes provoked by photorespiratory H₂O₂, a transcriptional regulon of genes associated with anthocyanin biosynthesis was identified. In addition to the genes known to be involved in anthocyanin biosynthesis, several unknown genes that can be put forward as potential candidates for a function within the production of anthocyanins in leaves.

The 1495 differentially expressed genes with CV > 2 were subjected to hierarchical average linkage clustering. Different prominent clusters of transcriptional changes stand out clearly: cluster A (484 genes) represents mainly genotype-independent HL-repressed genes; cluster B groups 437 genes that are exclusively induced by HL in the CAT2HP1 plants; cluster C contains 111 genes that are repressed in the CAT2HP1 plants and cluster D (463 genes) comprises mainly (genotype independent) HL-induced genes.

As an alternative for a CV analysis, genes were classified according to their fold change in expression. Therefore, the threshold for positive response was set at threefold change in expression. The expression of 906 genes was affected by HL itself. Of the 906 exclusively HL differentially regulated genes, 379 were upregulated and 527 were downregulated. Screening for differentially expressed genes in response to photorespiratory H₂O₂, revealed 349 and 88 H₂O₂-induced or H₂O₂-repressed genes, respectively. In our analysis, HL drives after 3 h the upregulation of nearly 380 genes in control plants. When assessing the expression profiles of these genes in both HL-exposed CAT2HP1 and control plants, a clear subcluster could be recognized in which the induction by HL was significantly delayed in the CAT2HP1 plants. Whereas in control plants transcripts levels increased rapidly within 3 h of HL, they only reached their highest expression levels after 8 h in the CAT2HP1 plants. Within this subcluster, genes known to be involved in the regulation, biosynthesis and sequestration of anthocyanins were predominantly present. To enable a more robust identification of other genes in the regulon, we selected all genes whose expression levels were at least threefold induced after 3 h or 8h of HL stress, but had at least a 1.5-fold lower expression in the CAT2HP1 compared to control plants. The expression characteristics of 176 genes matched these criteria. To further validate the robustness of the selected genes, we assessed their expression during leaf senescence. Senescence is a well-characterized process in which anthocyanin levels are upregulated (Hoch WA et al (2001) Tree Physiol. 21, 1-8). Expression profiles of the 176 genes during the HL treatment and their behavior during senescence were clustered together and resulted unexpectedly in a major division into two prominent clusters. Cluster B (105 genes) grouped genes involved in the anthocyanin biosynthesis and regulation together with 69 genes previously not associated with anthocyanin biosynthesis and/or regulation.

### 2. Functional analysis of the genes

Full-length cDNAs were PCR-amplified with gene-specific primers from cDNA obtained from Col-0 Arabidopsis plants and cloned into the GateWay destination vector pB7WG2D, which is a binary vector for overexpression in plants (Karimi et al. (2002) Trends Plant Sci. 7(5):193-5). Constructs were transformed into Arabidopsis thaliana Col-0 plants through *Agrobacterium-*mediated floral dip transformation (Clough and Bent (1998) Plant J. 16(6):735-43). Primary transformants were selected through resistance to basta resistance and were selfed. Progeny plants were assessed for transgene overexpression through RT-PCR, Northern blot analysis or Western blot analysis and segregation analysis was performed to identify lines with single T-DNA locus. Selected lines were subjected to a phenotypic analysis (visual scoring for increased coloration) and biochemical analysis (determination of anthocyanins via methanol-extraction or HPLC analysis) under ambient and high light conditions (1000 µmol m⁻² sec⁻¹).

### 3. Anthocyanin measurements

Plants were grown on MS medium for 14 days and exposed to continuous HL irradiation (approximately 1000 µmol m⁻² sec⁻¹) for 23 h. Fresh weight was recorded for each sample, and ranged from 0.099 to 0.185 g per sample. Samples were frozen in liquid nitrogen en ground with mortar and pestle. Anthocyanins were measured according to a procedure based on the methods of Rabino and Mancinelli (1986), and Feinbaum and Ausubel (1988). Total plant pigments were extracted in 0.75 ml of 1% HCl/methanol, and 0.5 ml of distilled H₂O was added. Chlorophyll was separated from the anthocyanins by back-extraction with chloroform. The quantity of anthocyanin pigments was determined by spectrophotometric measurements of the aqueous/methanol phase. The absorbance at 530 nm minus the absorbance at 657 nm was used as a measure of anthocyanin content, and values were normalized to the fresh weight of each sample. Results are expressed as absorbance per g FW. The results of some transgenic lines are presented in Table 1.

### 4. Analysis of anthocyanins from Arabidopsis thaliana via HPLC

### Sample preparation

Leaves are harvested, freeze-dried and gently grinded into a rough powder. Approx. 100±10 mg of sample is weighed exactly into a test tube and extracted with 2 ml of MeOH for 1 hour using magnetic stirrer (700 rpm). The tube is centrifuged (10 min, 3000 rpm) and the supernatant is collected. Extraction procedure is repeated with 30 minutes extraction time and the supernatants are combined. The extract is then filtrated through a 0.45 µm syringe filter. Chlorophyll is removed from the extract to avoid interference in the analysis of the anthocyanins by adding water (half the volume of the extract) and petroleum ether (1:1 with the MeOH-H₂O solution) and vortexing for 5 seconds. After 15 minutes the petroleum ether fraction containing chlorophyll is removed. The petroleum ether extraction is repeated three times. The remaining extract is evaporated to dryness. The dry extract is weighed and dissolved in 1 ml of MeOH. The sample is hydrolysed to formulate the anthocyanins into aglycons. 200µl of 37 % HCl is added and the sample is held in 90 °C water bath for 60 minutes. Anthocyanin aglycons are analysed by HPLC. Cyanidin chloride is used as an external standard (concentration range from 8.4 ppm to 210 ppm).

### Detection

HPLC analysis is performed using Waters equipment combined with PDA detector and with Empower software. Reverse-phase separation is attained in room temperature using an Agilent Hypersil C-18 (5µm, 4.6 x 150 mm) column. Samples of 30 µl were injected. A gradient solvent system is used with solvent A being formic acid/water (10:90 v/v), solvent B being methanol/acetonitrile/formic acid/water (10:1:10:79 v/v/v/v) and solvent C being methanol/formic acid/water (10:10:80 v/v/v). The following gradient, with a flow rate 0.9 ml/min, is used for elution: from 0 to 24 min 80 - 40 % A and 20 - 60 % B, from 24 to 36 min 40 - 20 % A and 60 - 80 % C, from 36 to 37 min 20 - 80 % A and 80 % C to 20% B followed by isocratic elution from 37 to 50 min 80 % A with 20 % B.

### Materials and methods

### Plant material, growth conditions and stress treatments

Catalase deficient (CAT2HP1) and control (PTHW) plants were obtained as described by Vandenabeele et al. (2004). Unless mentioned otherwise, the plants were grown under controlled conditions in phytotron exposure chambers, which had been specially designed for plant stress research (Thiel et al., 1996). The light regime was 12h/12h at 100-140 µmol m⁻² sec⁻¹, the climate adjusted to a relative humidity of 70% and 22°C day/18°C night temperatures. For high light (HL) treatments, six-week-old plants were transferred to a sun simulator with identical growth conditions and exposed to continuous HL irradiation (photosynthetically active radiation 400-700 nm at approximately 1600-1800 µmol m⁻² sec⁻¹). 0, 3 and 8 hours after the onset of HL stress, middle-aged leaves of 20-30 plants per line were sampled and pooled for RNA-analysis. The two biological repeat experiments were done with a temporal interval of one year.

### Microarray analysis

In two independent experiments, RNA was isolated from 20-30 control or catalase deficient plants using TRIzol Reagent (Invitrogen, Carlsbad, CA, USA). The concentration of total RNA was determined with a Nanodrop ND-1000 spectrophotometer, and the quality was examined with the RNA 6000 Nano Assay (Agilent Technologies, 2100 Bioanalyzer). Each of the different pools of control and CAT2HP1 plants, subjected to 0, 3 and 8 hours of HL irradiation, was hybridized to one Affymetrix chip (Genechip® Arabidopsis ATH1 Genome Array; Affymetrix, Santa Clara, CA, USA). For each hybridization, 15 micrograms of total RNA was used. Affymetrix chip analyses were performed at the ETH-Functional Genomics Center (Zurich, Switzerland) and the VIB Microarray Facility (Leuven, Belgium), respectively. Conditions for reverse transcription, RNA labeling, hybridization and scanning were performed according to manufacturer's instructions (https://www.affymetrix.com/) Raw data were processed with the statistical algorithm of Affymetrix Microarray Suite (MAS) 5.0 as described by Liu et al. (2002). Subsequently, a per chip normalization was performed, dividing all measurements on each chip by the 50^{th} percentile value (median). To calculate the median, measurements were limited by flag values: only measurements flagged as present were used. Genes with at least four present calls over the 12 different data points were retained for further analysis. Expression values were obtained by taking the average of the normalized values of the two independent repeats. As a selection criterion for differential expression a coefficient of variation (CV) was used, which was calculated as the ratio of the standard deviation on all measurements of the time course and the (absolute value of the) average expression over the time course. Expression values of genes with a CV higher than 2 were taken for hierarchical cluster analysis, using CLUSTER and TREEVIEW software (Eisen et al., 1998), to obtain a global view of the transcriptional changes. For the in pair comparison at different time points, fold changes were calculated using the average expression value of the two independent experiments. Only fold changes with at least two present calls (i.e. detectable expression) over the four data points were used. Analyses were based on annotations compiled by TAIR (http://www.arabidopsis.org/)

### Publicly available Affymetrix GeneChip data

The GeneChip data were retrieved from the international AtGenExpress repository (from The Arabidopsis Functional Genomics Network - http://www.unifrankfurtde/fb15/botanik/mcb/AFGN/AFGNHome.html) and downloaded from TAIR (http://www.arabidopsis.org/servlets/Search?type=expr&search action=new search). Raw data were processed with the statistical algorithm of Affymetrix MAS 5.0 (Liu et al., 2002) and we performed a per chip normalization as described above. Growth stage annotations were based on Boyes et al. (2001).

### Tables

**Table 1: Anthocyanin measurements in transgenic Arabidopsis thaliana lines overexpressing SEQ ID NO: 13, 15, 18, 23, 24, 30, 52, 54, 56, 63 and 68 versus the untransformed line. Values are means values from three independent transformants. Anthocyanin values are expressed as absorbance per gram fresh weight (abs/g Fw).**

| **Transgenic line** | **Anthocyanin content (abs/g Fw)** |
|---|---|
| SEQ ID 13 | 0.11 |
| SEQ ID 15 | 0.14 |
| SEQ ID 18 | 0.21 |
| SEQ ID 23 | 0.25 |
| SEQ ID 24 | 0.12 |
| SEQ ID 30 | 0.15 |
| SEQ ID 52 | 0.17 |
| SEQ ID 54 | 0.11 |
| SEQ ID 56 | 0.13 |
| SEQ ID 63 | 0.14 |
| SEQ ID 68 | 0.11 |
| Untransformed line | 0.06 |

## Claims

1. Use of the polynucleotide sequence consisting of SEQ ID NO: 54, or a functional fragment thereof possessing the biological property able to modulate the production of at least one flavonoid in an organism or cell derived thereof, or a homologue thereof possessing the biological property able to modulate the production of at least one flavonoid in an organism or cell derived thereof, to modulate the biosynthesis of flavonoids in plants or plant cells.

2. Use according to claim 1 wherein said flavonoids are flavonols or anthocyans.

3. Use according to claim 2 wherein said flavonols are of the quercetin type.

4. A recombinant DNA vector comprising the polynucleotide sequence consisting of SEQ ID NO: 54, or a functional fragment thereof possessing the biological property able to modulate the production of at least one flavonoid in an organism or cell derived thereof, or a homologue thereof possessing the biological property able to modulate the production of at least one flavonoid in an organism or cell derived thereof.

5. A transgenic plant or plant cell derived thereof that is transformed with a recombinant DNA vector according to claim 4.


SEQUENCE LISTING

<110> VIB vzw
      Universiteit Gent

<120> Means and methods to modulate flavonoid biosynthesis in plants
      and plant cells

<130> FVB/ANT/207/EPd

<150> EP 05104855.1
<151> 2005-06-03

<160> 69

<170> PatentIn version 3.5

<210> 1
<211> 2004
<212> DNA
<213> Arabidopsis thaliana

<400> 1
atgtggcaaa cgtggccacg tcagccaatt ctactagata ttttttcaaa tccaaatact      60

ctttccacaa ccgttagatc atggtcggtt cgccacccac tttcaatcat aaccgttaaa     120

acattcgcta gattttttct agatattttc ttttctccac actattatag aaagaataaa     180

gttctttttt ttgctctctt ctcatttatc tctccactca caaatatttt gatttgtttt     240

gtaactgttt ctctttctct ggagctttct tcttcttctt caataatcga tttaggtttt     300

tcaaagctaa gtgtttgtgt tgtgataatg actagtagcg aggaagtagt tgaagtgacg     360

gtggttaaag cacctgaagc tggcggagga aagttatcac gtcggaagat tcggaagaaa     420

gacgccggtg ttgatggttt ggtgaagtgg gagagatttc tcccgaaaat cgcgcttaga     480

gttttgctcg ttgaagctga tgattctact agacagatta tcgctgctct tctcaggaaa     540

tgtagttaca gagttgctgc agtacctgat ggcttaaaag cttgggagat gctaaaagga     600

aagcctgaaa gtgttgattt gatattaaca gaggttgatc taccttcaat atctggatat     660

gctctgctaa cacttatcat ggagcatgat atttgcaaga acattcctgt tataatgatg     720

tcgacacagg actcggtgaa tactgtgtat aagtgtatgt tgaaaggtgc ggctgattat     780

cttgttaagc cgttgaggag gaatgagctt agaaatcttt ggcagcatgt ctggagaaga     840

caaacttcac ttgctcctga tagctttcca tggaatgaga gtgttggaca gcagaaagcc     900

gagggtgcgt ctgcaaacaa ctcgaacgga aagagagacg atcatgttgt gagtgggaat     960

ggtggtgatg cccagagctc gtgtacaaga ccagagatgg aaggtgagag cgcagacgtg    1020

gaggttagtg cgagagacgc agtacagatg gagtgcgcaa agtctcagtt taatgagaca    1080

cggcttctag caaatgagtt gcagagtaag caagcagaag ccattgactt catgggagca    1140

tcgtttagaa gaactggacg acgtaacaga gaagaaagtg ttgctcaata cgaatctcgg    1200

atagagcttg atctttctct gagaagacct aatgcttctg agaaccaatc ttctggagac    1260

cggccttctc ttcatccttc tagtgcctca gctttcacac ggtacgttca caggccgttg    1320

cagacacaat gttcagcctc cccagtggtt actgatcaaa gaaagaatgt tgcagcaagt    1380

caagatgata acattgtgct aatgaaccaa tacaatacat ctgaaccgcc tccaaatgct 1440

ccaagaagaa acgacaccag cttttacact ggagctgact cacctggtcc accgtttagt 1500

aatcagctga attcttggcc gggacagagt tcatacccta cgccaacccc tatcaacaat 1560

atacagttca gagatcccaa cacagcttat acatctgcaa tggctcctgc ttcactctcc 1620

ccaagcccta gttccgttag cccgcatgag tacagttcca tgtttcaccc attcaacagt 1680

aaacccgagg ggttacaaga ccgggattgt tccatggatg tagatgagag gagatacgtc 1740

tcttctgcaa ccgaacatag tgcaataggc aatcacattg atcagcttat tgagaagaag 1800

aacgaagatg gctattcatt atccgtcggg aaaattcagc aatctcttca acgagaagcc 1860

gctttaacca aattccgaat gaagcgaaag gacagatgtt atgagaaaaa ggttcgttac 1920

gagagccgga agaaattagc agagcaacga ccacgaatca aaggccaatt cgttcgtcaa 1980

gtccaatcca cacaagctcc atag 2004

<210> 2
<211> 651
<212> DNA
<213> Arabidopsis thaliana

<400> 2
atgaactcat tttctgcttt ttctgaaatg tttggctccg attacgagtc ttcggtttcc 60

tcaggcggtg attatattcc gacgcttgcg agcagctgcc ccaagaaacc ggcgggtcgt 120

aagaagtttc gtgagactcg tcacccaata tacagaggag ttcgtcggag aaactccggt 180

aagtgggttt gtgaggttag agaaccaaac aagaaaacaa ggatttggct cggaacattt 240

caaaccgctg agatggcagc tcgagctcac gacgttgccg ctttagccct tcgtggccga 300

tcagcctgtc tcaatttcgc tgactcggct tggagactcc gaatcccgga atcaacttgc 360

gctaaggaca tccaaaaggc ggcggctgaa gctgcgttgg cgtttcagga tgagatgtgt 420

gatgcgacga cggatcatgg cttcgacatg gaggagacgt tggtggaggc tatttacacg 480

gcggaacaga gcgaaaatgc gttttatatg cacgatgagg cgatgtttga gatgccgagt 540

ttgttggcta atatggcaga agggatgctt ttgccgcttc cgtccgtaca gtggaatcat 600

aatcatgaag tcgacggcga tgatgacgac gtatcgttat ggagttatta a 651

<210> 3
<211> 540
<212> DNA
<213> Arabidopsis thaliana

<400> 3
atgcaagact cttcctctca cgaatcgcaa cgtaacctcc ggtcaccggt gccggagaaa 60

accggaaaga gttctaagac taaaaatgag caaaaaggtg tttctaaaca accaaatttt 120

cgtggggtca gaatgagaca atggggaaaa tgggtgtctg aaattagaga accaagaaag 180

aaatcaagaa tatggctcgg tactttctct acgccggaga tggcggcgcg tgcacacgac 240

gtggcggctt tagccatcaa aggtggctct gcccacctta atttcccgga gctagcttac 300

catttgccga gaccggctag cgcggaccct aaagacattc aagaagccgc cgccgcagca 360

gctgccgttg actggaaagc accggagtct ccgtctagca ccgtgacgtc atctccagtc 420

gccgacgacg ctttctccga tcttcctgat cttttgcttg acgtgaatga tcacaacaaa 480

aacgatggat tctgggactc gtttccgtac gaagatcctt tcttcttgga aaattactag 540

<210> 4
<211> 642
<212> DNA
<213> Arabidopsis thaliana

<400> 4
atgaactcat tttcagcttt ttctgaaatg tttggctccg attacgagcc tcaaggcgga 60

gattattgtc cgacgttggc cacgagttgt ccgaagaaac cggcgggccg taagaagttt 120

cgtgagactc gtcacccaat ttacagagga gttcgtcaaa gaaactccgg taagtgggtt 180

tctgaagtga gagagccaaa caagaaaacc aggatttggc tcgggacttt ccaaaccgct 240

gagatggcag ctcgtgctca cgacgtcgct gcattagccc tccgtggccg atcagcatgt 300

ctcaacttcg ctgactcggc ttggcggcta cgaatcccgg agtcaacatg cgccaaggat 360

atccaaaaag cggctgctga agcggcgttg gcttttcaag atgagacgtg tgatacgacg 420

accacgaatc atggcctgga catggaggag acgatggtgg aagctattta tacaccggaa 480

cagagcgaag gtgcgtttta tatggatgag gagacaatgt ttgggatgcc gactttgttg 540

gataatatgg ctgaaggcat gcttttaccg ccgccgtctg ttcaatggaa tcataattat 600

gacggcgaag gagatggtga cgtgtcgctt tggagttact aa 642

<210> 5
<211> 927
<212> DNA
<213> Arabidopsis thaliana

<400> 5
atggcgtcgt ggatgaaagc ggtgctaatc tctactggcg tcgtagccac ggctatgcat 60

ctaaaggtta ttgttcctgt ggctatggat ttctcacaaa atccgattat tttgagctct 120

ttcctcacgt ggctgaaacc gccgtatctt tacgtcatca ctaacgtcat catcatcgtt 180

gtcggagttt cctaccggat tactactgtc tccagccacg tcgacggcaa agactatgag 240

gcttcttaca gtggcgacaa taagtttcag actgatcatc agcagatcgt ccaagaagct 300

cctctaaggc gacgaacgga gacgaaagat gcggattttg gtttcatcgg caaagttttg 360

cagatcgtta aggagccgga ggttgtgtat gaagagaagg agaggccggc gacggtagag 420

gaggaggaga agaagtgtat aattgtggtg agcaaatcgg aaaatcaacc tccggtggag 480

aagcctcttg ttacggctag gatcggccaa aagaaaccgg tggttaagac tacaccagca 540

gaaaggaatt ctatgagagc gttgagagtt gcgaaaccga aacgtaacga gacgttagag 600

aatacgtgga agatgattat ggaaggcaac aagtcaacgc ttccgttgac cagttattac 660

aagagacccg acacgttcgg acttggcgaa gagacaaaac aatcaggtgt tttgaagaaa 720

tcggagacgt ttagtgacag aactaactgt taccagtctc tgccgccgcc acctccgccg 780

ctagtgaagg tgaagaaggt gaaagtgtca cggagtaggg atgagcttaa ccggaaagta 840

gaagcgttta taaaaaaatg caacgacgag aggttcgcgt cgatgaaact ggacaacgaa 900

gtggctcgtc atggtctttc ttattaa 927

<210> 6
<211> 2460
<212> DNA
<213> Arabidopsis thaliana

<400> 6
atggcggaca agctagctct tcctcttctc cttccctgca ctccttcctc taaaccttat 60

tctcacgacc aaaaccacca tatctctcgg acgccttttc ttactacgtc tctttcgtca 120

ccacctcctc cgcctgtaga gcctctcctc cacgatgttt tccttcacca gaaccctaat 180

tccagacaac ccatcagctc tcaaacatct agaaaccgta accggactcg aattggcaag 240

tcacgtgacc ctaacctcgg taaaccttgg tcttaccatg gtctttctcc acaaggtcag 300

caagttcttc gttccctcat cgaacccaat tttgattccg gtcaattaga ttctgtactc 360

tctgagctat tcgagccttt taaggataaa ccagagtcta cctcgtcgga gttactagct 420

tttcttaaag gattaggatt tcataagaaa ttcgatttgg ctctgcgtgc ttttgattgg 480

tttatgaagc aaaaggatta tcaatccatg ttggataact ctgttgttgc tataatcatc 540

agtatgctag gtaaagaagg cagagtatct tccgctgcaa atatgttcaa tggtttgcag 600

gaagacgggt tttcgcttga tgtctactct tatacttcgt tgatatcagc gtttgctaat 660

agcggaaggt atagggaagc tgtaaatgtg ttcaagaaga tggaggaaga tggttgtaaa 720

ccgactttga taacgtataa tgttatcttg aatgtgtttg ggaaaatggg tactccttgg 780

aataagatta cgtctcttgt tgagaagatg aagagtgatg ggattgctcc ggatgcgtat 840

acttacaaca ctcttataac ttgttgtaaa cgaggctctt tgcatcagga agctgctcag 900

gtttttgaag aaatgaaggc tgctgggttt agttatgata aggttactta taatgcgtta 960

ttagatgttt atggaaagtc tcatcggcct aaggaagcta tgaaggtttt gaatgaaatg 1020

gtgctcaatg gattttctcc gagcattgtg acttacaact ccttgatctc tgcatatgcg 1080

agggatggta tgctggatga ggcaatggag cttaaaaatc agatggcgga aaagggaacg 1140

aaacctgatg tttttactta tacaacactt ttgtcagggt ttgagagggc tgggaaggtc 1200

gaatctgcta tgagtatttt tgaagagatg agaaatgcag ggtgcaaacc aaatatttgt 1260

acttttaatg cctttataaa gatgtatggt aacaggggaa agtttactga aatgatgaag 1320

atatttgacg agatcaatgt gtgtggtctc tcccccgaca ttgtcacttg gaatacacta 1380

ttagcagtct ttggccaaaa cgggatggat tcagaagtat cgggtgtatt caaggaaatg 1440

aagagagctg ggttcgtacc cgaaagggaa actttcaaca ccctaatcag tgcgtatagc 1500

cgctgtggtt cgtttgaaca agctatgact gtttacagac gaatgcttga tgctggggtc 1560

actcctgacc tttccaccta taacactgtg ttggcagctt tggcccgtgg aggaatgtgg 1620

gaacaatctg aaaaagttct tgcagagatg gaggatggtc ggtgcaaacc aaatgaatta 1680

acttactgct ctctacttca tgcatatgca aatggcaagg agattggtct gatgcattct 1740

ctagcagaag aggtttattc tggagttatc gagcctcgag ctgtgctttt gaagaccctt 1800

gtcttggttt gtagtaagtg tgatcttttg ccagaggctg aacgtgcatt ctctgagctc 1860

aaagaaagag ggttttcacc agacataacc acattaaatt ccatggtctc catatatgga 1920

agaaggcaga tggtggcaaa ggcgaacgga gtcttggact acatgaaaga aaggggtttc 1980

acaccaagca tggcgaccta caatagcctc atgtatatgc atagtcggtc tgcagatttc 2040

ggaaaatcag aggaaatctt gagggaaata ctggctaagg ggatcaagcc agacatcata 2100

tcgtacaaca cagtcattta cgcctattgt agaaatactc ggatgagaga tgcatctaga 2160

atattttcag agatgaggaa ttcagggatt gtccctgatg ttatcaccta caatacgttt 2220

attggttctt atgcagctga ctcaatgttt gaggaggcca tcggcgtcgt taggtacatg 2280

atcaagcatg gttgtagacc aaaccagaac acctacaact ccattgtcga tggatactgc 2340

aagctaaaca ggaaagatga ggcaaaactt tttgtcgaag atctgaggaa tcttgatccc 2400

catgctccca aaggcgagga tcttaggttg ctggaacgga tagtgaagaa gtggccatag 2460

<210> 7
<211> 2535
<212> DNA
<213> Arabidopsis thaliana

<400> 7
atgaggatta tgattaaggg aggtgtttgg aagaacaccg aagatgagat tctcaaagcc 60

gccgtgatga agtatggtaa gaaccaatgg gctcggatct cgtctcttct cgttcgtaag 120

tctgctaaac agtgtaaagc tcgctggtac gagtggctcg atccatctat caaaaagact 180

gaatggacca gagaagaaga tgagaagctt ctacatcttg ctaaacttct gcctactcaa 240

tggagaacta ttgctcctat tgtgggtcgt acaccatctc aatgtcttga gaggtatgag 300

aagctccttg atgcagcatg cactaaggat gaaaattatg atgcagcgga tgatccacga 360

aaattacgtc ctggtgagat tgatccgaac ccagaagcaa agcctgctcg tcctgatccg 420

gtagacatgg acgaagatga gaaagaaatg ctttctgaag caagagctag attggctaac 480

acgaggggaa agaaggctaa aagaaaagct agagaaaaac aacttgagga agctagaagg 540

cttgcttctc tgcaaaaaag aagagaacta aaagcagctg ggattgatgg aaggcatagg 600

aaaagaaaga gaaagggaat cgactataat gcagaaattc cttttgaaaa gagggcacct 660

gcgggatttt atgatactgc ggatgaagat cgtcctgctg atcaagtaaa atttccaact 720

accattgaag aacttgaagg aaaaagaaga gctgatgtag aagcacattt acgcaaacaa 780

gatgttgcaa ggaataaaat tgctcagaga caggatgctc cagcagctat attgcaagca 840

aacaagctga atgatccgga agttgttagg aagaggtcaa agctgatgtt accaccaccg 900

cagatttcag accacgagct agaagaaatt gctaagatgg gctatgccag tgaccttctt 960

gccgagaatg aggagctaac agaaggcagt gctgctactc gtgcactttt ggcaaattac 1020

tcacaaacac caaggcaagg aatgacaccc atgaggacac ctcaaagaac tcctgctggt 1080

aaaggtgatg ctattatgat ggaagcagaa aacctggcca gattaagaga ctctcagaca 1140

cctttgctag gaggagaaaa tcctgagttg cacccttctg acttcactgg ggtcactccg 1200

agaaagaagg agattcaaac gcctaatcca atgttgaccc cttcaatgac tcctggtggt 1260

gctggtctta ctccaagaat tggcttgacg ccatcaaggg atgggtcttc tttttctatg 1320

acacccaaag ggactccctt cagggatgaa cttcacatta acgaagacat ggacatgcac 1380

gaaagtgcaa aacttgagag gcagagacga gaggaagcta gaaggagttt acgctctggt 1440

ttgactgggc ttcctcagcc aaagaacgag taccaaatag ttgcacaacc tcctcctgag 1500

gaaagtgaag agccagaaga gaaaattgag gaagacatgt cagacaggat agcgagggaa 1560

aaggcggagg aagaagcaag acaacaggca ttgcttaaga agagatccaa ggtcttgcag 1620

agagatcttc ctagaccccc agctgcttca ttggcagtaa ttaggaactc gttgctttca 1680

gctgatggag acaaaagttc tgttgttcct cctactccga ttgaggttgc agataaaatg 1740

gtaagagagg agcttctaca gttgctggag catgataatg caaagtatcc gcttgatgac 1800

aaagctgaga agaagaaagg agccaagaac cgtaccaacc gttctgcttc tcaagttctt 1860

gcaattgacg attttgatga aaatgagctc caagaggctg acaaaatgat aaaggaggag 1920

gggaagtttc tgtgtgtgtc aatgggacat gagaacaaga cacttgatga ttttgtagaa 1980

gctcacaaca catgcgtgaa tgatctcatg tatttcccca ctcgaagcgc ttacgagctc 2040

tcaagtgttg ctgggaacgc ggacaaagtt gcagcttttc aggaggagat ggagaatgtg 2100

agaaaaaaga tggaggagga tgagaagaag gcagaacaca tgaaggccaa gtacaaaact 2160

tatacaaagg gtcatgagag gagggcagag accgtgtgga cccaaataga ggcgacattg 2220

aagcaggctg agattggtgg aacagaagta gagtgcttta aagcattgaa gaggcaagaa 2280

gagatggctg catcttttag gaaaaagaat ttgcaagagg aagtgataaa gcaaaaggaa 2340

acagagagta aactgcagac tcgctatggg aatatgttgg caatggttga aaaagcagag 2400

gagataatgg tcggtttccg agcacaggca ttgaagaaac aagaggatgt tgaagattct 2460

cacaaactga aagaagctaa gctagccact ggagaggaag aggacatagc catagccatg 2520

gaagcttctg cataa 2535

<210> 8
<211> 3444
<212> DNA
<213> Arabidopsis thaliana

<400> 8
atggccgccg acgaactgat gccgtctcac aggtcacaca ggactcccaa atcaggtcct 60

accgcgagga agaaatctga actagataag aagaagcgtg gaatctccgt tgacaagcag 120

aaaaacctta aggcgtttgg tgttaaatcg gttgttcatg cgaagaaagc aaaacatcac 180

gctgcggaga aggagcaaaa gcggcttcat cttccgaaaa ttgatcgtaa ttatggcgaa 240

gctcctcctt tcgtcgtcgt ggttcaaggc cccccaggag ttggaaagtc tctcgtgatt 300

aaatctcttg tgaaggaatt tacaaaacag aatgtacccg aggttcgagg acctattacc 360

attgtacaag gtaaacagag aaggtttcaa tttgtggagt gcccgaatga tatcaatgcg 420
atggtggatt gtgcaaaggt tgctgatcta gccctacttg ttgtagacgg gagttatggt 480
tttgagatgg aaacctttga attcctcaat attatgcaag tgcatggatt tcctagagtt 540
atgggtgttc tcactcacct tgataagttc aatgatgtta agaagctgag aaaaacaaaa 600
catcatctca agcatcggtt ttggactgaa atatatcatg gagctaaatt gttttattta 660
tctggtctca ttcatgggaa gtatacgccg cgtgaagttc acaacctcgc ccgctttgta 720
attgttatca agcctcagcc attgacatgg cgaacagcac atccttatgt gttggttgat 780
cgccttgaag atgttacccc tccggagaaa gttcagatgg ataagaaatg cgatagaaat 840
atcactgtgt ttggttacct acgtggttgt aacttcaaaa aaaggatgaa ggttcatatt 900
gctggagttg gtgacttcat tgtagctggg gtgactgctt taactgatcc ttgtccttta 960
ccttcagctg gcaagaaaaa agggctgagg gacagggata agcttttcta tgctcctatg 1020
tccgggattg gagatcttgt gtatgacaaa gatgctgttt acatcaacat aaatagtcac 1080
caagttcagt actctaaaac tgacgatgga aagggagaac ctactaataa aggaaagggc 1140
agagatgttg gtgaagattt ggtaaagtcg ttgcagaaca caaagtattc tgttgatgag 1200
aaactagata agacattcat taactttttt ggcaaaaaga ctagtgccag ttcagaaaca 1260
aaacttaagg ctgaagatgc gtatcactct ttgccggaag gttctgacag tgagtctcaa 1320
tctggcgatg atgaggagga tatagtaggt aatgaaagtg aaatgaagca ggaaactgag 1380
attcatggtg gaaggttgag gaggaaagct atcttcaaga cggacttgaa tgaagatgat 1440
tttgaggaag cagacgatct tgaattggat tcatatgacc cagatacata tgattttgag 1500
gaagcagacg atgctgaatc agacgataat gaagttgaag atggtggaga tgactctgct 1560
tccgattcag ccgatggtga accaggggat tatcagatag atgataagga ctctggtaac 1620
atatcacaat ggaaagcacc cttgaaggag atagccagaa agaagaaccc caacttgatg 1680
caaattgtgt atggagcatc atcattagct actcccttga taaatgagaa ccatgacatt 1740
agtgatgatg acgaaagtga tgatgaagac ttctttaagc caaaaggaga acaacacaag 1800
aatttaggtg gtggattgga tgtgggatat gtcaactcag aggattgttc taaatttgtg 1860
aattatggat acctaaagaa ttggaaagag aaagaagtat gtgagagcat tcgtgatcga 1920
tttaccactg gtgattggtc aaaagctgct ctgagagaca aaaatttagg tactggcggt 1980
gagggagaag atgatgaact ttatggtgat tttgaggatc tagagacggg agagaagcac 2040
aaaagccatg agaacttgga atcgggtgca aatgaaaatg aagatgaaga tgcagaagtc 2100
gttgagcgtg atgggaacaa tcctcgtagt caagccgatg aaccaggata cgctgataaa 2160
ttgaaggaag cgcaggaaat tacaaaacag aggaatgagt tagaatacaa tgatcttgac 2220
gaggaaactc gaattgagtt agcaggattc cggactggaa catacttgag gctggagatt 2280
cacaatgttc cttatgagat ggttgaattc tttgatcctt gtcatccaat tctagttgga 2340
ggtattggtt tcggcgagga caatgttgga tatatgcagg cccggttgaa gaaacatagg 2400
tggcataaga aagtactaaa gacaagagat cctattattg tgtctattgg atggagacgc 2460

tatcagacta ttcctgtatt tgccattgaa gatcgcaatg gcaggcatcg aatgctcaag 2520

tatactccag aacacatgca ctgccttgct tcgttctggg gtcctcttgt cccacccaac 2580

actggctttg tcgctttcca gaacctgtca aacaatcagg caggatttag gataacagcg 2640

acttctgtag ttctggagtt taatcaccag gcccgtattg taaagaaaat caagctggtt 2700

gggactccgt gcaagatcaa gaaaaagact gcatttatca aagacatgtt cacttctgac 2760

cttgaaatag ctcgatttga aggttcatct gttcggacag ttagtggcat tagaggacaa 2820

gtaaaaaagg ctggaaaaaa catgcttgat aacaaggctg aagaagggat tgcgaggtgt 2880

acctttgaag atcaaatcca tatgagcgac atggtattct taagggcttg gactacagtg 2940

gaagttccac aattttacaa tcctctaacg acagccttgc aaccccgcga taagacctgg 3000

aatgggatga aaacttttgg cgaactccgt agagagctga atattcctat tccagtgaat 3060

aaggattcac tctacaaggc aatcgaaaga aagcaaaaga agttcaatcc actacagatt 3120

ccaaagcgtc tagaaaaaga tttaccgttt atgtcgaaac ccaaaaatat accaaagcgg 3180

aaaagaccat cactagagga taaaagagca gttataatgg aaccgaaaga aagaaaagag 3240

catactatca tccagcaatt ccagctgctt caacatcaca cgatgaagaa gaaaaaggca 3300

acggatcaga agaagaggaa agagtatgaa gcagagaaag ctaagaatga ggaaataaat 3360

aagaaacgta ggagagaaga gagacgggac agatatcgtg aggaagataa acagaaaaag 3420

aagacgagaa gaagccttga ttaa 3444

<210> 9
<211> 729
<212> DNA
<213> Arabidopsis thaliana

<400> 9
atgggaagag gtagggttca gctgaagagg atagagaaca agatcaatag gcaagttact 60

ttctcaaaga gaaggtctgg tttgctcaag aaagctcatg agatctctgt tctctgcgat 120

gctgaggttg ctctcatcgt cttctcttcc aaaggcaaac tcttcgaata ttccaccgac 180

tcttgcatgg agaggatact tgaacgctat gatcgctatt tatattcaga caaacaactt 240

gttggccgag acgtttcaca aagtgaaaat tgggttctag aacatgctaa gctcaaggca 300

agagttgagg tacttgagaa gaacaaaagg aattttatgg gggaagatct tgattcgttg 360

agcttgaagg agctccaaag cttggagcat cagctcgatg cagctatcaa gagcattagg 420

tcaagaaaga accaagctat gttcgaatcc atatctgcgc tccagaagaa ggataaagcc 480

ttgcaagatc acaacaattc gcttctcaaa aagattaagg agagggagaa gaaaacgggt 540

cagcaagaag gacaattagt ccaatgctcc aactcttctt cagttcttct gcctcaatac 600

tgcgtaacct cctccagaga tggctttgtg gagagagttg ggggagagaa cggtggtgca 660

tcgtcgttga cggaaccaaa ctctctgctt ccggcttgga tgttacgtcc taccactacg 720

aacgagtag 729

<210> 10
<211> 1134
<212> DNA
<213> Arabidopsis thaliana

<400> 10
atgaagaaga aggtgtctca gcagaagtta ctgtacagat ggaagaggaa ggtatacgcc 60

acgttgatgt tcgctttctg ctttgggact ttcgtattta tacaagctcg tttcgcatct 120

atacaagctc gtttcaatcg aatctctgcg tctctcgatt cgcttaaaaa gcctcgtcta 180

gatcagagac cacagattgc cttcctcttc attgcccgga atcgactccc tctcgagttt 240

gtctgggatg ctttctttaa gggtgaggat ggaaagttct caatatatgt tcattctaga 300

cctggatttg ttctcaacga ggctacaacg cgatccaagt actttttgga tcggcaactt 360

aatgacagta tacaggtaga ttggggtgaa tcaaccatga ttgaagcaga acgtgtattg 420

cttagacatg cacttagaga ttcatttaat caccgctttg tttttctttc tgatagctgc 480

atacctctgt acagtttcag ctacacgtat aactacatca tgtcaacacc aactagtttc 540

gttgatagct ttgcagatac aaaagatagc cgttataatc ctagaatgaa tcccattatt 600

cctgttcgta actggagaaa aggatcacag tgggtcgttc tgaatagaaa acacgcagaa 660

attgtggtga atgatacctc tgtctttcct atgtttcagc agcattgcag gagaaaatca 720

cttccagagt tttggcgaga tcgtcctgta ccagctgaag gttggaagga acacaactgt 780

atacctgatg agcactatgt tcagacattg ctatctcaaa agggtgtaga tagcgaactc 840

acacgaagat cactgacaca ctcagcttgg gacctttcat cctcgaaaag taatgaacgt 900

cgtggatggc atcctatgac ttacaagttt tctgatgcta ctcctgatct tatacagtcc 960

attaagggaa tcgacaatat caactacgag actgaatacc ggcgagaatg gtgtagcagt 1020

aaagggaaac catcaccgtg cttcctcttc gccaggaagt tcactcgtcc cgccgctctc 1080

cgcctactcc gtgaaactat cttgttagag ggcaaagagc atgacaataa gtag 1134

<210> 11
<211> 2106
<212> DNA
<213> Arabidopsis thaliana

<400> 11
atgtcgttaa aactcaacac tccttttcca attttcgcgc catctctatt tcctaatcat 60

aacccaagag cacccagcga gatccgattc tctagatggg gcaacgctaa tgccgaacgg 120

ttcgagcagc gtcgccggag ccaagaagaa ctcgaggctg agatccgtcg ggaccgccga 180

ttcgacgccg ctactaaaat cgtccatacc catgattccg aagcagcagc tgctgagcct 240

aaaacgtcac cgtttagatc aagaggcact ccttcacttc cctctgctcg ttcgattccg 300

ggtcgaagat ccaaatactc caaacccgat tcaggaccca atagacccaa gaacaaacct 360

agagtacccg attcgccgcc gcaactagac gctaagcctg aggttaagct aagcgaagat 420

ggattaactt acgtcatcaa tggagctcct ttcgaattca agtacagtta cacggagacg 480

ccaaaggtta agcctttgaa gcttcgtgag cctgcttacg cgccttttgg acctacgact 540

atgggaaggc catggactgg tcgtgctccg cttcctcagt cgcagaagac gccgagagaa 600

ttcgattctt ttcgattgcc tcctgtgggg aagaaagggc tgaagccggt gcagaaaccg 660

ggtccttttc gacccggggt aggtccaagg tatgtttact ccaaggagga gattttagga 720

gagccattga caaaggaaga ggtcagagag ctggttactt cttgcttgaa gacaacaagg 780

caattgaata tgggcagaga tggtttgacg cataacatgt tgaacaacat acatgatcta 840

tggaagcggc gaagggtttg taagattaaa tgcaaaggag tttgtacagt cgatatggat 900

aatgtttgcg agcagttaga ggagaaaatt ggtgggaagg tgatatatag aagaggaggt 960

gtgcttttcc tattccgtgg cagaaactat aaccacagga caagaccgcg gttccctctt 1020

atgttgtgga agcctgtagc acctgtttat ccaaggctaa ttcaacaagt gcctgagggt 1080

ttaactcgtc aggaagctac caatatgcgg aggaaaggac gagagctcat gcccatttgc 1140

aagctaggga agaatggtgt gtattgtgat cttgtgaaaa atgttaaaga agcatttgaa 1200

gtttgtgaat tggttcggat cgattgtcaa gggatgaaag gcagtgattt taggaaaatc 1260

ggtgccaaac tcaaggatct tgttccatgt gtgctcgtat cttttgaaaa cgagcagatt 1320

cttatctgga gaggacgaga atggaaatcg tctctcacaa ctccagataa aaagggtgat 1380

atccttgaag atatcgaagt tgatactgcc ttgccagaag atgacgaacc atcggtgtca 1440

ccaaatcaga gtcaaactat gacccagaac cctcctctgg attctatgga actgcaaaat 1500

gatccagatg gtcacgattt gagcccttca actgtagatt cctcggaaat ggaaggcaca 1560

atcaattctt tacagagctg gtctacaaaa gatgtaactg agccaacggt agatagtttt 1620

cttcgagacc ttgaagaacc tgaagacgaa ccagaaacat cggaagagat cagcaaacaa 1680

agcatagaga gagttctgat tttgatgaaa caagctgtgg agagcgggac tgcacttgtg 1740

ttagatgctg ctgatctgga cgcagacaca gtcttttcaa aagctgttgc cttttcgagt 1800

gtagcttcac caggaccagt tttccagcat ggcttgagaa aacaaccaac ggttaagaag 1860

caggaaagcc aagaattcgg gtacggagac ttggaggcaa aatcaagtaa tgtagtggtt 1920

tctaggaatg cttccaaatc aagtaatgtt gtggtttttg ggaaaagaga agttgcagag 1980

aggggggaaa gagaggagaa ggaggaggga tcgaagaaga aaatggacga gtttgctgaa 2040

gattacagag aagtgatgcc gcatggaaca ttgaaggtag atgaactagc taaactactt 2100

gcataa 2106

<210> 12
<211> 1746
<212> DNA
<213> Arabidopsis thaliana

<400> 12
atgttttcgt tatcgttaat ccaaccgcgt ctccggattt cagagattcc ggtgactcaa 60

tcctacaaat ctccgacgat atgttacagt agcgattcaa gaactaagcg agaggaacag 120

agacacgtga gattacctgg gtttcgatta gtttctggaa agagagcatc tttcgattcg 180

ggttttagtg gtttcaaagg agagaatgtg aatcaggatg attcgtcttc tttcgatagc 240

gaaagagttg attatgctct gttagcggag tggctacagt cttctaatgg gatgcgactc 300

attaaaagga tccatgcgat ggcgttgaaa cttggtgatt tggtttatgc acgtaaagtg 360

ttcgacagta tgcctgagaa aaatactgtt acttggactg ctatgattga tgggtatttg 420

aagtatggtc ttgaggatga ggcttttgca ctgtttgagg attatgtgaa gcatggaata 480

cgttttacga acgagaggat gtttgtgtgt ttgttgaatc tgtgtagtag gagagcagag 540

tttgagttag ggagacaagt tcatggtaat atggtgaaag ttggagtggg gaatctcatt 600

gtggagagtt ctcttgttta tttttatgcg caatgcggtg aattgacaag tgcgttacga 660

gcttttgata tgatggagga gaaagatgtg atatcttgga ctgctgttat atcggcgtgt 720

tcgagaaaag ggcatggaat taaagctata ggcatgttta tcggaatgtt gaatcactgg 780

tttttgccta acgagtttac ggtgtgcagt attttgaagg cttgtagtga ggagaaagcg 840

ttaagattcg gaaggcaagt acacagcttg gttgttaaga ggatgataaa gacagatgtt 900

tttgtgggaa cttcgctgat ggacatgtat gctaagtgtg gggagatttc tgattgcaga 960

aaagtgtttg atggaatgag taatagaaac acggtcacat ggacttcgat tatagctgct 1020

catgctcggg aaggttttgg tgaggaagct atcagcctct tccggataat gaagaggcgg 1080

catttgattg ctaacaattt gacagtagaa cttcatgcac agattatcaa gaattcgatc 1140

gaaaagaatg tctatatagg aagtactttg gtgtggctgt attgtaaatg cggagaatct 1200

cgtgacgctt tcaatgttct ccagcaattg ccatctagag atgtggtttc atggaccgct 1260

atgatctctg gatgttcgag cttaggacat gaatcggaag cgctagactt cttgaaagag 1320

atgattcaag aaggtgtaga gccaaaccca tttacatact cctcggcttt aaaagcttgt 1380

gcgaattcag aatctcttct tatcggtaga tcaatccatt ccattgcaaa gaagaatcat 1440

gctctatcaa atgtctttgt gggaagtgct ttgattcaca tgtatgcaaa atgtggattt 1500

gtctcggaag cttttcgggt ttttgacagt atgcctgaga agaacttggt ttcatggaag 1560

gcgatgataa tgggttatgc gaggaatggg ttttgcaggg aagcattgaa gctaatgtat 1620

agaatggagg cagagggatt tgaagttgat gattatatat ttgcaacaat tctctctact 1680

tgtggagata ttgagctcga tgaagctgtt gaatcttctg caacttgtta cttggagaca 1740

tcttga 1746

<210> 13
<211> 843
<212> DNA
<213> Arabidopsis thaliana

<400> 13
atggatctag aagattggga aatactcccc aaaatcaact acaagggtct cgaacttgat 60

ctcggtcatg aagaggatca tgaagttacg aagatgatga gaaacaccgc aaaaagcttc 120

gacagtgatt acttcatctg cccaattcaa gattctgtcg gaaagacaga gtttcttcat 180

cagagatcta gcgtggtccc cacacaactc ctccagattc caataacttg ggaacctttg 240

tcccccgtgg acgacaaaga tcacaataag tacctggatc cggatttctc ggaaccagac 300

ccggaacttt tgacggagtc ttttccgtcg ccgagaataa ccttcaagaa atcgaaggaa 360

accgaatttg ccgacatgaa aatagattca ccagcagcga ggttcactag tcctctgccg 420

cagaacgatg agagacactc tgactcagaa ggagggttag gaggagagtc ttatgatgag 480

atcatgggat cagaggttga agaaagcagt gacttgagta gcaagaaaga ggttgattgg 540

gatgaaggtg aaagaacgaa tctgtggaag aagggtctta atggaattgg agctatatgt 600

tcatttggtg ttgcagctgc tgcagccacc atatgtgtct tcttccttgg acacaacagt 660

agcatccaag gtggtcggaa caagaaccag atcctcaggt tccagattta ctctgatgat 720

aataagcgga tgaacgaggt agtgaaacat gcaacaaagc taaatgaagc aatctctgtg 780

atgaaaggtc ttccggtggc aagagctcaa atatcttttg gaggatacta cgatgcactt 840

tga 843

<210> 14
<211> 1818
<212> DNA
<213> Arabidopsis thaliana

<400> 14
atggcggatc ctctaaacgg caagtccttc tttatctgtt tctccctttt attctccttc 60

actctgcttt tcatttcgcc gttgtatgcc accgagtctc cggttatcga agatgtttct 120

accgatgttg ctgtgtctgt tagcgaaacc aatcgagaag ctgttctatt gcataattta 180

gaggaactcg ttaagaatct gacggaatta gtcgctaatc tagatgctaa gttatctgca 240

actccattaa aggagaagaa cgagatctca gttgatgatg acatcggaga agagaaagag 300

agaggaaggg ctaaggcgtt ttcagtgact aaatacagtc cgttttggtc ggagaggttt 360

cagtttacat cagctgtgaa actcaattcc gatgcgactt gtatcaatgt gttgccgttt 420

agagatttcg aaggttcaag caagtacttt gcaattggtg attctaaagg tagagtttat 480

gtgttcttga gaaatggtga tgttttgatt gagtttttca ccactgttga ttctccggtt 540

actgctatgg tttcgtattc atctgtgttt aagaactcga gtttcgtggt tacgggtcat 600

cagaacggtg cggtgttgtt gcatcggatt cacgagggat cgaatggcga agattggaac 660

tcgaattcgg tttctatgga acatgttggg aagtttgatg tggatgattc agctgatcct 720

gtgactttgt tggaagtgca tcatgtgggt cgtgttaggt atatattggc gactgattta 780

agcgggaagc tcacggtttt aactgagaac aggacggttt atgggtcggt tattccatcg 840

agtagaccgc tcgtgttctt gaagcagaga ttgttgtttc ttactgagtc tggtgctggt 900

tccttggact taagaagcat gaagataaga gaaactgagt gtgaaggact gaaccattcg 960

cttgcgagaa cttatgtttt tgatgctgcg gaacggtcta aagcttatgg attcacatcc 1020

gagggcgaga tcattcacgt attgcttcat ggagatataa tgaacttcaa atgtagggtt 1080

agatccaaga agaagtttca aatggaggag ccagtagctt tacaatcaat caaaggctat 1140

cttctagtta tcaacgaaga aaaggttttc gctttcaatg tatcgactca gcattatgtt 1200

cgtactgcgg gtcctcggct tttgttctca gcgggattag aagagatcag atccgcgttc 1260

ttgagccatc gcgaatcatc ttcacgaacc accacagtag taaagactag gccgttaata 1320

gctagcgaca gggaaaacct tcttgtgatc ggtttagaaa acggatattt cgctgtttac 1380

aaatcgaagc tgccaactct caaaggagac ttcaacacaa tgctttggag cagtcctgtg 1440

ttcttcttca tactatttct attcggggct tggcatttct ttgccaagaa gaaagaatcg 1500

ctcactgcat ggggaccaga tgatcctttt accccgaccg gcgcacaaaa tagttcggcg 1560

aaagagccaa catttactga accttcaaga agaaacgatg acctcatgga tctacggaga 1620

aggtacgctg gtggctcata ccggtcagtt ggagctaacg acccgagttc aagagctccg 1680

gttgatggaa actatagaac aaccgcacag gatcataaca attatcgcgg tggtggctcg 1740

ggtcttgatt caaacgggtt tggtaataga agagatcatt tgtttggtaa caacaaagtt 1800

ttggataacg aaagttag 1818

<210> 15
<211> 255
<212> DNA
<213> Arabidopsis thaliana

<400> 15
atgcaagacg ccgagacatc acgacagccg gcgaagtctt tgtccgatcg agtgaagact 60

aactgtttat ccatggcagt aacatgccag gaagggttta gctatgtcaa agcctttttt 120

gttggccaga caaagagatt gacggcaaag aacgagaagg aagctacgga ggctcatcta 180

acggagacaa aaatgcaagt tgacgcaacc gatgaagcag agaatgccaa gaagagactt 240

catcaatctt cttaa 255

<210> 16
<211> 1737
<212> DNA
<213> Arabidopsis thaliana

<400> 16
atggctttgc gtttaggtgt ttctataggg gcagctttgg gttcctctca ttgggacgac 60

ggacaacgag tacgacaacg tgacttctcc gcttctgtga atttcaccgc accggttacg 120

agccggagga gcttaagggg tagtagaacc ggtgtgagga ttcttagggt ttcaaatgaa 180

ggacgcgaat cgtacctcga tatgtggaag aacgctgttg atcgcgagaa gaaagagaag 240

gcctttgaaa aaattgcaga gaatgttgta gctgttgatg gtgagaagga gaaaggagga 300

gacttggaga agaagagcga tgagtttcag aagatcctcg aggtttccgt tgaggaaaga 360

gatcggattc agcgaatgca ggtcgttgat cgtgccgctg ccgcaatctc cgcagctaga 420

gctattctcg cctctaacaa ttccggcgac ggcaaagaag gattcccaaa tgaagacaac 480

actgtcacaa gtgaagtcac agagacaccg aaaaatgcta aacttggaat gtggagcaga 540

acagtgtatg tgccacggtc agaaacttca gggactgaga caccaggacc agatttttgg 600

tcatggacac ctcctcaagg tagtgaaatt agttctgtgg acttgcaggc tgtggaaaag 660

cctgctgagt ttccaacttt gccaaatcct gtattggaga aagataaatc agcggattct 720

ctttcgatac catatgagag tatgctttct tctgaaagac atagctttac tatcccgcct 780

tttgagtctt tgattgaggt tcgaaaagag gctgagacga agcctagctc cgagacttta 840

tcgacagaac atgaccttga tctcatatct tcagcaaacg cggaagaagt agctcgtgtt 900

cttgatagtt tggatgaatc ttcaacgcat ggagttagcg aagatggatt gaagtggtgg 960

aagcaaacgg gtgtggagaa aagacctgat ggtgtggttt gcaggtggac aatgatacgt 1020

ggggttactg ctgatggtgt tgttgagtgg caagataagt attgggaggc ttctgatgat 1080

tttgggttca aggaacttgg ttctgagaaa tcaggacgtg atgccactgg aaacgtgtgg 1140

cgtgagttct ggagagagtc aatgagccag gagaatggtg ttgtgcatat ggagaaaact 1200

gcagacaaat ggggaaagag tggacaaggt gatgaatggc aagagaaatg gtgggagcat 1260

tacgatgcta ccggaaaatc agaaaaatgg gctcataagt ggtgcagcat tgaccgcaac 1320

acgcctcttg acgctggcca cgctcatgtc tggcacgaga ggtggggaga gaagtatgac 1380

gggcaaggcg gaagcacaaa gtacacagac aagtgggcgg aacggtgggt aggtgacggt 1440

tgggacaaat ggggagacaa atgggacgag aactttaacc cgagcgctca aggagtgaaa 1500

caaggtgaga cttggtggga agggaagcac ggcgacagat ggaaccgaag ctggggagaa 1560

ggtcacaacg gatcaggatg ggttcacaaa tacggaaaaa gcagcagcgg tgaacactgg 1620

gacacacatg taccacaaga aacttggtat gagaagttcc ctcactttgg cttcttccac 1680

tgttttgaca actctgttca gctccgagcc gttaagaagc cttctgatat gtcctag 1737

<210> 17
<211> 2301
<212> DNA
<213> Arabidopsis thaliana

<400> 17
atgagtatca tgctatcaat ttcccggcgc cagaactctt atattctgct caaccattct 60

cgattcctcc ggcgtttttc ttatgatgtt gacccacggc cggaaatcaa atcggagagc 120

caggaatttg tagtagtcaa atttgtgaaa actcttcaaa ataccccgca acatgattgg 180

gcgtcgagcg agtcgctaag tgcgcttgtc gtatcttctt cttctgcttc tcctttagta 240

ttctcgcaaa tcacgcggcg gctaggatcg tattctctag caatctcgtt cttcgagtac 300

ctggatgcga agtctcagtc tctgaaacgc cgtgaagaat ctctctcctt ggcgcttcag 360

tcggtcattg aattcgccgg tagtgaaccg gacccgcgtg ataaacttct ccgtctctac 420

gagatcgcca aagagaagaa cattcctctt actatcgttg ccactaagct tctgatccga 480

tggtttgggc gtatgggtat ggtgaatcag tcggttcttg tatacgaaag actcgattcg 540

aatatgaaaa actcgcaggt tcgtaatgtt gtggtagacg tcttgctaag aaatggactt 600

gtggatgatg ccttcaaggt gctcgacgaa atgcttcaga aagaatctgt ttttcctcct 660

aatagaatca cagcggatat tgtgttacac gaggtttgga aggaaaggct tttgacagaa 720

gaaaagataa ttgctttgat ttcaagattt agctctcatg gtgtctcccc aaactctgtt 780

tggttgactc ggtttatatc aagtctatgc aaaaatgctc gcgccaatac tgcttgggat 840

attttgagcg acctgatgaa gaacaaaacc ccacttgaag ctcctccctt caatgcgctt 900

ttgtcttgct taggaaggaa tatggacatt agtagaatga atgatttagt cttgaagatg 960

gatgaggtga aaatccggcc tgatgttgtg actttaggga ttcttattaa cactttatgc 1020

aaatcaagaa gggtagatga agctctcgaa gtttttgaac aaatgcgtgg aaagagaact 1080

gatgatggaa atgtgattaa agctgattcg attcatttta atactctcat tgacgggctc 1140

tgcaaggtgg ggaggttgaa agaagcagag gagttattgg taaggatgaa actggaagag 1200

agatgtgtgc ccaatgcagt tacttacaat tgcttgattg atgggtattg cagagctgga 1260

aagcttgaga cggctaaaga agtcgtttct cggatgaaag aggacgagat taaacctaat 1320

gtggtaactg ttaatacaat cgttggtggg atgtgcaggc accatggatt gaacatggcg 1380

gttgttttct ttatggatat ggaaaaggaa ggcgtgaaag ggaatgtggt tacttatatg 1440

acattgattc atgcttgttg cagcgtcagt aatgtagaga aggctatgta ttggtatgaa 1500

aaaatgttgg aagctggttg ttctcctgat gcaaagatct attatgcttt gatctctgga 1560

ttgtgccaag ttagacggga tcatgacgcc attagagtgg tggagaaact gaaagaagga 1620

gggttttctc ttgacttatt ggcttacaac atgcttattg ggttgttttg tgataagaat 1680

aatgcagaga aagtctatga gatgctaacc gatatggaaa aagaagggaa gaaacctgat 1740

tccatcactt acaacactct gatttcgttt ttcggtaaac acaaggactt cgagagtgtt 1800

gagagaatga tggagcagat gagagaagac gggttagacc cgactgtcac gacatatgga 1860

gcggtgattg acgcttattg ctcagtcggc gaattagacg aagcattgaa gctctttaag 1920

gacatgggtt tgcactcaaa ggtcaatccg aacactgtaa tatacaacat tctcataaac 1980

gcattttcta agctggggaa tttcgggcaa gcgctctctc tgaaagagga aatgaagatg 2040

aagatggtga gacctaatgt tgaaacttac aatgccttgt ttaagtgtct taacgagaaa 2100

acccaaggag agacattact taaactgatg gatgagatgg tcgaacagtc ttgtgaacca 2160

aatcagatca caatggagat tctaatggag cgtctctcag gttctgatga gttagttaag 2220

ctgaggaagt ttatgcaagg ctactctgtt gcttcgccga ccgagaaagc ttcacctttc 2280

gatgtcttta gcttgggata a 2301

<210> 18
<211> 1116
<212> DNA
<213> Arabidopsis thaliana

<400> 18
atgggaagag cgccatgttg cgagaaggtc ggtatcaaga gagggcggtg gacggcggag 60

gaggaccaga ttctctccaa ctacattcaa tccaacggtg aaggttcttg gagatctctc 120

cccaaaaatg ccggattaaa aaggtgtgga aagagctgta gattgagatg gataaactat 180

ctaagatcag acctcaagcg tggaaacata actccagaag aagaagaact cgttgttaaa 240

ttgcattcca ctttgggaaa caggtggtca ctaatcgcgg gtcatctacc agggagaaca 300

gacaacgaaa taaaaaatta ttggaactct catctcagcc gtaaactcca caacttcatt 360

aggaagccat ccatctctca agacgtctcc gccgtaatca tgacgaacgc ttcttcagcg 420

ccaccgccgc cgcaggcaaa acgcagactt gggagaacga gtaggtccgc tatgaaacca 480

aaaatccaca gaacaaaaac tcgtaaaacg aagaaaacgt ctgcaccacc ggagcctaac 540

gccgatgtag ctggggctga taaagaagca ttaatggtgg agtcaagtgg agccgaggct 600

gagctaggac gaccatgtga ctactatgga gatgattgta acaaaaatct catgagcatt 660

aatggcgata atggagtttt aacgtttgat gatgatatca tcgatctttt gttggacgag 720

tcagatcctg gccacttgta cacaaacaca acgtgcggtg gtgatgggga gttgcataac 780

ataagagact ctgaaggagc cagagggttc tcggatactt ggaaccaagg gaatctcgac 840

tgtcttcttc agtcttgtcc atcggtggag tcgtttctca actacgacca ccaagttaac 900

gatgcgtcga cggatgagtt tatcgattgg gattgtgttt ggcaagaagg tagtgataat 960

aatctttggc atgagaaaga gaatcccgac tcaatggtct cgtggctttt agacggtgat 1020

gatgaggcca cgatcgggaa tagtaattgt gagaactttg gagaaccgtt agatcatgac 1080

gacgaaagcg ctttggtcgc ttggcttctg tcatga 1116

<210> 19
<211> 7395
<212> DNA
<213> Arabidopsis thaliana

<400> 19
atggataaag agacggagat tctctcccgt ctcgcggcga accaccttca tctggctcaa 60

ttcgagccat tgaaggctac gttactcgct ctcagggttc gtaaccctga cctcgcactc 120

accattctcc aaaccatcgt ctccaacgct ggaagattcg ataatgtcct ctggtcacgc 180

tcttgtcctt ccccgtctct tctctcgttc ctctccacga ttgagcttct gagattcgaa 240

aatcctactt ctccttgggg atttgattca gaaactctaa gtttgcgtgc cgatttcttg 300

ttgatggttc aggttttgat cgatagagtt acagagagga ttaaggaaga tgaggagagt 360

gaggatgaaa attctggatt agggaattgt ttaagggtgt tgcaaggtgt tttggagtta 420

ggtgttgaga ggttgaagtt tgttgttgat actagtagta gtgaaggaag taataagatt 480

gaggaagatg cagttgtgtc tttgaggagt atagtattgg attactctga tgttttcgat 540

gctttatgtt gtaatattca gaggcaactt gcgggttgcg agagttacgg tacatgtttg 600

gttgaggaag ttcagggaga agaacagaga aaggagatga atgaggccac atgtattggt 660

tctccggagc tggataacat caatgtgttt gctttgatac agaggaatgt tcagttagca 720

cagttggatg ctatgaaaac aaagttggat gaaggtgatg agcgcggggc agctgatcgc 780

attcgttatc ttcaccttga ttatggagta gagaaagaga actatcatgc tgttctaaaa 840

gctctccttt caagagttat ggagaaaaag gatgaatatg gtgattcctg gcacatggtg 900

cgccagaact tgctgtttat gtataaagaa gctctctcat cgaattgtgg agatcttgtt 960

cagatgatcc agggtattca agatgatatg ctcctcccac atagccaact acatttatct 1020

ctcgacaatg aacaaattcc actccctctt gaatgtttcc ggcgatatct tgtagacttg 1080

aaaactgaga gaaatataga ggacaaaagt tctcctatga gcagggcaat taattcttgt 1140

ctcagagata tgtatcatta tgctcgtatt tctggatcac atgttcttga gtgtgtgatg 1200
tgtgctgctt tgtcttctgt aaagaaagag aagcttcagg aggctaatga tgttcttact 1260
ttgtttcccc gacttcgccc tttagtagcc tccatgggtt gggatctatt gccgggcaaa 1320
actgcaaccc gtagaaaatt gatgcggcta ctttggacta gtgactcgca agcacttcgg 1380
ctagaagaat cttctcttta tggaaaccag acagatgaac tggaacttgc atctttcgct 1440
gcttgtgtca attctggtaa atcatggact ccaaaggcat ctttcttgat gcatggtaat 1500
gtgtcatccg cgcatgatga tgcggaggtg gatccttttg ttgaaaatct tgtattggaa 1560
aggctttcag cgcaaagtcc acttcgggta ttgtttgacg ttgttccggg cataaaattc 1620
caagatgcta tttcactgat tagtatgcaa cctattgctt caactgcaga agcctggaag 1680
aggatagaag atattgaact gatgcatatg cgttatgctc tggaggcaat cgttttagca 1740
ctaggtgcaa tggaagaggc tatgaaggat gagacagatg ctagtcatcg agtagtattt 1800
taccatttaa aagacctcac taaccatttg gaggccatta aaaatgttcc acgcaagata 1860
atgatggtga acatagttat ttcactctta catattgatg atatccgtct cagttctacg 1920
caaagtgcct cctcggcatg tttttctgaa aaaagtaaca cacctggttt ggatcctggc 1980
gatcttggta cagaagggga aaaggaaatt gttatttctt tcacaaaaca gctactcgat 2040
gttttacgcc gcaatcttcc atcacatcca attgaacaag agtgtcagct ggatggtaat 2100
tacagtactg atggaagaca ggctttagaa tggagagtat ccatggctaa gcgtttcatt 2160
gaagattgtg aatggcgatt atctgttatg cagcatcttc tgccactttc tgaacgccag 2220
tggggtttaa aggaggtttt gagtattcta agggcagccc ctgaaaaact gcttaatctc 2280
tgtatgcaaa gagctaagta tgacattgga gaagaggcag ttaatcggtt tgcgttatca 2340
gcagaggaca aagctactct tgaattagct gaatgggttg ataatgcgtt caaaggaaca 2400
ctggtagaag atgtaatgtc tcgtactgct gaaggagcag ctgccgtgca agatttagat 2460
tttcattctt taggttctca attgagtcca ttggctatgg ttttactttt tgcgcagtct 2520
caagttatgt tatcggaaat ttaccctgga ggagctccga aggtggggtt tacttactgg 2580
gatcaggtcc acgaagttgc aataatttct gtattgcgaa ggatcttaaa gcgtctgcag 2640
gaattccttg aacaggatga ccctcaaatt cttcaagcca gttttagtgg agataccata 2700
atttcatctt gcacggaatc tcatagacag ggacaaaaag atcgtgctct tgcaatgcta 2760
catcaaatga ttgaggatgc tcataggggc aagcgtcagt tcctgagtgg taagcttcat 2820
aacttagcga gagcactcgc tgatgaaaaa ccagaagttg acgtactcaa aggggacgga 2880
tcagacatgg ccgttgagaa ggatggagtt cttggtcttg ggctaaaata tacaaagcaa 2940
agtcctggtt cagcaaatag agccgtggat ggaaatcctg tttcacatga aacagaagac 3000
aagggaaaga agtcatttgg cccattaagc aacaaaacct ctacttatct atctcagttt 3060
atcctctata ctgctgctat tggtgatata gtagatggaa ctgacacaac ccatgatttc 3120
aactttttct ctcttgttta tgaatggcct aaagacctat tgacgcgtct ggtttttgat 3180

cgaagtagca cagatgcagc tgcaaaagtt gctgaggtta tgtctgctga ttttgttcat 3240
gaagtgatat cagcatgtgt tcccccagtt tatcccccac gttctggtca tgggtgggct 3300
tgtattcccg tcattccaac cactccatgt tcccactcag agggtaaagt gctctctcct 3360
tcaatagagg ctaaacccaa ctgttatgtc cgttcctcag caacacctgg tgtccctctg 3420
tatcctcttc agttggatgt tatcaggcat ttggtaaaaa tttcaccagt acgagcagtt 3480
ttagcttgcg tctttggtgg gagcatattg tacaatggca gtgattctat catatctagc 3540
tccttgaacg atgagtttcc aagttctcct gatgcagaca gattgtttta tgaattttct 3600
cttgatcagt ctgagaggta tcccacttta aaccgatgga tacagatgca gactaatctg 3660
catcgagttt cggaatttgt tgtgacaccg aagcaaaaac ctgatgacac acggattaag 3720
cctgatgaaa gaactgggat caagagactt cttgaacatg atagtgactc agagtcagat 3780
acagaagaaa cattttctaa aaataacatt caaccagcat tgacagacgg cagtgctcgt 3840
gatggtggat cctttgaaaa tggagtttgt agaactgatc ctaccgtttt cctttctttt 3900
gattgggaga atgaagtacc gtatgagaaa gctgtaaata gactaattga tgaaggaaaa 3960
ctaatggatg ctttagcact ttcggaccgc ttcttgcgga atggggcttc tgattggtta 4020
cttcagctcc taatcaaaag tagagaagaa aatccttcaa catcaggacg atctcaggga 4080
tatggaggcc agagcaacag ttggcagtat tgcctgcggc taaaggacaa acagctggca 4140
gcaacactgg ctcttaagtg ttgcatagga gacaagctct gcagaagtac agccacatac 4200
tttcggcaga tgatcgccat aatagctggc aagaggttat ctttcttcct tttgtttgag 4260
atcatgtttg gctcttggta tgctcgatgt gtcactctca aaaatctaaa tgggaaacag 4320
gtagaagccg aatgtaaaga agaccctgaa ggcttggctc taagattggc tgggaaagga 4380
gctgtttccg ctgcgctaga agtggctgaa agtgcaggat tatcaataga tcttagaaga 4440
gaattacaag gacggcagct tgtaaagctt ctaaccactg atccactcaa tggtggtggt 4500
ccggcggaag catctcggtt tttatcttca cttcaagact cggctgatgc tctaccagtg 4560
gttatgggtg caatgcaatt attacctgac cttcggtcga aacagctcct ggtccatttc 4620
tttctcaagc gaagagatag caatctgtcg gatttggaag tcgcccggct taattcttgg 4680
gctttaggtc tgaaagtgtt agctgcatta ccacttcctt ggcaacagag atgctcttcg 4740
cttcatgagc atcccaattt aatatttgaa gctctgctaa tgagaaaaca attacaatac 4800
gcctcactga tactcaaaga gttcccagca ttaagagata acaatgttat catggcctat 4860
gcagcaaaag ctatttctgt gacaattatc ccaccaccaa gagaacctcg aataactgtg 4920
tctgcgtcaa ggttaagaca gaaatcaaga gcagggccag cagtaaaagc atccttcact 4980
agtagcttaa gcaattttca gagagaggct cgaagggcat tctcatgggc tccacgtaat 5040
gctgaaaacc ggacgacgtc aaaggatgtt tatcgcaaaa gaaagaattc cggactgggg 5100
gcttctgaga gagctgcatg ggaggcgatg acaggtattc aagaggacca gggatcatct 5160
tattcggcag atgggcaaga taggctacct tctgtttcta ttgctgaaga atggatgcta 5220
actggcgaca aaaccaaaga tgaaggtgtt cgtgcatctc acaagtatga aagcaccccc 5280

gatattattc tctttaaggc tctactatca ctttgttcag atgagctagt atcagcaaga 5340
agtgccatgg acctatgcat cagtcaaatg aaaaacgtat tgagctccaa acagttgtca 5400
gagggtgcgt ctgttgaaac aattggccga gcatatcatg caacggaggc atttgtgcag 5460
ggtttgtcgt atgcaaaatc attgctgaga aagcttctag gtaccactga atcgaccaat 5520
aacaatggtg aaagaagtag agatgttgat gatatatctt ctgatgctgg cagctccagt 5580
gttggcagtc agtcaacgga tgaaccatcg gatgttctct cacttacaga aatctggttg 5640
gggcgcgcag agttgctgca gagcctctta gggtctggaa tttctacttc tcttgatgac 5700
attgctgatc aattgtcatc cgaatgtcta cgagacagat taatatctga tgaacgatat 5760
agtatggcgg tgtatatgtg caagaaatgc aagattgatg ttttccccgt gtggaaagca 5820
tggggtcttg ctttgttacg gatggagcgc tatgctcaag ctcgagtcaa attcaagcaa 5880
gcctttcaat taaaggggga agacattcct gatgtcattc aggagataat aaatacaata 5940
gaaggaggtc cgcctgtgga tgtgtcaatt gtacgttcca tgtatgacca tttggcgaaa 6000
agcgcaccta caattttgga cgattcttta tcagcagact catacctaaa tgttctgcat 6060
atgccatcca ctttccctcg ttcagagagg tcgcggagat ctctggaatc ggaaaagaat 6120
agttctgtac ctggttcaga ctttgaagat gggccccgaa gcaacttgga tactacacgc 6180
tattctgagt gcaccaacta cttgcaggaa catgctcgcc aaaacctgct tgggtttatg 6240
ttccgccatg gtcactttaa agatgcatgc atgttattct ttccgcaaag tggtcttccc 6300
cctcctttgc aaacttcatc tgtgggcgca gtaagcacat cttcatcacc tcaacgaact 6360
gatcccttgg caactgaata tgggaccatt gaaagtttgt gcgagttctg tgttggttat 6420
ggagctatct catcccttga ggaagtaatt acagaaagac ttgaatccgc aaagaatcaa 6480
gatcaagcca taaatcagta catagctgga gctcttactc gtatctgtgc tttctttgag 6540
atcaaccggc atttcaatta cctatacaag tttctggtac tcaagaagga ttatgtcacc 6600
tctgggtatt gttgtattca gctttttatg aattctacaa ctcaggagga tgctgtaagg 6660
catcttgagc atgcaaagaa atactggtct ctaactatcc tcggggtaca ggcacacttt 6720
gaagaagcat tgacagcgcg tcatagaggt tcagactcaa aaaaacttgt tacaaagggt 6780
gttagaggaa aaagtgccgc agagaagctg agtgaagaaa ctcttgttaa gttgtcctca 6840
cgggtgaaaa tgcagattga tgtggtgaag tccttcagtg actctgaagg agcaccatgg 6900
aagcattcct tgtttggaaa tccaaatgat tcagagacat ccaggagaag atgtgaaata 6960
gtggagactc ttgttgagaa aaatttcgac ttagcttatt ctgttatata tgaattcaag 7020
ctctcagctg ttgatatata tgctggtgtt gctacgtcac tagctgatag gaagaaaggc 7080
agtcagttga cagaactttt caaaaacatt aagggaacaa tccaggatga tgactgggat 7140
caggtcctgg gtgctgccat caatatatat gccaacaagc acaaagagcg ccctgaccgt 7200
ctcatcgaca tgttaacaag cagccatcga aaggtgctgg cttgtgtggt atgtggccgc 7260
ctgaaaagcg cattccagat tgcatctaaa agcggaagcg tggctgatgt tcaatatgta 7320

gctcatcaag ccttacatgc caattcgcac acagtactcg atatgtgcaa gcaatggcta 7380

gctaaataca tgtaa 7395

<210> 20
<211> 1488
<212> DNA
<213> Arabidopsis thaliana

<400> 20
atgtgtttta ataacattga aactggtgat gaagtggaaa ccgagaggca agtgtttggt 60

tcatctgaag aagatgaatt tcgagttgaa gatactgcta gaaataccaa caatgtacag 120

atttctcaac aacagcagca accgctagct catgttgtga agtgggagag gtatctccca 180

gttagatcgc ttaaggttct tctggtggag aatgatgact caacacgcca tattgttact 240

gcccttttaa agaattgcag ctatgaagtt actgctgttc cggatgtcct tgaagcctgg 300

agaattctag aagatgagaa aagttgcatt gatcttgtct taacagaggt tgacatgcct 360

gtgcattcag gaaccggtct gctgtccaag attatgagcc ataagacact taagaacatc 420

cccgtcataa tgatgtcatc acatgattct atggttctgg tctttaagtg tttgtcgaat 480

ggtgctgttg attttctcgt gaaacccatt agaaagaacg aactaaagaa tctttggcaa 540

catgtctgga gaagatgtca cagctctagc ggaagcggaa gtgagagtgg aatacatgac 600

aagaagtcgg tgaaacctga aagcacccaa gggtcagaaa atgatgccag catcagtgat 660

gaacacagga atgaaagtgg gagtagtggt ggtttgagta accaagatgg tgggagtgat 720

aacgggagtg gaactcagag ttcttggaca aaaagagcca gtgatactaa gagcacctcg 780

ccttcaaatc aatttcccga tgcacccaac aagaaaggaa cctatgaaaa tggatgtgca 840

catgttaata gactgaagga ggctgaagat cagaaggaac aaataggcac gggatcacag 900

acaggaatgt ctatgagtaa gaaagctgaa gaaccaggag atcttgaaaa gaatgcaaag 960

tattctgttc aagctttgga gagaaacaat gatgacacgc tgaatcgctc ttctggtaac 1020

tcacaagtag aaagcaaagc accttcatct aaccgagaag atttgcaatc actcgagcaa 1080

actctgaaaa aaacaagaga ggatagagat tacaaagtcg gtgatcgaag tgtgttgagg 1140

cattcaaatc tctctgcatt ctcaaaatac aataatggtg ctacttctgc taagaaggct 1200

ccagaagaaa atgtggaaag ttgttctcct catgacagtc ctattgcaaa actgttgggt 1260

tcgagttcaa gcagtgacaa tcctttaaag cagcagtcta gtggaagtga ccgatgggca 1320

caaagagaag ctgctttgat gaagtttcgc cttaaacgta aagagcgatg ttttgagaaa 1380

aaggttaggt accatagcag gaagaaacta gctgagcaac ggcctcacgt caaaggtcaa 1440

ttcattcgca agagggatga tcataaatca ggaagtgaag acaattga 1488

<210> 21
<211> 3555
<212> DNA
<213> Arabidopsis thaliana

<400> 21
atgcgtagat tctctaccat tgttgatctt ctcatcagca aaaaaccatc ttctcaagca 60

aattctagaa ttgatctcat ttgtaaaagg ttccatattt caagagttct caataacgat 120
ttcgtagaat caacagagag aaagaatggg gttggtttag tttgtccaga gaagcatgaa 180
gatgaattcg ccggtgaagt cgagaagatt tacagaattt tgcggaatca ccattctaga 240
gttccaaaat tggagcttgc tcttaacgaa tcaggtattg atctgcgacc cgggttgatc 300
atacgagtgt tgagtcgttg tggcgatgct gggaatctag gttatagatt ctttctgtgg 360
gcaacgaagc aacctggtta ttttcatagc tatgaagtgt gtaaatcaat ggtgatgatt 420
cttagtaaaa tgcgacaatt tggagctgtt tggggtttaa ttgaagagat gaggaagacg 480
aatccggagt tgattgagcc ggagttgttc gttgtattga tgcggaggtt tgcttctgct 540
aacatggtga agaaagcagt tgaggtgctc gacgaaatgc ctaagtatgg gttagagcct 600
gacgagtatg tttttggttg tttgttagat gctttgtgta agaacggtag tgttaaggag 660
gcttcaaagg tttttgagga tatgagagag aagtttcctc cgaatttgcg gtattttact 720
tcgttgttgt atggttggtg tagggaaggg aagttgatgg aagctaaaga agttttggtt 780
cagatgaagg aagctgggct tgagcctgac attgtggttt tcactaactt acttagtgga 840
tatgctcatg ctgggaaaat ggcggatgcg tatgatctta tgaatgatat gagaaagaga 900
gggtttgagc cgaatgtgaa ttgttacacg gttttgatcc aggcgttgtg taggacggag 960
aagagaatgg atgaggcgat gcgggttttt gttgagatgg agaggtatgg atgtgaggct 1020
gatattgtga cttataccgc gttgataagt gggttttgta aatggggaat gattgataaa 1080
ggttatagtg ttttagatga tatgagaaag aaaggagtca tgccgtcgca agtaacatat 1140
atgcagataa tggtggctca tgagaagaaa gaacaatttg aagagtgttt ggagttgatt 1200
gagaagatga agcgaagagg ttgtcatcct gatcttctca tttacaatgt agtgataaga 1260
ttggcttgta agttagggga agtgaaagaa gctgttcggt tatggaacga aatggaagct 1320
aatgggctaa gccctggagt tgatacgttt gttattatga tcaacggatt tacaagccaa 1380
ggtttcctaa tcgaagcctg taatcacttc aaagaaatgg taagccgagg aatattctct 1440
gcgcctcaat atggaacgct gaagtcattg cttaataacc ttgttagaga tgataagctc 1500
gaaatggcga aagatgtatg gagttgcata tccaacaaaa cctcttcctg tgagctgaat 1560
gtatcagctt ggacaatatg gatccatgct ttgtacgcaa aaggtcacgt gaaggaagcg 1620
tgttcgtatt gtcttgatat gatggagatg gatttgatgc cgcaacctaa tacttatgcg 1680
aaacttatga aaggattgaa taaactgtat aataggacga tcgctgcaga gattacagag 1740
aaggtggtga agatggcaag tgagagagag atgagtttta agatgtataa gaagaaaggt 1800
gaggaggatt tgattgagaa agctaaacct aaagggaata aagaaggaaa gaagaaaggg 1860
acagatcatc aaaggtataa gggaagagtg tctcttgctc ggaaccgtct taggtcggaa 1920
acgccatcat cttttcttgc tcgagaccgt cttaggtcaa aaacgccatc atcgtctcca 1980
ttttcttcga agcggcatac gcctaagaca agcgaaatag aagaagagtc gactccaaaa 2040
gattcagttt tgttaaaccc taaagatcct tcaagcgcac ctaagctctt ccttgtacag 2100

cctcgtttag caccgccaaa gtatctacaa gcgaagctga acgaagcgct ttgtctcgcg 2160

aattcgcttg aagagcaacg atatgggtac tttgaatctg atttctttga caaggaattg 2220

ccttctcatg ttgttgttca aaaccctgtc cgtagatcgt ctaaacctcg cgaagaagtt 2280

gatgctgttt tcgtaaacgc cattttgacc gctatccaac aacggaattt agagcgaata 2340

tgggcaaaac ctgtcttaga ccgtgtgggt cttataatcg aaatatttaa tgctcatgcg 2400

catacaaagg aagcaaaact acaggctgag ttagctgctt tgatgtacaa taagagcaga 2460

ctggttcgag tgcgtggtac tgatggacgc catacttttg ggcagtttgg agaagctgaa 2520

gttgtcagtg cccgagggag agcaggaagc aagggaaccg gtggcggttt tgtaggtggt 2580

gcaggagaaa ctgagcttca gcttcaacgc cgaagaatat cagaccggag gattcgcttg 2640

ttatcccaaa ttaaagaagc ccagcgaaca cggctattgc agcgtgctgg acgtaagaaa 2700

agagtggggt tagagggtga gagttcagga accattgctg ttgttggtta cacaaatgct 2760

ggaaaatcga ctctgataag tgcactaaca aagactgctc tctactgcaa tgagcgattg 2820

tttgccacat tagatcctac actcaagagt gcccatcttc cttctggaaa ctttgtgctt 2880

cttagtgaca ctgtcggatt catatcagat ctgcctatac agctggtgaa agcttttcaa 2940

tcgactctgg aagaagttgt tgaagctgat ctacttctgc atgtagttga ttcaacagct 3000

ccaaatatcg aggagcatcg ttcaacagtg cttcatgtcc taaatcaaat tggagtacct 3060

gaagagaagc ttcaaaatat gattgaagtc tggaataaga ttgattatga agaagacgaa 3120

gtggaggaag agaaatatct agatgatggc gaaggagtag gagaagaaga cgaagacgaa 3180

gctgatttaa aagctgaaga aactgttgat gcatctgaag caacagtaga tgaagaccaa 3240

atccaaaacg gagacggtga cgacgctgat gggtggctat tgtctgaaga tgaaaatgct 3300

gacgaccctg agttctggaa agttccggaa gttgctaaag tagatgctgc aaataagaaa 3360

ggaccagatg ttagagtttc tgcattaacc ggagttggtt tgaaggagtt gctgtatctt 3420

attgatgaca aaatgaaaga gaagaagctc aagtctccga ctatagtcga aaggagtgag 3480

cttcataagc gtaaatggag gccacctcgt aacgatgatg aagaggagag attaatcccg 3540

ttagatcaac gttga 3555

<210> 22
<211> 2337
<212> DNA
<213> Arabidopsis thaliana

<400> 22
atgaacattc tccgacctcc gacgtcatca tcatcttcgt cgtttcctcc atacccaaag 60

cccgtttcat taacccctcc ggtatctttc actctcatcc acaaccccat aaacctctgc 120

tctataaacc caccattcac caacgctggt cgaccaattt tccaacggtc cgcctccggc 180

actgctaata gctccgccga agacctctcg tctttcttgg gctctccctc agaggcgtat 240

tcaacacaca acgaccaaga gcttttgttt ctcctccgca atagaaaaac cgatgaagct 300

tgggctaagt atgttcaatc cactcatctc cctggaccaa cttgtcttag ccgtttagtt 360

tctcaattat cttatcaatc caaacccgag agtctcacgc gcgcacaatc tatcctcacg 420
cgcctccgca atgaacgcca gctgcatcgc cttgacgcta attccctcgg tctcctcgcc 480
atggctgcag cgaagtctgg ccaaacactt tacgccgtct ccgtcatcaa gtccatgatt 540
cgttctgggt atttacctca tgttaaagcg tggacagctg cagtagctag tctctctgct 600
tccggagatg atggtccgga agaatctatc aaactcttca tcgctattac gcgacgagtc 660
aaacgatttg gtgaccagtc tttggttggt caatctaggc ctgatacggc ggcatttaat 720
gcggtgctta acgcttgtgc taaccttggt gatactgaca agtattggaa gttgttcgag 780
gaaatgtctg agtgggattg tgagcctgat gtcttgactt acaatgttat gattaagctt 840
tgtgcgaggg ttggtcggaa ggaattgatt gtgtttgtgt tggaaaggat tattgacaag 900
gggattaagg tttgtatgac tacaatgcat tctcttgttg cagcttatgt tgggtttgga 960
gatttgagaa ctgctgagag gattgttcaa gcgatgaggg agaaaaggag agatctttgt 1020
aaggttctac gagaatgcaa cgctgaggat ttgaaggaga aagaagagga agaagcagaa 1080
gatgatgaag atgcgtttga ggatgatgaa gactcgggtt attcggctcg ggatgaggta 1140
agtgaagagg gggttgtaga tgtgttcaag aaattgctac ctaactcggt tgatccgagt 1200
ggtgagccac cattgttgcc taaagtcttt gcaccagact caaggatcta cacgacgttg 1260
atgaaaggtt atatgaagaa tgggcgtgtg gcagacacag ctagaatgct tgaggcaatg 1320
aggcgtcaag atgatagaaa cagtcaccca gatgaagtta catacactac ggttgtgtca 1380
gcttttgtaa atgcagggtt gatggataga gcaagacaag tgttagccga gatggctcgg 1440
atgggtgttc ctgcaaatag gattacttat aatgttctgc tcaaaggata ttgtaagcag 1500
ttgcagatag atagggcaga ggatttacta agagagatga ctgaagatgc ggggatcgag 1560
ccagacgtgg tttcctataa cattataata gatggatgca ttcttataga tgatagcgca 1620
ggagctctag cgtttttcaa tgaaatgaga acgagaggga ttgcaccaac taagattagt 1680
tacacaactt tgatgaaggc ttttgcaatg tcggggcaac ccaagttggc gaatagggtg 1740
tttgatgaga tgatgaatga tccaagggtc aaagttgatt tgatcgcgtg gaacatgttg 1800
gttgaagggt actgcaggct aggtttgatt gaggatgctc agagagtagt gtcaagaatg 1860
aaagaaaacg ggttttaccc aaatgtggca acctatggga gtctagccaa tggggtttcg 1920
caggcgagga aacctggtga tgctctcttg ctttggaagg agataaagga aaggtgtgcg 1980
gtgaaaaaga aagaagcacc ttcagattct tcttcagatc ctgctcctcc gatgctgaaa 2040
ccagatgaag ggttgttaga tacactagcg gatatatgtg tcagggctgc ttttttcaag 2100
aaggcattgg agataatcgc atgtatggag gagaatggga tacctccgaa taagactaag 2160
tacaagaaga tctatgtgga gatgcactcg aggatgttca ctagcaaaca tgcttcacaa 2220
gccagaatag ataggcgggt agaacgaaag agagcggctg aagctttcaa gttttggctc 2280
ggtttgccta attcttatta tggaagtgaa tggaagttag gtccaagaga agactag 2337

<210> 23
<211> 1365

<212>  DNA
<213>  Arabidopsis thaliana

<400>  23
atgaacccaa cccaaaaacc cgaaccggtt tacgatatgg tcatactcgg agcatccgga      60

tttaccggta agtacgtcgt cagagaagct ctcaagttcc ttcaaacacc gtcttcttct     120

ccgttaaagt ctctagcttt agcgggtcgt aacccgaccc gtttaaccca atctctcgaa     180

tgggccgccc gcccgaaccc accaccttcc tctgtcgcta tcctcactgc tgatacatct     240

gaccctgatt cacttcgtcg tctctgtact caaaccaaac tcatcctcaa ttgtgttgga     300

ccgtttcgta tccatggtga tcctgtcgtc tctgcttgtg ctgattcagg gtgtgattat     360

ttggatataa gtggtgaacc tgagtttatg gagagaatgg aagctaacta ccatgataga     420

gcagaagaga ctggctcttt aatcgtttct gcttgtggtt ttgattcaat tcctgctgaa     480

ttgggtcttc tctttaatgc taaacaatgg gtatctccat cggttcctaa ccagattgaa     540

gcgtacctta gcttggagtc tgacaaaaaa attgctggga actttgggac ttatgagtct     600

gcggttttag gtgttgctaa tgcagaaaag cttaaagaat taagacgttc aagaccaaga     660

aggccaagac caacgatttg tggtcctcct gctaaaggac caacattaga aaaccagaag     720

acgattggtc tttgggcttt aaagctacct tcagctgatg cagtagttgt tcgtagaact     780

ctcacaactc taacagagaa accacatggg cttcctggga ttaatgaaag tcctgagcag     840

atacaaaaga gagaagcatt ctggtcatcg atcaagcctg ctcattttgg tgtaaagata     900

acgtccaaat ctctctttgg gatattccga tatgttacac ttggagtgtc acttggttta     960

ctttccaagt tctccttcgg aagatggctt cttttgaaat tcccttcagt tttcagcctt    1020

ggttggttcc agaagaaagg tccaagtgaa gaagaggtag aaagcgctac gtttaagatg    1080

tggttcatag gtcgtgggta cagcgaagag agtctagctt cacaaggaga aacaaagcct    1140

gacttggaaa tcattacaag aatttcagga cctgagattg gatatataac caccccgata    1200

acacttgttc aatgcggttt gatagtcttg ggccagcgcg aaagcctagt taaaggagga    1260

gtctacacac ccggcattgt gtttggttca accgatatcc agcagcgact tgaggataat    1320

ggtatatctt ttgagctgat ttcaaagatc aagactcaag gataa                    1365

<210>  24
<211>  1398
<212>  DNA
<213>  Arabidopsis thaliana

<400>  24
atgacaccgg ctattttttc tccgacgact cttcctccat caactgctac atggccatgt      60

tcaacatctc agaagctcat caccgttaga tcaccactca agttcaagtg tagagcaact     120

tcatcatcat cgtctatcac tgactttgat ctttatgacc tcttgggtat tgatcgaagt     180

tctgataagt ctcagatcaa atcagcctat cgtgcgttgc agaaacgatg tcatccagat     240

atcgcaggag atcccggtca tgatatggcc atcattctta acgaggctta ccagcttctc     300

tctgatccga tctcgcgcca agcctatgac aaggagcaag caaaactaga agaactcaga     360

ggctatacag ggaaaccgat atactcggtt tggtgtggac cagaaacaga gcaacgagct 420

gcgtttgtgg acgaggttaa gtgtgttggg tgtttgaagt gtgctttgtg tgcagagaaa 480

acatttgcta ttgaaactgc ttacgggaga gcgagggttg ttgctcaatg ggctgatcct 540

gaatccaaaa tcaaagaagc catcgaagct tgccctgtag actgcatttc aatggtggag 600

agatctgacc ttgctccatt ggagttcctt atgtcaaagc aaccacgagg caacgtgagg 660

atcggggttg gaaacacggt tggtgagcgt gtctccaatg tatttgttga tgtcaagaag 720

ttccaagaaa gatacgctaa agctatgagc agaaccacaa aagagacctc ccagagagaa 780

gtacaaataa gtgcagtaga ggcgattagg tccatttcca attggctata ctggagatca 840

tcaccgtaca cgaaaccatt gagtccagaa tcaaacatga gtctaacttt taccaaaaga 900

aagaaagctg ttgatccaga tatcagaaag cttcaagatg ttgtggcagc aatgaaacaa 960

gcagaccaaa gcggaagaac caaagagaaa ggatcagctt acttgcttgg agaagattac 1020

tggagtccat caaacgctgc tcttccctca tctggaaaca acaacggttc caaagctagc 1080

tcgaatccgc aagtgactcg taagacattt ccttcagaag agaaaccaac tagtagaaga 1140

gaaaatagaa gacagttcag gataaagaaa tttccaattg ggacagccat agtagcagta 1200

ttcttggttc agtaccaagc aagttacaga gccgcctctg agctcaacga ccatatcggc 1260

ggctcgctgg ctttatccat agttaacagt ccatggcagc agatattgtt agcaggagtt 1320

acatggtact tcattggagc aatgttactc caacttgtgg aagctgttca acacaagcta 1380

gaagataaag aaacataa 1398

<210> 25
<211> 3522
<212> DNA
<213> Arabidopsis thaliana

<400> 25
atggctagtt catcttcatc tgagagatgg atcgatggtc ttcagttctc ttccttgtta 60

tggcctccgc cacgagatcc tcaacaacat aaggatcaag tcgttgctta tgttgaatat 120

tttggtcaat ttacatcaga gcaattccca gatgacattg ctgagttggt ccggcatcag 180

tatccatcaa ctgagaagcg acttttagac gatgtgctgg cgatgtttgt ccttcatcat 240

ccggagcatg gtcatgcagt cattcttcca atcatttcat gtcttattga tggctcgttg 300

gtgtacagca aggaagctca tccgtttgcc tctttcatat ctttagtttg cccaagtagt 360

gagaatgact attcggagca atgggctttg gcatgtggag aaatccttcg cattttgact 420

cattacaacc gtcccattta taaaactgag cagcaaaatg gagatacaga gagaaattgt 480

ctgagcaaag ctacaactag tggttctccg acttcagagc ctaaggctgg atcaccaaca 540

cagcatgaaa ggaaaccttt aaggcctttg tctccatgga tcagtgatat actacttgct 600

gctcctcttg gtataagaag tgactatttc cgatggtgta gtggtgtaat gggtaaatat 660

gctgctggag agctcaagcc gccaaccatt gcttctcgag gatctggtaa acatcctcaa 720

ctgatgcctt caaccccaag atgggctgtt gctaatggag ctggtgtcat actgagtgtt 780

tgtgatgatg aagttgctcg atatgagact gctacgctga cagcggtcgc tgtccctgca 840
cttcttcttc ctccgccaac gacatcctta gatgagcatc tagttgctgg ccttccagct 900
cttgaaccat atgcacgttt gtttcataga tactatgcca ttgcaactcc aagtgctacg 960
cagagacttc ttcttggact cttagaagca ccaccgtcgt gggctccaga tgcacttgat 1020
gctgctgtac agcttgtgga actccttcga gctgctgaag attatgcatc tggtgtaagg 1080
ctacccagga actggatgca tttgcacttc ttgcgggcta taggaattgc tatgtctatg 1140
agggcaggtg ttgctgctga tgctgcagcc gctttgcttt tccgcatact ctcacagccg 1200
gcactgcttt ttcctccgct aagtcaagtt gagggagtag aaattcagca cgcgcctatt 1260
ggtggctaca gttcaaatta cagaaaacag atagaagttc ctgcagcaga agcaaccatt 1320
gaagccactg cccaaggaat tgcctcaatg ctttgtgctc atggtcctga agttgagtgg 1380
agaatttgca ctatatggga agctgcttat ggtttgatcc ctttaaattc ttcggcggtt 1440
gatcttcccg aaatcatagt tgctacccca ctgcaacctc ctatcttgtc atggaattta 1500
tacattccac tcctcaaagt acttgaatat cttccacggg ggagtccttc ggaagcatgc 1560
ttgatgaaaa tatttgttgc cactgtggaa acaatactca gtagaacttt tccgcctgaa 1620
tcttccaggg aactaaccag aaaagctaga tcgagtttta ccacaagatc agcgaccaaa 1680
aatcttgcta tgtctgagct tcgtgctatg gtccatgctc tctttttaga atcatgcgct 1740
ggtgtggaat tagcttcacg cctacttttt gttgtgttga ctgtatgtgt tagccatgaa 1800
gcacagtcta gtggtagcaa gagaccgaga agtgaatatg ctagtactac tgaaaatatt 1860
gaggcgaatc aacctgtatc taacaatcaa actgctaacc gtaaaagtag gaatgtcaag 1920
ggacagggac ctgtggcagc atttgattca tacgttcttg ctgctgtttg tgctcttgcc 1980
tgtgaggttc agctgtatcc tatgatctct ggtgggggga acttttccaa ttctgccgtg 2040
gctggaacta ttacaaagcc tgtaaagata aatgggtcat ctaaagagta tggagctggg 2100
attgactcgg caattagtca tacgcgccga attttggcaa tcctagaggc actcttttca 2160
ttaaaaccat cttctgtggg gactccatgg agttacagtt ctagtgagat agttgctgcg 2220
gccatggttg cagctcatat ttccgaactg ttcagacgtt caaaggcctt gacgcatgca 2280
ttgtctgggt tgatgagatg taagtgggat aaggaaattc ataaaagagc atcatcatta 2340
tataacctca tagatgttca cagcaaagtt gttgcctcca ttgttgacaa agctgaaccc 2400
ttggaagcct accttaagaa tacaccggtt cagaaggatt ctgtgacctg tttaaactgg 2460
aaacaagaga acacatgtgc aagcaccaca tgctttgata cagcggtgac atccgcctca 2520
aggactgaaa tgaatccaag aggaaaccat aagtatgcta gacattcaga tgaaggctca 2580
gggagaccct cagagaaggg tatcaaagat ttcctcttgg atgcttctga tcttgcgaat 2640
ttcctcacag ctgatagact cgcagggttc tattgtggta cacaaaagct tttgaggtca 2700
gtgcttgcag agaaaccgga gctgtctttc tccgttgttt cactgttatg gcacaaactg 2760
attgctgctc ctgaaatcca gcccaccgca gaaagcacct ctgcgcaaca aggatggaga 2820
caggttgttg atgcgctatg caatgtcgta tctgcaacgc cagcgaaagc agcagcagca 2880

gttgtccttc aggctgaaag ggagttgcag ccttggatcg ccaaagatga tgaagaaggc 2940

caaaaaatgt ggaaaatcaa ccaacggata gtcaaagtgt tggtggaact catgcgcaat 3000

catgacaggc ctgagtcact ggtgattctc gcaagtgcat cagatcttct tctgcgggca 3060

actgatggaa tgcttgttga tggagaagct tgtacattac ctcaacttga gctacttgaa 3120

gccacggcaa gagcaataca gccggtgcta gcttgggggc catctggact agcagtggtc 3180

gacggtttat ccaatctatt gaagtgtcgt ctaccagcaa caatacggtg cctttcacac 3240

ccaagtgcac acgtacgtgc cttaagcacg tcagtactac gtgatatcat gaaccaaagc 3300

tccataccca tcaaagtaac tccaaaactg ccaacaacag agaagaacgg aatgaatagt 3360

ccgtcctatc gattcttcaa cgccgcctca atagactgga aagccgatat ccaaaactgt 3420

ttaaactggg aagctcacag cttgctctcc acaactatgc ctactcagtt tctcgacact 3480

gcggctcggg aactcggctg tactatatcc ttgtcccaat aa 3522

<210> 26
<211> 1989
<212> DNA
<213> Arabidopsis thaliana

<400> 26
atgcagtttc ttcgacttct tacacttctt gtttcttcct acttcttctt cttcatcaac 60

ttctcctcct cactgaatcc agatgggttg tctctacttg ctctcaaatc cgcaatctta 120

cgagacccga cacgtgtaat gacttcctgg tctgagtccg acccgactcc atgtcactgg 180

cctggaatca tctgcacaca tggccgagtc acctcactcg ttctctccgg aagaagactc 240

tcaggttaca taccctctaa actcggtcta ctcgactcac tcataaaact cgaccttgct 300

cgtaacaatt tctcaaaacc agtgccgact cgtctcttca acgccgttaa tctccgttac 360

attgatctct ctcacaactc aatctccggc ccaattccgg cccaaatcca atccctcaag 420

aatctcactc acattgattt ctcctccaat ctactcaacg gttcactccc tcagtcactc 480

actcaactcg gaagcttagt cggcacactc aatctctctt acaacagttt ctccggcgaa 540

attccgccgt cgtatggccg ttttccagtc tttgtcagct tagatctcgg ccacaataat 600

ctcaccggaa aaatacctca gattggctct ctcttaaacc aaggaccaac agcgttcgcc 660

ggaaactctg agctctgtgg tttcccatta cagaagctgt gtaaagatga aggtacgaac 720

cctaagctcg tcgctccaaa accagaaggc tcgcaaatcc tcccgaagaa accaaaccct 780

agcttcatcg acaaggacgg aagaaagaat aaaccgatca ccggatccgt aacggtttct 840

ctcatctccg gagtctcaat cgtaatcgga gcagtttcta tctccgtatg gctgattcga 900

agaaaattaa gctccactgt gagtacaccg gaaaaaaaca acacggcggc gccattggat 960

gatgcggcgg atgaggagga gaaggaaggt aaattcgtgg tgatggacga aggattcgag 1020

ctcgagctcg aggatttgct gagagcatcg gcttacgtcg tcggaaagag cagaagtggg 1080

attgtgtaca gagtagtggc cggaatggga tcaggtacag tggcggctac gtttacgtca 1140

tccaccgtcg ttgctgtgag aaggctaagc gacggagatg ccacgtggcg gcggaaggat 1200

ttcgaaaatg aagtggaggc tataagtaga gtccaacatc caaatatcgt acggctaaga 1260

gcttattact atgctgagga cgagaggctc ttgatcactg attacatacg caacggcagc 1320

ttgtactctg ccttacatgg tggaccctcg aatactctgc cttcactctc ttggcctgaa 1380

agattactta ttgcacaagg aacagctcgt ggcttgatgt atatacatga gtacagccca 1440

agaaagtacg ttcatggcaa cctgaaatca accaaaatcc tgcttgatga tgaattactg 1500

cctcgcatct caggcttcgg tcttacacgt ttggtatcag gttactccaa actcatcggt 1560

tcgctatccg ccacaaggca aagcttagac caaacctact taacctctgc tacaacggtg 1620

acaagaatca cagctcccac tgttgcttac cttgcacctg aggctcgggc ttcttctggt 1680

tgcaaattat ctcagaagtg cgatgtctat tcgtttgggg ttgtcctaat ggagttgttg 1740

actggccgtt tgcccaatgc ttcctctaaa aacaatggcg aagaactcgt gcgtgttgtg 1800

aggaactggg tcaaggaaga gaagccgttg agtgagattt tagacccgga gattctgaac 1860

aaaggtcacg cagataagca agttattgca gccattcatg tcgccttgaa ctgcacggaa 1920

atggatccag aggttcgtcc gaggatgaga tcagtgtctg agagtctcgg ccggatcaaa 1980

tcggactga 1989

<210> 27
<211> 942
<212> DNA
<213> Arabidopsis thaliana

<400> 27
atggcggcga cgtcgctggt tctgacgtgc gcatcccctc tattcagcag ccctcgggtt 60

atttctgcta cgaagaagct gactacagag ttgtcgattt ctacagctaa attccgaaga 120

agatgctcgg gaaacaatga tgaagtgctt ctagaaggaa tgccaccgga gtattacgat 180

gatgaatggc aagctcgaca gagagagaag accaaagaac tgcggcggat gcagcgggag 240

gaagaagaag aagaggagag aaagattgaa gaataccgtg aaattggcac gaggttgaag 300

gaatttcccg agcaagactt aaggaaagcc agaaagctcg tctccagctt catcagagct 360

gccgaggaag tcgaagagag aattgaagaa gcagccgaga aaggagaact tgacgagctt 420

gtcctcatga tcatatggaa ccggcttgac cttgctaggc gcgatgatga gaaggacgcc 480

atcagaagtc ttgatctttt gtatagaaga gtcgagacag agatcttaaa acggcaagca 540

agtcctgcaa tgaaactgct gaatgatctt ctaaatatgc atgatggctt tgaagacgat 600

gcttggctca aggactgcag aaaacgaatg gctgagacct tcccccgaga agaccccttc 660

agcattctaa tgccaccggg attcgacatt gatatgcatc aaggacagtt gcgaccgccc 720

attgagactg agacagacaa cacccttctg agagtagact ttgtaagaga agtggatgca 780

ctgctacagg aagtgaggat agaggaagac gctacaactg gtagcaaagg agaagggctt 840

gatcctgaag ctatagcact taagtttaag caacaggaga agcaacgaac catccgccaa 900

attgaagcca ttcttgattt agccctcaac ttgaagtggt ag 942

<210> 28
<211> 1278
<212> DNA
<213> Arabidopsis thaliana

<400> 28
atggagaaaa tgaatgtccg ttttatgatt gtgttgatgg taatgtctct ggttctgggt 60

ttttcgtcgg cagttgattt cagatggagg aaaactgcag gattctcaga tagattcacc 120

agagctgttt cttcagtcgt gttcccagtt catggcaacg tttatcctct tgggtactat 180

aatgtaacca tcaacatagg acaaccacca agaccttatt atcttgatct tgatactggt 240

agtgatctca cttggctcca atgtgatgct ccttgtgttc gttgcttgga ggcgcctcat 300

ccactgtatc agcctagtag tgatcttatt ccttgcaatg atccactgtg taaggctttg 360

catttgaata gtaatcagag atgtgagact ccagagcaat gtgactatga ggttgagtat 420

gctgatggag gatcttctct cggtgttctt gttagagatg tcttctctat gaactataca 480

cagggtctcc ggctcactcc ccgtcttgct ctaggttgtg gatacgatca aatcccaggg 540

gcttcgagtc atcatcctct agatggagta ttagggcttg gtagggggaa agtaagcatt 600

ctgtcacagc ttcatagcca aggttatgta aagaatgtta tcggtcattg cctaagcagt 660

ttaggtggag gaattctctt ttttggcgac gatctttatg attcttcaag agtctcatgg 720

acaccaatgt ctcgtgaata ctcaaaacac tactctcctg caatgggagg ggaacttcta 780

ttcgggggaa gaacaacagg attgaagaat ctattaacag tatttgacag tggaagttct 840

tacacatact tcaattccaa ggcataccaa gccgtaacat atttgctaaa gagagaacta 900

agcggaaaac cgttgaaaga agcacgggat gaccacacgc tgcctctatg ctggcaagga 960

cgtagaccat tcatgagcat tgaagaagtc aagaagtatt tcaagcctct agctcttagc 1020

ttcaaaacag gctggagatc aaaaactctg tttgagatac ccccagaagc ttatctaatc 1080

atttctatga aggggaatgt atgtttggga atcttgaatg gcacagaaat aggtctccag 1140

aacctaaacc tcatcggcga tatatcgatg caagatcaga tgataatcta cgataacgag 1200

aaacaatcaa tcgggtggat gccagtggat tgcgatgaac ttgcttcact aaaagcagct 1260

caagtatatg aatactga 1278

<210> 29
<211> 1059
<212> DNA
<213> Arabidopsis thaliana

<400> 29
atgcattgcg gatgtgtatt cgcttcacaa actgtgtcat cacttctccc atttgaaatt 60

aaaacctatg cgtcaaagct tcgagcttcg tcagcccaat taccgagaac ccagattcag 120

ataaaccctt cagatgatct ctctatctac ggttcagata aatctccggc gaatagagtt 180

tcgttgccgt ctcatgtaaa ttctatcacc agtactacaa acccttttgt caaacactgc 240

ttgaagctcc gccaaagttc ctcgtatcgc cacgctcatg gctctgttct tgtcgtcgga 300

actatcccca tcagggaagt atgtatgttt caaacgaata agcaaggaat gaccactgaa 360

attgagtgcc tacttcttca tgaggaagct aagattccac aaggattaga gagtctaagt 420

atccgaattg ttagagtaag ttctttagtg atgaagaaac tctctggagt gcaatctact 480

gaatctgttg aagccattgc cttgatgaga atccctagca gctttactga tcttaaagat 540

gataaagaca tcataacaga ctgcaacaaa tggttccctt ctgctcacag agttcttgtt 600

ctggacagca tacaggatcc agggaacctt ggcacattag tcagatcagc tatggctttt 660

aattgggatg gtgcatttct acttccgggt tgttgcgatc cgtacaacga caaagctctt 720

cgagcaagcc gaggtgcttc gtttcagcta cctatagttt ccgggaattg gaaccatctt 780

aagcttctag aaaatgagtt ccagatgaag ctattagctg gtcatccagc aacgactact 840

cagaaactga aacctgtctc caaactttcg gtagagtttg ctcaatcttt agcagagaag 900

cctttatgct tgattttagg tagtgaaggg aatggtttgt ctgagcaggc acggaaagta 960

tgcgtgctag tgagcattcc catggcaggt gactttgaat ctcttaacgt ctctgttgct 1020

ggtggtattt tcttgtacat gcttcaaaat cttgtttag 1059

<210> 30
<211> 1038
<212> DNA
<213> Arabidopsis thaliana

<400> 30
atggctgcga gcgatgaagt taatcttatt gagagcagaa cagtggttcc tctcaataca 60

tgggttttaa tatccaactt caaagtagcc tacaatatcc ttcgtcgccc tgatggaacc 120

tttaaccgac acttagctga gtatctagac cgtaaagtca ctgcaaacgc caatccggtt 180

gatggggttt tctcgttcga tgtcttgatt gatcgcagga tcaatcttct aagcagagtc 240

tatagaccag cttatgcaga tcaagagcaa cctcctagta ttttagatct cgagaagcct 300

gttgatggcg acattgtccc tgttatattg ttcttccatg gaggtagctt tgctcattct 360

tctgcaaaca gtgccatcta cgatactctt tgtcgcaggc ttgttggttt gtgcaagtgt 420

gttgttgtct ctgtgaatta tcggcgtgca ccagagaatc catacccttg tgcttatgat 480

gatggttgga ttgctcttaa ttgggttaac tcgagatctt ggcttaaatc caagaaagac 540

tcaaaggtcc atattttctt ggctggtgat agctctggag gtaacatcgc gcataatgtg 600

gctttaagag cgggtgaatc gggaatcgat gttttgggga acattctgct gaatcctatg 660

tttggtggga atgagagaac ggagtctgag aaaagtttgg atgggaaata ctttgtgacg 720

gttagagacc gcgattggta ctggaaagcg tttttacccg agggagaaga tagagagcat 780

ccagcgtgta atccgtttag cccgagaggg aaaagcttag aaggagtgag tttccccaag 840

agtcttgtgg ttgtcgcggg tttggatttg attagagatt ggcagttggc atacgcggaa 900

gggctcaaga aagcgggtca agaggttaag cttatgcatt tagagaaagc aactgttggg 960

ttttacctct tgcctaataa caatcatttc cataatgtta tggatgagat ttcggcgttt 1020

gtaaacgcgg aatgttaa 1038

<210> 31

<211>  4134
<212>  DNA
<213>  Arabidopsis thaliana

<400>  31
atgaagcgaa ttagggatga tatttacgca accgggtctc aatttaaacg tcctttgggc      60

tcttctcgtg gcgaatcata tgagcaatct ccaatcactg gaggagggag cattggtgaa     120

gggggaatca acactcagaa attgactacc gatgatgctt tgacctactt aaaggaagta     180

aaggagatgt ttcaagatca gcgagacaaa tatgatatgt tccttgaggt tatgaaagac     240

tttaaggcac aaaagactga tacatctggt gtgatttcac gagtgaagga gctgtttaag     300

gggcataaca atttgatttt cgggtttaac acctttttgc ctaaggggtt tgaaataacg     360

cttgatgatg tagaagctcc ttcaaagaaa actgttgaat ttgaagaagc cataagcttt     420

gttaataaaa ttaagacacg gttccagcac aatgaacttg tctataagtc gtttctggaa     480

atcttaaata tgtatcggaa ggataataag gacatcactg aggtttacaa tgaggtgtct     540

actctttttg aggaccactc ggatttgctt gaagagttca ctaggttttt accagactcg     600

ttggcgcctc atacagaagc ccagttactt cgtagtcaag cccaacggta tgatgaccgg     660

ggatcaggcc ctcctcttgt gcgtcgaatg tttatggaga aggatcgccg acgagaaaga     720

actgttgctt ctcggggtga tcgtgatcac agtgttgacc gttctgacct taatgatgat     780

aaatcaatgg ttaagatgca cagagatcag aggaaacgtg ttgataagga taatagagaa     840

aggagaagcc gtgatttgga agatggagaa gcagagcaag ataacttgca acatttttca     900

gagaaaagga agtcctcgag aagaatggag gggtttgaag cttattctgg tcctgcttca     960

cattctgaga aaaacaatct aaaaagcatg tacaaccaag catttttgtt ttgtgagaaa    1020

gtcaaggaga gattatgcag ccaagatgat tatcaagcat tcttgaagtg tctcaatatg    1080

tttagcaatg gaattatcca aaggaaagat ctgcagaatt tggtttccga tgtgcttgga    1140

aaattccctg atctcatgga tgagttcaat cagttctttg agcgttgtga gagtattgat    1200

ggtttccagc accttgctgg tgttatgagc aaaagtaggc agcagtctcc tagcttcttg    1260

tctatgagta tacttttctc ttttttttcg tacgttatag gaatagaaat aacactgcct    1320

ggtacacttg ctgcagaatc acttggtagt gaagaaaatt tatctagatc agtgaagggg    1380

gaggaaaaag atagagaaca caaacgtgac gttgaggctg ctaaggaaaa agagcgatcc    1440

aaggacaagt acatggggaa atctattcaa gagcttgatc tatctgattg tgagcgttgc    1500

actcctagct accggcttct ccctccagat tatccaatcc cgtctgtgcg ccacagacag    1560

aaatcaggag ctgctgtgtt aaatgatcac tgggtttctg tcacttcagg aagtgaagac    1620

tactctttta agcacatgcg caggaaccaa tatgaagaaa gcttgtttag atgtgaagat    1680

gatagatttg agttggacat gctgttggaa tctgtgggat ctgctgccaa aagtgcagaa    1740

gaattgttga atattatcat tgataagaaa ataagttttg agggctcctt ccggattgaa    1800

gaccatttca cagcactaaa tctaaggtgt atagagagac tttatggaga ccatggtctt    1860

gacgtgacag acttaatacg taagaatcca gctgctgcac ttcctgtaat tctaactcga    1920

ttgaagcaga aacaagatga atggacaaaa tgccgtgaag gttttaatgt ggtctgggcg 1980
gatgtgtatg cgaaaaacca ttacaaatca cttgatcacc gcagcttcta ttttaagcag 2040
caagattcta agaatttgag tgcaaaagcg ctggtgtctg aagtcaagga cttgaaagag 2100
aagtctcaga aagaagacga tgttgttctg tctatttctg ctggttacag gcaaccgata 2160
attcctcacc tcgagtatga ctatctcgac agagctattc atgaagacct gttcaaacta 2220
gtccaatttt cttgtgagga gatatgttct acaaaagagc agactggtaa agttctgaag 2280
ctctgggcta attttttgga gctgatgctt gatgttgcac ccagggccaa ggggtcagat 2340
tctgttgaag atgttgtaga aacccagcat cagcgtgcat ttaccagtgg ggaggctaat 2400
gagagttctg atgcgataag tttggtttct aggcaactaa aatttgctac caatggagat 2460
gtgcatgctt catctggggt ctccaagcat ggtgagactg gtttgttgaa tagggattct 2520
tcagggaaag aaaatttgaa ggatggtgat cttgctaata aagatgttgc cacctgtgct 2580
gaaaaacccc aaaaagatca agaaattgga aatggagctg ctaaaagatc tggagatgtt 2640
gatgaaagag tggccacttc aagttcgtct ttcccaagtg gggtcgaaaa caataatggt 2700
aaagtaggaa gcagagattc gtcaggttca cggggcatat tatccaaacc aagtgaagct 2760
atagataaag ttgatagcat tcaacatacg cagggagttg atataggccg aattatagtc 2820
ttaggaaatg gtctgcagtc agatacttct aaagccaaca gtaattatga tgaatcgggt 2880
ggtccatcca aaattgagaa ggaggaaggt gaattatcac ctgttggtga ttccgaagac 2940
aactttgttg tttacgaaga tcgtgagttg aaggctactg cgaaaacaga acattcagtt 3000
gaagctgaag gagaaaatga tgaggacgct gacgatgagg atggtgatga tgcttccgaa 3060
gctggtgagg atgcttcggg aactgaatct attggtgacg aatgttcaca ggacgataat 3120
ggcgttgagg aagagggtga gcatgatgag attgatggta aagctgaaag tgaaggagag 3180
gcagagggaa tggagtcaca tcttatagaa gacaaagggt tgtttccgtc atcagaacgt 3240
gttctattat cagttaagcc tctgtcaaaa catatagctg cagcggcttt ggttgatgag 3300
aaaaagaagg attccagagt attctatggg aatgacgact tttatgtcct tttcaggctt 3360
catcgagtga gtgcaattga ttcttatgat ttgctttctc acatcctgta cgagagaatt 3420
ctgtctgcga aaacatattg ctccggcagt gaaatgaaac tgagaaacac taaagatact 3480
tgttcaccag atccttatgc aaggtttatg aatgctctgt ttagtctgct taatggctca 3540
gctgaaaatt ccaagtttga ggatgaatgc cgagctatta ttggaaacca gtcatatgtt 3600
ttattcactt tggaaaaact gatatacaaa ttggttaaac agcttcaagc tgttgtagct 3660
gacgacatgg acaataagct tcttcagttg tatgagtatg agaattcccg gagacctggg 3720
aggtcttcct ctccatctcg tttgtcaatc cagcttatgg ataacataat tgaaaagccc 3780
gacgcttatg cagtctccat ggagcccaca tttacgagtt atttgcaaaa tgagtttctc 3840
tccaactcat cagggaagaa agagctacag gacattgtgc tacaaaggaa catgcgtgga 3900
tacaatggtc tggatgatct tgcagtagct tgcaaggcca tggaaggtgt acaagtaatt 3960
aatggccttg aatgcaagat gtcttgctct tcctacaaga tttcgtatgt tttggacaca 4020

gaggatttct tccacaggaa gaagaaacag aagaagagca acaacttgtc actggctaaa 4080

ttatcacaga atagaatagc aagattccac aagtttctct cagcttcaag atga 4134

<210> 32
<211> 2976
<212> DNA
<213> Arabidopsis thaliana

<400> 32
atgcttcagc caagtcctcc tcactactct tcctctagag atgtcagaca tcatcatcat 60

catcatcatc atcatcatca tctagctctg agttctaaag ctagggtttt tccactttca 120

cttccctgta acttctcctc tagggtttct tttaagcttc aacttcactg cgccgcttct 180

tcctcttctt cagtttctcc acctcgatgc tctaaaccta acccaagctc tcgaaaacgc 240

aaatatggcg gcgtaatccc ttccattttg cgttctcttg actcttccac tgatattgaa 300

acaactctag cttctctttg tctcaattta agccctaaag aacaaactgt tcttcttaaa 360

gagcagactc gttgggaaag agttcttcgt gtgtttcgat ttttccagtc tcaccaaagt 420

tatgttccta atgtgattca ttacaacatt gtgttaagag ctttagggag agcggggaaa 480

tgggatgaat tgaggctttg ttggattgag atggctcata atggtgtttt gcctactaac 540

aatacttatg gtatgcttgt tgatgtttat ggtaaagctg gtcttgttaa ggaagctctt 600

ctttggatta agcatatggg acagagaatg catttccctg atgaagtcac tatggctact 660

gttgttagag ttttcaagaa ctccggtgag tttgatcgtg ctgataggtt ctttaaaggt 720

tggtgcgctg gaaaagttga tcttgatttg gattctattg atgatttccc taagaatggt 780

tcagctcaat ctcctgtgaa cttgaagcag ttcttgtcga tggagctttt taaggttggt 840

gcaaggaatc ctattgagaa aagtctgcat tttgcatctg gttcagactc ttctccgagg 900

aagccaaggt taacttccac cttcaacact ctgattgatt tgtatggaaa ggcaggtcgt 960

ttaaacgatg ctgctaatct cttctcggag atgttgaaat ctggagtacc tatagatact 1020

gtaacgttta acacgatgat acatacttgt ggaactcatg ggcatttgtc agaggctgaa 1080

tccttgttga agaagatgga agagaaaggg atatcccctg atactaagac atataatatc 1140

cttttgtctc ttcatgctga tgctggggac attgaggcag ctcttgagta ctataggaaa 1200

attaggaaag taggactttt tcctgatact gtaactcatc gagctgttct tcatatcttg 1260

tgtcagcgga aaatggttgc agaagttgaa gctgtgatag ctgagatgga cagaaatagc 1320

attcgcattg atgagcactc tgttcctgtt attatgcaga tgtatgtcaa tgaaggttta 1380

gtcgtacagg caaaagctct gtttgagagg ttccagttgg attgtgtgct ttcgtcaacg 1440

acacttgcag cagttattga tgtctatgct gaaaagggac tgtgggttga agcggagact 1500

gtgttctatg gaaaaagaaa catgtcaggc cagaggaatg atgttttgga gtacaatgtc 1560

atgatcaagg cttatggtaa ggccaaactt catgagaaag cactttctct cttcaaaggg 1620

atgaagaacc aagggacttg gcctgatgag tgcacttaca attccctatt ccagatgctt 1680

gctggggttg atttagtgga cgaagcccag cggatcttgg ctgaaatgct ggattcgggc 1740

tgtaaacctg gatgcaagac ctatgctgct atgatagcta gctatgtgcg gcttggcctg 1800
ttgtctgatg cagttgacct gtacgaggca atggaaaaaa caggggtgaa accgaatgaa 1860
gttgtttatg gttccttaat taatgggttt gctgagagtg gaatggtcga agaagcgatt 1920
caatacttta gaatgatgga agaacatggc gttcagtcca atcatatcgt tctgacttcc 1980
cttatcaagg cttatagcaa agtggggtgt cttgaagaag ctaggagagt gtatgacaaa 2040
atgaaggatt cagaaggtgg cccagatgtt gctgcatcaa acagcatgct aagtctgtgt 2100
gcagatcttg gcatagtttc tgaagcagaa tccattttca atgctctcag agaaaaaggc 2160
acatgtgatg ttatttcgtt tgcaacaatg atgtacttgt acaagggcat gggcatgctc 2220
gacgaggcta ttgaagtggc tgaagaaatg agagagtctg gtctactaag tgactgcact 2280
tcatttaatc aggttatggc ttgctacgct gctgatgggc agttaagtga atgctgtgaa 2340
ctgtttcatg agatgttagt tgaaagaaag ctcttgctgg attggggaac atttaaaacg 2400
ctcttcacgc tcttgaagaa aggtggggtg ccaagtgaag ctgtgtcgca gctacaaacc 2460
gcatacaatg aagctaaacc actggcaaca ccagcaatca ctgcaactct gttctcagcc 2520
atgggtttgt atgcatatgc gctggaatca tgccaagagc tcacaagtgg tgaaattcct 2580
cgcgagcatt ttgcatacaa cgcagtgata tacacttata gtgcatcagg agacattgac 2640
atggccctaa aggcatacat gagaatgcag gaaaaaggtc tagaaccaga tattgtcacg 2700
caagcctacc ttgttgggat atacgggaaa gcgggaatgg tggaaggtgt gaagagggta 2760
catagccggc tgacgtttgg ggagcttgaa ccaagccaat cgttgtttaa agcagttaga 2820
gatgcttatg tgagtgcaaa cagacaggac ttggctgatg tggtgaagaa agagatgagc 2880
attgcttttg aagctgaaag ggagtgtagt tcaagatctg gagaagaaga agaagacgat 2940
gaagaggaaa attctgaaga agacgaggca ttttga 2976

<210> 33
<211> 2217
<212> DNA
<213> Arabidopsis thaliana

<400> 33
atgggccgat atgagctaca ctatggaggt gatcggcgga ataatgcgcc ggcaatgaga 60
agagattata acggcggatt gatcgccttt tcgagatatt tcagcttctt ttctagcaga 120
acatgttcac cggaatcatc tatcaataac cagtttaggc ttctctgcat cacttgtgat 180
accctgacaa cgacgcacaa tttctctcag cttctcagac aatgcatcga cgaaagatcg 240
atatcaggaa tcaagactat ccaagcccat atgctgaaat ctggttttcc ggccgaaatt 300
tccggcagca aactcgtcga cgcgagttta aagtgtggcg atatcgatta cgcacgacag 360
gtgttcgatg gaatgtctga gagacatatt gtaacatgga actctttaat tgcttattta 420
attaagcaca gaagaagcaa ggaagctgtt gagatgtata gattgatgat tacgaataat 480
gttttgccag atgagtacac gttgtctagt gttttcaagg cgttttcaga tttgagtctt 540
gagaaggaag cacagagaag ccacggactt gctgtgattt tgggtttgga agtctcaaac 600

gtgttcgttg gaagtgctct tgtggatatg tatgtaaagt ttggtaaaac gagggaggcg 660
aagttagtat tggaccgcgt ggaggagaaa gatgtagttt tgatcacagc tttgatcgtt 720
ggttactcgc agaagggtga agatactgaa gctgtgaagg catttcaaag tatgttggtg 780
gagaaagttc agcctaatga gtatacttac gctagtgtat tgatttcttg tggaaactta 840
aaggatatag gtaatggcaa gttgattcat ggacttatgg tcaagtccgg ttttgagtct 900
gcgcttgctt cacaaacttc tcttcttacc atgtatttga ggtgcagttt ggtcgatgat 960
tccttgcggg ttttcaagtg tattgagtac ccaaatcagg tgagttggac gtctcttata 1020
tcagggcttg tccaaaatgg tagagaagag atggctctaa tcgaatttag aaaaatgatg 1080
cgtgattcaa tcaagcctaa ctcttttaca ttgtcaagtg ctctcagggg gtgctcgaat 1140
ctcgcaatgt ttgaagaagg tagacagatt catggtatag tgactaaata tggttttgat 1200
agagataagt atgccggatc agggctcatt gatttatatg ggaaatgtgg atgctcagac 1260
atggcaagat tggtttttga taccttgagt gaagttgatg ttatatcttt gaacacaatg 1320
atatacagtt atgcacagaa cggttttgga cgcgaagcac ttgacttgtt tgagagaatg 1380
ataaatcttg gactgcagcc gaacgatgta acagtcttga gcgtactctt ggcttgtaat 1440
aactctagat tagttgagga aggttgcgaa ctctttgact cctttagaaa ggataagatc 1500
atgttaacaa atgatcatta cgcgtgtatg gtagatttgc ttggacgggc agggagatta 1560
gaggaagcgg aaatgcttac aaccgaggta ataaacccgg atttggttct gtggaggacg 1620
ctgcttagtg cttgtaaggt tcatagaaag gtagaaatgg cagagcggat aacgagaaaa 1680
atcctagaga tagaacctgg ggatgaagga actctcattc taatgtcaaa tctctacgca 1740
tccactggga aatggaacag ggtgattgag atgaagagca aaatgaagga tatgaaacta 1800
aagaagaatc cagcaatgag ttgggttgaa atcaataaag agacgcatac attcatggct 1860
ggagatttgt tttcgcatcc caactctgag cagattcttg aaaatctcga agagctgatt 1920
aagaagtcta aagatttggg atatgtagaa gacaaaagct gtgtgtttca agacatggag 1980
gagactgcaa aagagagatc tctgcatcaa catagcgaaa aactcgccat agctttcgca 2040
gtgtggagaa atgttggtgg aagtataagg attctaaaga accttagagt ttgtgttgat 2100
tgtcacagtt ggatcaagat cgtgtcaaga gttatgaaga gagaaattat atgtagagat 2160
tcaaaaaggt ttcatcattt cagagatggg tcttgttcgt gtggggatta ttggtaa 2217

<210> 34
<211> 2748
<212> DNA
<213> Arabidopsis thaliana

<400> 34
atggcgtcta cagcggctga acaagacgag agaaaaattg tatcggtagc atcgaacgct 60
agccaggaca tcaaaacggc tgctgctgca tcgcggatca gtagccaaaa cggcgcttct 120
ccatctccgt cgctcaactc caaggacttc atcgtctcag cagcagctaa catcgcttct 180
cagccgttac agaactacga ttcgaacgtt tggggagtcc tcaccgcaat ttcaagcaat 240

gctcgcaaac gccggcaggg cataaatata cttttgactt ctgatgagca ttgcttagga 300
cggctgccat gtcacgctag ttatcaggta gaatcaaatg caattagcgg gaatcactgt 360
aaggtattcc gtaagccggt aacaggcggt gatggggatg atgtaactgt ctttatggta 420
gacacaagca caaatggtac gtttctcaat tgggaaaggt taacaaagaa tggccctgaa 480
gtcagggttc aacacggtga catcatatcg cttgctgttc ctccagagca tgagaaggca 540
tttgcatttg tataccgcga agtacttggt aataatcctg cgctgtcctg catgaacaga 600
aaaagaaaag cagaggatac tacttgtgaa attaagaggc agaagggcat aggcatcagt 660
ggtcccaatg gtccaatatc tttggatgat tttaagagcc tccagcgttc aaacacagaa 720
ctgaggaagc aattagaagc ccaggtgctt accattgaca ctctgcgtaa tgagtcccgc 780
tcaattgttg agcaccatga aagtgattat ttgagtatct ctactgaaat atctttgcat 840
ttgcaggaaa taaaacagat aaaagaatcc actgcaaaat catttcataa tgaactgatt 900
gagctacgtg atcaattaga tacgaagcag aaggaactgg cgcaggtcaa caaattatca 960
gctgaacaga agaattccat agatgaactt ggtgagagag taagcgcttc tttgcaaact 1020
ctcagtgaag caaatgaagt aattcaaagt caaaaggcat ctatagctga actgaagacg 1080
gggttggatg aagagagaaa ccaaagaaga gaggaaagag aaactgccat tgctgaactc 1140
aaagctgcga tacatagatg ccaaattgaa gctcaggaag aattgaaaag attttctgat 1200
gctgctatga gacacgagag ggaacaacaa gaagtaatca acaaaatgaa ggagtcagag 1260
aaagaaaagt caatgcaagt cgaaacattg atgtcaaaat tggaagatac aaggcagagg 1320
ttggtgtgtt ctgagaatag aaaccgtctg ctagaagctc aagtttctga ggagcagctt 1380
gcttttgctg atgcacaaaa aaaactggaa gaacttgacc ttcaagtaaa aagactgcaa 1440
aaggatctgg acagtgaaaa ggcagctcga gaagaagcat gggcgaaagt gtctgcctta 1500
gaactagaga taagtgctgc tgttcgagac cttgacgtcg aaagacagag acaccgtggt 1560
gcaagggaaa gaatcatgct ccgtgaaact cagatgcggg cattttattc tacgactgag 1620
gagatctcgg ctttgtttgc aaagcagcag gaacagctca agactatgca gagaactcta 1680
gaagatgagg ataattgtga caatacttca ctagatattg atcttaatcc aataaacaga 1740
agtcccaaca gagctaatac gcagggagat aaaagagcaa cttcccattt gaattttgct 1800
gccagggcaa gctcgtccac ttcagggcaa aggtctacca gaaatgaagt tgtggatacg 1860
tcatgtgagg atgcagatgc tacccaaaag catgattgtg aaatcatgag tcaggaaggc 1920
caaaacaccc aagaagcaga gtatccaagc tctgataaag ttgcaaaggg tggctttggc 1980
tcagatatag aaggtattgg tacagcaccc acttcgggaa cagaccctgt aggaacagag 2040
caagtcaatg aaactcaaag tccaggaaat gattatgaga gaaatgatca tctgaggaag 2100
tctattattt tagctggtga tacaatgcaa atagattgtg aaactcaggt acatgaaagt 2160
gttcagattg aaggagctgt tctcttgtta aggaacccga acgatcgaag ggatactcaa 2220
gacatagagg gagtaggtac tatagggacg tcggatcttc tagcttctga agttgcgggg 2280

agttgggcta atagcacgaa tccttctgta catggagaaa acgaaactga aagaagtaga 2340

gaagatgaag agagtcagac tcaaaaaatc aaggaagtga ccatagtaca ggattctgct 2400

ggtcagatag gggaaagtca aactaaaccg acaagtccag gggtcctggt cactaacaag 2460

gatgatgcag agcgtggagt tattaacgag ccagtgggga tcactgatca agggaagata 2520

aaacatggta ctcgttcgga ctcagagaca gagagttgtt ctgactctga tgatgatcat 2580

gagaaggaaa aacacaatcc tgtctcagat tctgatacag agggttctga tatgaatgat 2640

gacaagggat cactctcgtc ggatcctgat acagaaagaa gccatgaagt tgatggggat 2700

cagaagaaac aagtggacac catggacgaa gacgataaag ctacttag 2748

<210> 35
<211> 2841
<212> DNA
<213> Arabidopsis thaliana

<400> 35
atggcgaata atcctccgca gtcttctggt acccagggtc agcattttgt tcctgcagct 60

tcacaacctt ttcaccctta tggacatgta cctccaaatg ttcaaagtca gcctccacag 120

tattctcagc cgatacagca gcagcagctc tttccagtga gaccaggtca gcctgtgcat 180

attacatcat cctcacaggc tgtatcagtt ccgtatattc aaacgaacaa gattctcact 240

tctggatcta ctcaaccaca gccaaatgca cctccaatga cgggctttgc tacatctgga 300

cctccatttt cttctccata tacttttgta ccatcatctt atcctcagca acaaccaaca 360

tccttggtcc aaccaaattc tcagatgcat gtagctggcg tccctccagc agcaaacact 420

tggcctgttc ctgttaatca aagcacatca cttgtttccc ctgtgcagca gactgggcaa 480

caaacaccgg tcgcagtttc cacagaccca ggaaacttga ctccgcaatc tgcatctgac 540

tggcaggagc atacatctgc tgatgggaga aaatgtctgt ttcatggttt tgggtctatg 600

aattcgcttt atctgatata tacttatctt tctaggtatt attataacaa gcggacaaag 660

caatcaaatt gggaaaaacc tcttgaactg atgacaccac ttgagagggc tgatgcatcc 720

actgtatgga aggaatttac aacacctgaa ggaaagaaat attattataa caaggttaca 780

aaggagtcta agtggacaat tccggaagat ttaaagttag ctcgggaaca agcccaacta 840

gctagtgaaa aaacgtccct ttcggaagct ggatctaccc ctctatccca ccatgctgca 900

tcctcgtctg atctagcagt tagcactgtg acttctgttg ttcccagcac atcttcagca 960

cttactggac attcttcaag ccctattcaa gcgggtttgg ctgtacctgt cacccgtcct 1020

ccctctgttg ctcctgttac tccaacatct ggtgcaatta gtgacactga ggctactaca 1080

atgtactatt tttccttggg aagttttgct gagaataagg aaatgtctgt gaatggaaaa 1140

gccaatttgt cacctgctgg tgacaaagca aatgtcgagg aacctatggt atatgctact 1200

aagcaggagg ccaaagctgc tttcaagtct cttttggaat ctgtaaatgt tcattccgac 1260

tggacatggg aacagacatt gaaagagatt gttcacgata aaagatatgg tgctttgagg 1320

acactcggcg agcggaaaca agcgtttaac gagtatcttg gccaaaggaa aaaagtggaa 1380

gctgaggaaa gacgaaggag gcagaagaaa gctcgggaag aatttgtcaa gatgctagag 1440

gagtgtgaag aactttcatc atccctgaaa tggagcaaag caatgagttt gttcgaaaat 1500

gatcagcgtt ttaaagctgt tgaccgtcct agggatcgtg aagatctttt tgacaattac 1560

attgtggaac ttgagaggaa ggaaagagaa aaggcagcgg aggaacatcg gcagtatatg 1620

gcagactatc ggaagtttct tgaaacctgt gactatatca aagctggtac acaatggcgc 1680

aaaattcaag atagactgga ggatgatgac agatgctcat gtcttgaaaa gatagatcgt 1740

ctgattggtt ttgaggaata cattcttgac ctagagaagg aagaagaaga gctgaagaga 1800

gtagagaaag aacatgtaag gcgggccgag agaaaaaacc gtgatgcatt tcgtacacta 1860

ttggaagaac atgttgctgc aggcatcctt acagccaaga cgtactggtt ggattattgc 1920

attgagttaa aagacttgcc ccaataccaa gctgttgcat ctaatacatc tggttcaact 1980

ccgaaagact tgtttgaaga tgtcacagaa gaattagaga agcagtatca tgaggataag 2040

agctatgtga aggatgctat gaagtcaaga aaggcaaatt ttaaatctgc tatttcagaa 2100

gatctcagta ctcaacagat atcagacata aatttaaagc ttatatatga tgacttggtt 2160

gggagagtga aggaaaaaga agaaaaagag gccagaaagc ttcagcgtct ggctgaagaa 2220

tttaccaatc tgttgcacac tttcaaggaa atcaccgtag cttcaaattg ggaagatagc 2280

aaacaactag tagaagaaag tcaagagtac agatcgattg gagatgaaag tgttagccaa 2340

gggttatttg aggaatacat aacgagttta caagaaaagg caaaggagaa ggagcgtaag 2400

cgtgacgagg aaaaggttag aaaagagaag gaaagggacg agaaagagaa acggaaagac 2460

aaggataagg agagaaggga aaaggaaaga gaacgtgaaa aagagaaggg aaaagagagg 2520

agtaaacggg aagaatcaga tggtgagact gctatggatg tgagcgaagg tcataaagac 2580

gagaaaagaa agggaaaaga tcgtgacaga aaacatcgaa gacggcatca caacaattct 2640

gatgaagatg ttagttctga tagggatgac agagatgagt cgaagaaatc atcccgtaaa 2700

catggtaatg atcgcaaaaa atcaagaaag cacgcaaact cgcctgaatc ggagagtgaa 2760

aaccggcata aaagacagaa aaaagagagt agtcgccgaa gtggtaatga cgagctagag 2820

gatggagaag ttggggagtg a 2841

<210> 36
<211> 459
<212> DNA
<213> Arabidopsis thaliana

<400> 36
atggaggagg gacgtcaaaa agacttgcaa ttgttggagg agattatcga caaaggtttg 60

aaacagaagc ttgtacatgc aactgcttca cgggacaaga tctttgaaga acaaaaaaca 120

ctctctgact tgcggaaaaa cctagaaact ctggagaaga atggtgtaaa tagtctcaaa 180

acaagggtca accttggttc agaagtttac atgcaagctg aagtgccaga tactcggcac 240

atattcatgg atgtaggcct cggcttttat gtggagttca cacggcaaga agctcttgac 300

tatatagcac aaagggagga aagaactcaa aaacaactag aagagtatac tggtgttatt 360

acgcagatca aagggcgcat caaactggct cattaccaga ttcagcaaat actcaatctt 420

cctgaagaga atccgtcatc ccggcaacgt gcgttttag 459

<210> 37
<211> 276
<212> DNA
<213> Arabidopsis thaliana

<400> 37
atgggtgatc ataatagctc gcaagcttct tacatccatt tggtgcatca tttgatagaa 60

gaatgtatag tattcaacat gggcaaagaa gagtgtatgg atgctctgtt caagcatgct 120

aatattaagc ctatcatcac ttccacagtg tggaaagagc tagcgaaaga gaacaaagag 180

ttcttcgagg catacgagag aagacgagaa gaaataccga ccgagaaaga gacagctcga 240

agaatccgtg atttgctttc acgaactaca atctaa 276

<210> 38
<211> 1599
<212> DNA
<213> Arabidopsis thaliana

<400> 38
atgttcgcat gtttgcgtat tggacgcttt attcgtctgg gtaacgttac cgttaaatcc 60

actaatttgg tactgaggtg tgtctttatc cgaaattttg ccacccatgc cgaccacctg 120

ttcgacgaat tgccgcaacg agacctctcc tcacttaact ctcaactctc gtctcacctc 180

cgcagtggaa acccaaatga caccttggct ctctttcttc agattcatag ggctagccct 240

gatctcagct cgcacacttt cactccggtt ctcggggcct gttccctctt gtcgtaccca 300

gaaacaggac gccaagttca cgccttgatg atcaaacaag gcgctgaaac aggaaccata 360

tccaaaactg cgcttattga catgtactcc aagtacggac acttggttga ttccgttagg 420

gtattcgaaa gcgttgagga aaaagatctc gtctcatgga atgctctgct ttcgggtttc 480

cttagaaacg gtaaaggcaa agaagctctt ggcgttttcg cagctatgta tagagaaaga 540

gtagaaatca gtgagttcac tttgtcttct gttgttaaaa cttgtgcctc tctcaagatt 600

ttgcagcaag ggaagcaagt tcatgccatg gtggtggtca ccggacgcga tctcgtggtt 660

ttaggaactg caatgattag tttttactcg agtgtaggtt tgatcaatga agccatgaag 720

gtttataaca gtttgaatgt tcatacggac gaggtgatgt tgaattcttt gatatcaggt 780

tgcattcgaa accgaaatta caaggaagcg tttctgctta tgagtaggca gagacccaat 840

gtgagagtgc tcagtagctc tcttgctggc tgctctgata actctgatct gtggattggt 900

aaacagatac actgtgtcgc tttacgtaat ggtttcgttt cagattctaa gctatgcaat 960

ggcttaatgg atatgtatgg aaaatgcggt cagattgtgc aagcgcgtac tattttcaga 1020

gctattccat ctaaaagtgt ggtttcttgg acgagtatga tagatgcgta tgcggttaat 1080

ggggatgggg ttaaagctct tgaaatcttc agggaaatgt gtgaagaagg aagcggagtt 1140

ttaccgaatt cagtgacatt tcttgttgtt atatcggctt gtgcacacgc aggactagtt 1200

aaagaaggta aggaatgttt tggtatgatg aaggagaagt atcggttggt tcctggaaca 1260

gagcattacg tatgcttcat cgatatctta agcaaggctg gtgagacaga agagatatgg 1320

agattagtcg agagaatgat ggagaacgat aatcaaagca ttccttgtgc tatatgggta 1380

gcggtactca gtgcttgtag tcttaatatg gatcttacgc gaggcgaata tgtagcaagg 1440

aggcttatgg aagagacggg tccagagaac gcgagcattt atgtgttggt ttcgaatttc 1500

tatgcagcga tggggaagtg ggatgtcgtt gaagaattga gaggaaaact gaagaataaa 1560

ggtttggtta aaacagcagg acacagctta ttcatatga 1599

<210> 39
<211> 438
<212> DNA
<213> Arabidopsis thaliana

<400> 39
atgttacgga acatgatgat gccttggaac agtagcgatc acaatgtagt tggaatgtta 60

acaaggcatt tcgccacaaa accaaaaccc aagatgaaac cgattgagct gaacacacca 120

ccggagcaaa ctcagacgat aacccgagtg atctttgata ttttgaagga tcatggacct 180

ctaaccattg ctgaaacttg ggatcgtgtc aaggaagtgg gattaagagg gctgacgagc 240

aagcgtcaca tgaagataat actaaggtgg atgagagaga gacagaagct gaagctgata 300

tgtaaccatg ttggtcctca caagcaattc ttgtacacta cttggttcac taaacacaac 360

ccttcttcta aattccccaa gttaccaccg gaaaatctca caggaaaatc ctctggccac 420

cctcctaaac ttccctga 438

<210> 40
<211> 1329
<212> DNA
<213> Arabidopsis thaliana

<400> 40
atggctttcg ttcgatatat cccttgtcgg aagattccac gaaatgttga tcaattcgag 60

ctgccatgtc ttggatcgct tcgagctttc ttctctactc agaagctcat aggggatgaa 120

ccagttctcg ttcgagattt catacacact gcattatatg atccaataca aggctacttc 180

tctcaacggt caaaatctgt cggggttttg gagagaagca ttaagttcaa ccagcttgaa 240

gggaggaaag catacatgaa actcttggaa aaagtataca agcagagtga catttcttgg 300

tttactccag tggagctttt caagccttgg tatgctcatg ggattgcaga agctatactg 360

cgtaccacaa atctctcagt tccattaaag atatacgaaa ttggtggtgg atcgggcaca 420

tgtgccaagg gtgtattgga ctatataatg ttgaatgctc cggagagaat ctacaagaac 480

atgagctaca cttctataga aatcagtccc tcacttgcta agattcagaa ggaaactgtt 540

gcacaagttg gaagtcatct atcaaagttc cgagttgagt gtcgtgatgc atctgaccta 600

gctggatgga agaatgtgga gcaacaaccg tgctgggtga taatgcttga ggtgctagat 660

aatctcccac atgatcttgt ctattccaaa agtcaacttt ccccatggat ggaagtcttg 720

gttgaaaata aaccagagag cgaagcactc tctgagctat acaagccttt agaagatcca 780

ctgattaagc ggtgcattga aattgttgaa catgaagatg atccggtttc aaaaccaaaa 840

gaaatttggt ctaaactatt tcccaaacct agacgtagtt ggcttccaac aggttgtttg 900

aaactgctag aggttttaca tgcaaagctg ccaaagatgt ccctaattgc ttcggacttt 960

agcttcttgc ctgatgtgaa agttcctggt gaaagagccc cattggtttc aacaaagaaa 1020

gatggatgta gctcagatta cagtagttat ctggacgcaa agggtgatgc tgatatattt 1080

ttcccaaccg atttctggct tctagaacga atggaccatt attgttccgg ttggaggaag 1140

atggaaaaag acgggacacc atcgaaaaaa ggaaggaaaa ggcgaactct cactcttgat 1200

acatcagcgt tcatggatga gtttggttta ccttcaaaga cgagaacaaa ggacggatat 1260

aaccccttac ttgatgactt caagaacact aagttctatc ttagtgtccc aacacacaac 1320

actaagtag 1329

<210> 41
<211> 16011
<212> DNA
<213> Arabidopsis thaliana

<400> 41
atggctattg atgggagttt caaccttaaa cttgccttgg agacgttctc tgtacgttgt 60

ccaaaggtcg cagcttttcc atgtttcact tcgattctca gcaagggagg agaagttgtg 120

gataacgaag aggtgattca tgctttaggg gatgcgtttc ttcacccgga gtttacagtt 180

ccgttggttc attgcttcct tccaataata agaaatgttg tagatagagt ggtgggtctt 240

cttcgtctag tggatgatct taagtcaagt attgactact cagacgatgt gtcatcagtt 300

ttggataatg ctatgacgga aggtattagt gtgattgatt tttatgtccg gcgtggacaa 360

aggttggagc ttcatgagtg tgcttgcttg gccttcagtc gtgcgcttca tttcaatacg 420

tctttgttag ggtctattct aaattatttt gagaaagctc caccaccata cgagcgaatt 480

cttgtgaaag atatagtttc tgagtcgcgc atggaggcta cagatgcgta cttgctttgt 540

cttcgagtat catatcgttt tcttgtcatt agacctgaag ttttctctaa gttgtgggat 600

tggtcttgtt acttggactc catgaaaagg ctctcagaat gtcctagaca acaaaggcat 660

ttcttggaaa agtatcgaga tgctgtgtgg tgtgggattc aaattctttc tgttgttttg 720

agatgcagtg acagattagc aggatgtttt ggttttgaag aggaagaagc actttcgtgc 780

ttgctacgct gggaggaatt ttgtcaggat atagaaatag agaaggctgg attatacatt 840

caattgccta catacacagc gttgaagtct ttgcaacaat ttaataccct tgtacctgga 900

attaacaagc gacaatcagc agggttagaa gcagatgagc cacagatgaa gattcggagg 960

ctggacacct gggatgtcaa ttctttctct gaaccatttg aaatccactc tagggtgaag 1020

aaatcttttg aaatggtctc attggctgtt agtcaaaagc gacctgttct tctgtatggt 1080

ccctcggggt ctggaaagtc tgccctcatt aggaagttgg ctgatgaaag tggtaaccat 1140

gttgtattta tccacatgga tgatcaactt gatgggaaaa cattggttgg cacttatgtg 1200

tgtactgatc aacctggcga attcagatgg cagcctggct cacttaccca ggcgattatg 1260

aatgggttct gggtggttct tgaggacata gacaaagctc catcagatgt tcccctcgtc 1320
ttgtcatctt tgctgggagg gtcttgctca ttcttgacca gtcaaggaga ggagatacgg 1380
atagcagaaa ctttccaact gttttcaact atatcgacac ctgaatgcag tgtgtcacac 1440
atcagagacg ctggaaattc gttgagtcct ctatggagga gaattgttgt atatccacca 1500
gatcgtgaga gcttgcaaag tatcctgggt gctaggtatc ctaacctagg tcctgttgca 1560
gagaagctta ttgaaacatt tgaaaccatc aactctgctc ttcgtcccca attttctagt 1620
tcaacaactg aaaactcagc tactttcagt tctccaagta gattttcact gagagatctg 1680
ctcaagtggt gtgaacgagt tcatggcctg ccctcctatg atggccatgc agtttatcag 1740
gaggcagcag atatattctc tgcgtctaat atgtcagtta aaaaccgagt ggcagtaagt 1800
gagattgtgg ctagtatttg gaatgtcgct gttccagaat ctcaggataa gcccccaatt 1860
caggaatttt ccaggattct aaaaattggt agagtttctc ttccacttgg tgaaactgcg 1920
tcacatgatc ggtctaggtt tgttgaaaca cgcacatcta cacggttact tgagaaaata 1980
gctcgctctg tcgagtacaa tgagccagtt ctcttagtag gagaaacagg gactgggaaa 2040
acgacactag ttcaaaatct tgcacactgg atcggacaga aactcactgt tttgaatttg 2100
agccagcaaa gtgatatagt tgatctattg ggtggtttta agcctattga tccaaagctt 2160
atgtgcacaa tggtgtacaa tgaattcaat gaattggcaa gagatttgaa gattaaggat 2220
gattcaaaaa ttatgaaatg gctgcaagat aattttagag ccaagaagtg gcatacattt 2280
ttgactgggt tattggacat tattaaaggc attgaaggta gaattactga acgcatggaa 2340
ggtaaaattg gggaagcaag gtctagatct ggtagaaaga ggaagaaacc agaagaagag 2400
ctcaaaaact gtgcgtgtct gaggacgaaa gtgaataaga tacgacaaca gatccattca 2460
ggtggaatgg tttttacctt tgttgaaggt gcgtttgtga ctgccctcag ggaggggcat 2520
tgggttttac tagatgaagt gaacttagcc ccaccagaga tattgggcag gctgattggt 2580
gttcttgaag gagtgagagg atcactttgt ttagctgaga gaggggatgt aatgggcatt 2640
cccagacatt tgaatttccg tttgtttgct tgtatgaatc cagccacaga tgctggtaag 2700
cgagacttgc cattctcatt ccgaagcaga tttacagagt atgctgtgga tgatgacata 2760
tgtgatgatg acctggagat attcgtgaga cgatttttag gtggacgtgg atctgacagt 2820
aagttagtag ccaacattgt ttggttttac aaagaagcta aaaggttatc tgaagaaagc 2880
ttgcaggatg gtgctaatca gaagccacag tacagcttaa ggtctctata ccgtgcgcta 2940
gaatatgcga taaaagcaga agctattggt ggttttcaga aagcattata tgatggattt 3000
tccatgtttt tcctctcctt attggatgct tccagtgcta agatcgtgga accgataata 3060
aagcgtatct ccggggaaaa tatccgaagc caaccacttc aaagatactt gggagaatta 3120
aaaggcagtt ctgataaatt tgttggcagt tatgttaaga cgaagagtgt aattgatcat 3180
cttaatcatt tggcgcatgc catttttatt aaaagatatc ctgtgctctt acaaggacca 3240
acatccagtg gaaaaacaag ccttgtcaaa tatcttgcag caataagtgg aaacaaattt 3300
gtaagaatca ataatcatga gcagactgat atccaagagt atttaggttc ctatatgact 3360

gattcttcag ggaagcttgt atttcacgaa ggagcgttgg tgaaggctgt caggggtggg 3420
cattggattg tcttagatga acttaatttg gctccatctg atgtcttaga ggcactaaac 3480
aggctgcttg atgacaatag ggagcttttt gtgcctgagc tgagtgaaac aatctcagcg 3540
catcctaatt ttatgctctt cgctacacag aaccctccta ctttatatgg tggacgcaaa 3600
atactgtctc gagcttttcg caatcggttt gtggagattc atgttgatga aattccagaa 3660
gatgaactga gtgaaattct tactacgaag tgtagtattg ctaacagtca tgcttcaaaa 3720
atggttgaag tgatgaaaga cctgcaacgc aataggcaga gtagcaaagc ttttgctgga 3780
aaacatggtt atataactcc aagagattta ttccggtggg cctatcgttt caggacttat 3840
gacggtacat ctcatgaaga actcgccaga gaagggtatt acatccttgc agaaaggctg 3900
cgtgatgaca ctgagaaggt agttgttcaa gaggtgctgg agagacattt ccgtgtcagt 3960
cttgccaaag atgatttgta caatatgcct gttcttcttg ttggtgacac tggaggaggc 4020
aaaacaacaa tctgccaaat actaagcgat gttaagaaga aaagattgca catccttaac 4080
tgtcatcaat acaccgaaac atctgatttc cttggtggat tctttcctgt gagagacaga 4140
tcaaaattga tcacagaata cgagaatcaa gtcaaacagt tggagctctc tcaggcattg 4200
acgccttttg gccaagatat tgttatttgt ggagatatta gtagagctga agtgtcgatc 4260
aaatcagtag aggtagcttt ggagaagtac aaaaatggtt cagttatagg agtggccgcc 4320
acgccacagg atgttgattt tcttgagaaa ataaggaaca atatggtgat gctgtatcaa 4380
aaatggcgtg caatatttgt ttggcaagat gggccccttg tggaagctat gagagctgga 4440
aatatcgttc ttgtggatga gatatctttg gctgatgaca gtgtattaga aagaatgaat 4500
agtgtgttgg agacagacag gaaattgtcc ttagctgaga aaggtggtcc cgtcttggag 4560
gaagttgtag ctcatgaaga ctttttgttt ctagccacca tgaacccggg tggtgattat 4620
ggaaagaagg aattgtcacc tgcgcttcgt aatcgtttta ctgagatatg ggtccctcct 4680
attacagata ctgaggagct cagaagtatt gccttttctg gcctgtccag tttgaaggaa 4740
tctaatgttg tagatcccat catcaacttc tgggagtggt tcaacaggtt gcatactggg 4800
agaacgctta ctgtcagaga tctcctctcc tgggttgcat ttgtcaacat ggcaactgag 4860
agtttaggac cagcatatgc tattcttcat ggagcatttc tcgtgttact tgacggttta 4920
agtctcggaa ctggtttctc tggaagggat ggtcaagatc tgagagaaaa atgcttcgct 4980
ttcctgttac aacaacttga gctttttgct agcgatacac tacctttgga gctttcaaga 5040
atggagctgt atggctgggg tgattccaaa gcaatttgtg aaaaaagtaa gagtgttcga 5100
catgagggca tgtttggcat cgatccattt tttataagca aaggtgatga aaatcctgag 5160
attggtggat tcgagttttt agcaccaact acccacagga atgtcttgag agtattgcgt 5220
gcaatgcagc tttcaaaacc aattttatta gaaggtagcc ctggtgttgg aaaaactagt 5280
ctgatattgg cgttgggaaa atattctggc cacaaggttg tgcgcataaa tctatcggag 5340
cagactgaca tgatggattt gctgggatca gatttaccag ttgaaagtga tgaggacatg 5400

aagtttgctt ggtctgatgg aattctcttg caggctctaa aagaaggctc gtgggttttg 5460
ttagatgaac tgaaccttgc cccacaatct gttctagagg gtttgaatgc gattttggat 5520
catcgtgctc aagtcttcat cccagaactg ggctgtacct ttgaatgccc tccaacattt 5580
agagtttttg catgtcagaa tccttccact caaggtggtg gcaggaaagg tcttcccaag 5640
tctttcctta accgattcac gaaagtttat gtggacgagt tagtggaaga tgattacctc 5700
ttcatctgtc gctcacttta cccatctgtt cctagtccat tgctttcaaa gcttattgct 5760
ctcaacagac agttacacga tggtactatg ttatatcgaa agtttggtca cgatggctca 5820
ccatgggaat tcaatctacg ggatgtgata agatcatgcc agtttatgca agaggcgata 5880
catgacttag aagttgaaag ctttctcaat gttctgtaca ttcaaagaat gcgtactgca 5940
actgaccgta aagaagttct gcgtatctat aaggctattt ttgataaaac cccgtcgata 6000
aatccgtatc ctcgggttca gctaaatcct gcgtacttag ttgttggaac tgctgccatt 6060
aaacgaaatt taaatcagtc taatattgcc agtgagcagt tgaaactttt gcctgaaatc 6120
cgtcaaaatc tggaagctgt tgcacattgt gtgcagaata aatggttgtg catcctagtc 6180
ggaccatcgt catctggaaa gacttcggtg atcagaatat tggctcagtt aacaggatat 6240
cctcttaatg aattaaatct ttcgtctgcg actgacagct ctgatctact cggatgcttt 6300
gagcagtaca atgccttccg taatttcaga ttggtgatga ctcgagttga gcaccttgtc 6360
gatgagtata acagtctgct attacagtct tcccaggagg cccttttcag caataggagt 6420
ggcttagttt ccagatggct ttcctattta aataagattg attcctctct cgtggagaac 6480
ccattattct tcttgaacga ctctgaaaca ctgtctacat tagaagaggt tgtagaagac 6540
ctggaacagg tcttgaaaga aggtgtttta cccgttagtt ggtcaaaaaa gtatctggaa 6600
caaatctcga agactatatt gcagttacaa actcatgaga aaaagcagtc tacaaagttt 6660
gaatgggtga caggaatgct gataaaggca atagaaaagg gagagtgggt tgtcctcaaa 6720
aatgctaatc tctgtaatcc cacggtactt gatagaatta actcattggt ggaaccgtgt 6780
ggatcaatca ctataaatga atgcgggatc gttaatggtg aacctgtcac tgtggttccg 6840
cacccaaact ttcgtttgtt cctgtctgta aatccaaaat ttggggaagt atcaagagca 6900
atgaggaata gaggcgttga ggtatttatg atggggccac attggcagct caatgaggat 6960
ggctcaaact gtgaagagct tgtgctgaga ggtgtggaaa ggtttcttgc tctgtcaggt 7020
attccaggtt ataagctggt tacttccatg gccaaagcac atgttcatgc atggctaaac 7080
ggtcaaagct ttggtgtacg gatcacgtat cttgagctcg aacagtgggt tcacctcttc 7140
caattgctgc tcatgaatgg taatcaactt ttgtggagct tacagctaag ttgggagcac 7200
atctatctct cttcgcttgg ggtaactgat ggaaaagaag ttgttgattt tgtgcgtgag 7260
acatatttat cagatgttga actttctgag cttgattcat ttatgggtgg ggatctgtac 7320
ctgcctggag gatggccaaa gcctttcaac ttgagagact tgacatggta ctcaagagaa 7380
acaacagtaa gacagaattg catgtatctg gagttcctag gagctcagta tgcctcacat 7440
cagcctaaaa taagcgacaa tgtcaaatca agagataggg agttggctgc tggggaacca 7500

agaattattt attctattga ttcttggacg cttaaaaaag tcttgtttcc taaagcctta 7560
attgggtcaa gctgtgcacc agatgcagca aattttgaaa atgatttggc ttcaaaaatg 7620
ctattgtttg ctgccaactg gacaatagaa caggcaaccg aagaggatat tcaactctat 7680
cttgcgtggt ttagttggtt tggttctaga ctgcaacaac actgtccgtt tctgctttgt 7740
tttctcaata cgttgaaggt tgagtttgag catccaattt ggaatcatat atctagatgt 7800
cggaaaaatc tgaaattcct ctgcagattg gatccagatg ctgttccaat tcctatgctg 7860
tcctccaaat tgattgatgt agccgcatca aatgaccagt ccaaacctta cagtaaatcc 7920
ctctttgagt ctctcaactc tgttggcgtt cttcgtcgtt cgtatcagca gtggcttgta 7980
gagagcaacg acaaccacac agatgtatcc acttttactc ggtttttgga ttcgcttcgc 8040
gtattggaga agaaaattct ttgcgaaatt gttggagcac catctttcag tgtgttgatt 8100
cagttgtaca ccgaagttat tgacaaccat tcattctttt ggtctggttt ggtctcttct 8160
tcagatgagt atctattgtt ttccttttgg tcactgataa aatctatcaa aaagatgcac 8220
agttttttcc ctggagaagt tcaggtggtt ctggaggaaa gcaaaaatat taacaacata 8280
gttttgcatg gtcaccctga aaagtctatg ctgtgggctt atgggggaca tccttccttg 8340
ccggtatctg cagagctgtt ccacaagcag caagagtttc tacagctgtg cagcacagtt 8400
tggccattga aatcagaatc agatgaacac ggaaatgatc atcttaccaa agccattcca 8460
ttttctggcc ctgaattatg tttgcttgcc ttggaaggtc tttgcatttc atcatacatt 8520
gctgacgaag acgatgtaga ttatgtagct gctgttcagc tggatgagat ctaccagact 8580
tttttggaga ggctgaaact agagaagaag agactggagg ataaaatggg tttcagtgag 8640
attgacaata ctgaaaatat aactgcttcc tgctgcgtgt tctgtccaga gattgtgact 8700
acagggtctg gatttagcag ttgggtgaag acatgtttta ttgctagcag tgaaagttgt 8760
tctctagacg tagagttact tgctgcactt cagcacctct tggttgctcg acctactgaa 8820
catcaggatc ttgtggacat tcgaaaactg ctcaaaccgg ctctagaata ttctttatcc 8880
tcaaccaggc ctccacagac tcttgtagct catcaaaaac tcctgtgggc aattgatgca 8940
catgcctctg aactaggagt ggacaccaaa attgctggtt ttgctctcga gatttggtac 9000
tggtggcatt ctgtattgtg gaaaaatagt caaattggtc tcatgaatat ctcagacact 9060
ggcaactgtc agattctgtc accttctatg ctgattcagc ctgtgaaaac agctaccgtt 9120
gctcagattc tggaaaatgt attttctgtt aaggattatt ctgttcaatc aatgaaactt 9180
ctttctgctt cacgatatct atggaaaagc tcacaaccct atcaagaaat gcctggttct 9240
ctattgtcaa ttgcacgttc ccttttccaa cagataatat atacgcacca aaagtcattt 9300
gagtcagaaa cgtttgtggc aattaagtct gtatttcatg caattgagaa aaagcagaac 9360
aagatggatg gaatacagaa tcttatctca ctgattggct catcaagcca taataaattg 9420
aaatccgtta ctcactcatt tgtcggacca ttagcaaaac gtctttattc cgatagctca 9480
tcaaatgaat tctactgcaa tcttggcttg gcgtggcttt atcttggagg actacgcttc 9540

catcttttga atagcttaga tgttatagat ccagccatga agatcacttg caagctgtta 9600
aagctagaag agaaaatctc atcacttgag ctaaacatca aggtccgggg agaatgtggg 9660
tatctgtctg gattgcttta ctctggaaac aatgacgaaa gcagtgaaca tacattatct 9720
aagctcaaaa ctgagcataa aagattgcaa agaaaggtta tttttagatc tgatccaaaa 9780
aagtaccagg atttacgaag ggcgctggat gaatttgctg gatttctcac acgtcccata 9840
agtctggtca acgatattga agtgcttgat tggaatcagg ttgttgagca ggttttcaac 9900
tggcaggaga cagcaatatc ttttattgat cggatgtcaa gtgactattc tgaatatgtc 9960
gatataactc agccaattca agtttcagtg tacgagatga aattgggttt atcactcttt 10020
gtatctggtg ctctcttggg aaaacttctc aacagatttg acatagacat ggttgactca 10080
gtcatggaaa caatttatgc cttaatgaga tttccaaggg actcgtcgat agcttcaact 10140
acctacaccg aatgtttgcc acctttgcac ctttcccatg gtgcaaattc tcgtgctaag 10200
tccttaggtt tggatgttgg cttgttgcac aaacttatct ctgtttcaag tgcagaagat 10260
tcgagaaaag cctcagagtt gcaactcaaa gttgctcttt ataaaaatct ccatgctcgt 10320
gttttacaat ttgtcgcaaa tactgggcta ctggatgaag cttcttttga gttattggac 10380
aagatatatg ttgaattggc gagaatttgg atggagatga agtttcaagc caaaacaaag 10440
gctgacaatc ttcctgggct gtacaaattt cgttcccggg acttcaaaat tgatagtgtc 10500
atggaagtag atatatctgc ccttggcaag tatttcccaa acgaaagttt ctctgagtgg 10560
caagagtatc tggctgatga tgatacgaag aatgtgaaag atatgacaca tattgaccag 10620
gatgaggaaa atttggagga tgattgggac ttgatacagg agcatctgga tagtatatat 10680
agcacacata atgagttatt tggtttctgt gacctctctg aaaagtctgg aagattctgt 10740
attactgaca gtagaagact ggattcgttc actgattcct atgaacttgg agtcagtatg 10800
atcaaagggc taaggggttt atttacatcg agcttggatg caaaacttgt tccagaacac 10860
ctacttcgtc tttgcctgga aaacaaaaaa aacttcactt caaactatca gtcagccagt 10920
aaatataact tttacaagga tttggatggt cctgagctgg ggaaaatggt caagtttctc 10980
actcctcttc aacaaagaat taattctcta ttgcaagaac gggaggacca tcctggtctt 11040
cagaaacttt ctggtgtact tcagatgctc ttggctattc cctccagtac tcctctcgca 11100
aaggctctct caggattgca atttctgctc tgcaaggttc acaagttaca ggaagaggga 11160
tgtaaattgc ccatctctga tcttttggag ccaattattt ccctagcaag ctcttggcag 11220
aaggtggaat ttgagcgctg gcctactttg cttgatgagg ttcaggatca gtatgaacta 11280
aacgctagga agttgtggct tcctttgttt tcagttctgt ttcagaagga tgctgtggaa 11340
atttcagaac atgaaaacga gtccatttca caaagtttgg tggagttcat tgaaacgtca 11400
aatgttggtg aatttaggag acgtcttcag cttctctttt gtttccttct tcaattaagt 11460
atgggtagct cgttggggat atattcaagt gtaatggagc agttagattt gaatagaaaa 11520
aatgttgaaa ctgagttaaa ggaggttctt aaactttgtc ggtgggagag gccagataat 11580
tatttgtaca atgagaccac taaaaggacc aggcaaaagg tcaagaaact gatacagaag 11640

tttacggaca tgctacgcct ccctgtaatg cttgttaagc cagacctgac gaaggaacga 11700
gctcaatttc tccctctact agatccagat cttatggatg gagcatccga catgaggatc 11760
gaggtcctag ttagtgcttt agatgcagag caattgaggg acaggtcttc atggtatgtt 11820
gtctggtgga ataaattaaa ggaatcggta ggacgctttc accaagaaat gcactataaa 11880
acattgctga tgggtgcaga gcatcagtat tcgtcccctg tctatcaggg tgattggaaa 11940
aatttgtgga gtacggttgc taggattggt gaaaccatag ctggctgttc agatctatgg 12000
agaaacagtg atagagatgt tgcaaagaag agggccctgt ttgaacttct caagttatta 12060
gaaagtagtg gtttgcagaa acacaagttt gaaaatatag agatgtcaaa tcactttaaa 12120
gggttgcttt atcagccagc atacgatcca aagcatctgt tactgctaac acataccaaa 12180
agtaacatac atccttccat gggtgtagaa gatcaaaaca aggaaaattc actagttgag 12240
tggagagtgg caaatgagtt ttactttaag agcttggctt cagtgcaact catgttaaat 12300
attgaccgaa aacactccga tgtaacagct gagcaggtta aacgggcaat ctcatttctc 12360
aatcatcttg tggaaataca acggcaacaa aggaaatctg cgtatgcctt tgccgaactt 12420
ttcaaccgct ttcgccaatg tgttttatct ctagcgagat tactgggtga ttcagttggt 12480
gcggatagaa aggatgattc tgtgttcagt ttcccccaaa atcaacatgc tgtcttcaat 12540
tgcttgtggc tacagaagca actctttgat aacattactg caatgcttct tgaggagtcg 12600
gccttactga gaacagttgg aagtacacac ttggattcct gtcaagctgt gaaaacctca 12660
tcacggagtt tgctcagctt tattgaaata ctaattccca tcgctcaaaa ttccaaggct 12720
tcgctggata ggcttctact tgattgcaac ggttttatca tcacaccaag tagcagtctt 12780
aagcagtttg tcactcagca tatggttcag gtgctacgcc agaactttga tcaacttacg 12840
gaccttgaga accaaatttc aagtttctgt gaaaacaatg agaaaagcta ttgcagagac 12900
gttcttctca gtcaattttc ccctgtgttt aaagagggga aattgttggc tgaaaatctg 12960
aactgcttac ttaacgtgag agaccagtca actggaatgg aacccaagga acgactattt 13020
cttgaagaaa atcttgcaag tatatttgca aatgttaagg atgtgattgg aaagctttgc 13080
tcttataaag atggaagtct ttctcaagaa gaggaaatga atattactac atgggatggt 13140
ctgtttaaga aggcagaaaa tgacttgaac cttgataacc tgtgtaaact cctgtccgaa 13200
tcatttggtt ccattgaaca actgttgaac tcatcaggcg tcctttcagc tggtgttgga 13260
gaccagttga agcaacttca agcatttttg gatcttttat tgagctttgg ggattgttac 13320
cttaaagagt ttttggcgat aagcaaaacg gtttcactga taacccatgt ccttgcaagt 13380
gttcttgccg atctatttac aaaaggattt ggcatctcca aaaatgaaga agatgatgac 13440
tctaaagttg acaaatcgga agctgcagaa ggtactggta tgggagatgg tgtgggggca 13500
aaagatgtaa gtgaccaaat agaagatgaa gaccaactgc atggcacaga taagaaggaa 13560
gaggaagaga aagagcaaga tgatgtgctg ggtaaaaaca aaggcattga gatgagtgac 13620
gaatttgatg gcaaagaata cagcgttagt gaggatgaag aagaagacaa ggaagacgaa 13680

ggaagtgagg atgagccgtt ggataatgga ataggagatg tgggatctga tgccgaaaaa 13740
gccgatgaaa agccatggaa caaggatgaa gaagatgagg aagaaaatat gaatgagaag 13800
aatgaatctg gaccatctat agtcgacaag gacacaagat caagggagct aagagccaag 13860
gatgatggtg ttgaaactgc tgatgagcct gaggagtcca atacttctga caaaccggaa 13920
gaaggaaacg atgagaatgt ggagcaggat gattttgatg atacagataa tttagaagaa 13980
aaaatccaga ccaaggaaga agcacttggt ggactaactc ctgatgtcga taatgaacaa 14040
attgatgatg acatggagat ggacaaaaca gaggaggtcg aaaaggaaga tgcaaatcag 14100
caggaagaac cttgttcaga agatcaaaag catcctgaag aaggtgaaaa tgatcaagaa 14160
gaaactcaag agccatctga ggaaaatatg gaggctgagg ctgaagatag gtgtggatca 14220
ccccaaaaag aagaacctgg aaatgatctt gaacaggaac cagaaacgga accaatagaa 14280
ggaaaagaag ttatgtcaga agacatgatg aaaccgaact tccgtaatga taatatttct 14340
ggcgtagagt ctggttcaca aaatccccat gggtctaatg tgctgggtgc aggaagtaca 14400
gcaccacaag aaaatttgtc tgctactgat gttacggatg aactcactga ttcaatggat 14460
ctgccttcga gtagtaacac ggaaatgaac ctcatgatga ccaacatggc caacggtgag 14520
acattgacag acaacttacc aaagatggaa tttcctcaaa accagtcatc tactgctcaa 14580
caaaccaagg tcaatcctta taggaacgtt ggtgatgcct tgaaggagtg gaaagaaaga 14640
gttagaatct cctctgacct tggagaaaag caagaggctg aaaatgagat ggaagaccct 14700
gatgctagtg aatatggatt tgcttctcag tttgatgcag gaacttccca agctctagga 14760
cctgcgttgc ctgagcaagt gaacacagat atgagagaag gggaatccga agaagaaaaa 14820
cttgcaggta atcaggatga tgtctctcca atggatattg atgacttgaa cccagaaaac 14880
aaacctgctg tccaatccaa accatcgatc agtaatagca tcgcggaaca ggtccaagaa 14940
ccagatacag ataggaccca ccaagagaac tctcctattc ataattttgg tgatggtaac 15000
agtaggatgg actctatggt ctctgtcgac aatactttct tgggggaaga ggcatgtaat 15060
ctggaccgga tgcaagtgac tgataatgac tcggaaagca atcaggataa tcaggaagat 15120
ccagatgcca gaagcaatgc tgttgttctt tggaggagat gtgaattgct tactgcaaaa 15180
ccgtctcagg agctggctga gcaactacgt cttatcttag aacccacgct tgctagcaag 15240
ctcagtggtg actacagaac gggtaaaagg atcaacatga agaaggttat tccatacata 15300
gcaagtcact atcggaaaga taaaatttgg ttgaggagga caaaaccaaa caagcgtgat 15360
taccaagttg ttatcgctgt ggatgactcg cgtagcatgt cagaaagtgg atgtggtgat 15420
tttgcaatta gagctttggc aacggtatgc cgagctatgt cacagcttga gctgggaagt 15480
ttggctgtgg caagtttcgg gaagcaaggg agcataaaga tgttacatga ttttggtcag 15540
tctttcacca cagaatccgg cattaagatg atctcaaatt tgacatttaa acaagaaaat 15600
ctcattgaag atcaaccagt cgtcaatctg ctgagaaaca tgaatgaaat gctagagaat 15660
ttggccagca caagacgaca gtcttacggg agcaacccgc ttcaacaact tgtactaatc 15720
atcggcgatg ggaagttcca tgagcgagag aagttgaaac gaactgttag aagctttctc 15780

cagcaaaaac gtatggtggt atatctgctt ctcgatgacg cagagcaatc tgtttttgat 15840

ttagcggact atgtatatga tggtgaaagg agaccttata agaaaatgaa ttacttggat 15900

tccttcccct tcccatacta cattgtgcta agagacatcg aagccttacc cagaacactt 15960

ggtgatgtgt tgagacagtg gttcgagctg atgcaaagct cgcgggactg a 16011

<210> 42
<211> 1005
<212> DNA
<213> Arabidopsis thaliana

<400> 42
atggagacta ccggagaagt tgttaaaaca accaccggga gcgacggagg cgttacggtg 60

gtgagatcca acgcgccgtc agacttccac atggctccga ggtcagaaac ttcaaacaca 120

cctcccaact ccgtcgctcc tcctcctcct ccaccgccgc aaaactcctt tactccgtcg 180

gcggctatgg atggtttctc aagcggaccg ataaagaaga gacgtgggcg ccctaggaag 240

tacggacacg acggagcagc ggtgacgcta tctccgaatc cgatatcatc agccgcacca 300

acgacttctc acgtcatcga tttctcgacg acatcggaga aacgtggcaa aatgaaacca 360

gcaactccaa ctccaagctc attcatcagg ccaaagtacc aggtcgagaa tttaggtgaa 420

tggtctcctt cctctgccgc cgctaatttc acgccgcata ttattacggt gaatgcaggc 480

gaggacgtta cgaagaggat aatatcattt tctcaacaag ggtctctagc tatttgcgtt 540

ttatgcgcaa acggtgtcgt ttcgagcgtt acacttcgtc agcctgattc atctggtggt 600

acattgacct atgagggtcg gtttgagata ttgtcactat ctggaacatt catgcctagt 660

gactcagacg ggacacgaag cagaacaggc gggatgagcg tgtcgcttgc tagccctgat 720

ggacgtgtag taggtggtgg tgttgctggc ttgctggttg cagccactcc tattcaagtg 780

gttgtaggaa ctttcttagg tggaacaaac cagcaagaac agacaccgaa gccgcataac 840

cacaacttca tgtcttctcc attaatgcca acttcttcga atgtagctga tcatcgaacc 900

atccgtccca tgacatctag tctcccgatc agtacatgga caccgtcttt tccttctgat 960

tcacgacaca agcattctca tgactttaat atcactttga cgtga 1005

<210> 43
<211> 1179
<212> DNA
<213> Arabidopsis thaliana

<400> 43
atgggtactc acattgatat caacaactta ggcggcgata cttctagagg gaatgagtca 60

aagccattgg cgaggcagtc ttcgttatat tccttaacgt ttgatgagct tcagagcaca 120

ttaggtgagc cggggaaaga ttttgggtct atgaatatgg atgagttact caagaacata 180

tggactgctg aggatactca agcctttatg actactacat cttcggttgc agccccggga 240

cctagtggtt ttgttccggg aggaaatggt ttacagaggc aaggctcctt gaccttgcct 300

agaacgctta gtcagaagac tgtcgatgaa gtctggaaat acctgaattc gaaagaaggt 360

agtaatggga atactggaac ggatgcgctt gagaggcaac agactttagg ggaaatgact 420

ctggaagatt tcttactccg tgctggcgtt gttaaagaag ataatactca gcagaacgaa 480

aacagtagta gcgggtttta tgctaacaac ggtgctgctg gtttggagtt tggatttggt 540

cagccgaatc aaaacagcat atcgttcaac gggaacaata gttctatgat catgaatcaa 600

gcacctggtt taggcctcaa agttggtgga accatgcagc agcagcagca gccacatcag 660

cagcagttgc agcagccaca tcagagactg cctccaacta tctttccaaa acaagcgaat 720

gtaacatttg cggcgcctgt aaatatggtc aacaggggtt tatttgagac tagcgcagat 780

ggtccagcca acagtaatat gggaggagca gggggtactg ttacagctac ttctcctggg 840

acgagcagtg cagaaaacaa tacttggtca tcaccagttc cttacgtgtt tggtcgggga 900

agaagaagca atacgggcct ggagaaggtt gttgagagaa ggcaaaagag aatgatcaag 960

aatcgggaat ccgctgctag atcaagggct cgaaaacagg cttatacctt ggaactggaa 1020

gctgagattg aaagtctcaa gctagtgaat caagatttgc agaagaaaca ggctgaaata 1080

atgaaaaccc ataatagtga gctaaaggaa ttttcgaagc agcctccatt gctggccaaa 1140

agacaatgct tgagaagaac ccttaccggt ccgtggtaa 1179

<210> 44
<211> 2505
<212> DNA
<213> Arabidopsis thaliana

<400> 44
atggttgata acagtaacaa taagaagagg aaagagttca tcagtgaagc agacatcgcc 60

actcttttgc agagatatga tactgtgacg atactgaagt tgctacaaga aatggcgtat 120

tatgctgaag caaagatgaa ttggaatgag ttagtgaaga agacaagtac tggaattact 180

agtgctagag aatatcagtt gctttggcgg catcttgctt atagagattc tctcgtccct 240

gtgggaaata atgctcgagt tctggatgat gatagtgata tggagtgtga attggaagca 300

tcccctggag ttagtgttga tgtagtaacg gaagctgttg cgcatgtgaa agtgatggct 360

gcttcctatg tgccaagtga gtccgatatt cccgaagact caacggttga ggctcccttg 420

accattaaca taccttacag cctgcatagg gggcctcagg aaccatcaga ctcatattgg 480

tcatcaagag ggatgaatat cacctttcct gtttttcttc cgaaagcagc tgaaggacat 540

aatgggaatg ggttagccag tagcttggct cctcggaaga gaagaaaaaa atggtcagct 600

gaggaggatg aggagctgat tgctgctgtt aagcgacatg gtgaaggcag ctgggccctt 660

atctctaagg aagaatttga aggagagcga acagcctcac aactctcaca gcggtggggg 720

gctataagga gaaggactga tacttcaaac acttctaccc aaactggcct acagcgaaca 780

gaagcacaaa tggcagctaa tcgtgcatta tctttagcgg tgggaaatcg gttaccctca 840

aaaaaacttg cagtaggtat gactccaatg ctgtcatccg gtaccatcaa gggagcacaa 900

gccaatggtg ccagcagtgg tagtacattg caaggtcaac aacagcctca gccacaaatt 960

caagcattat cacgggcaac aacatcagtg ccagttgcaa aatctcgagt tcctgtaaag 1020

aaaacaacag ggaactccac ttcgagagca gacctaatgg taactgctaa ttcagtagct 1080

gctgcagcct gtatgtctgg cctggcaacc gctgtaacag tgcctaagat tgaaccagga 1140

aagaatgctg tttctgcgtt ggtgccgaag actgaacccg taaaaaccgc ttccacagtt 1200

tctatgcctc gtccttcagg tatatcatca gcactgaata ctgagcctgt aaaaaccgct 1260

gtggcagcct ctttgcctcg ttcatcaggt attatttcag caccaaaggt tgagcctgta 1320

aaaaccgctg cttcagcagc ctctttgcct cgtccatcag gaatgatatc agcaccaaag 1380

gttgagcctg tgaaaaccac cgcctctgta gcctctttgc ctcgtccatc aggtattatt 1440

tcagctccaa aggctgagcc tgtaaaaacc gctgcttctg cagcctcttc gcctcgtcca 1500

tcaggaatga tatcagcacc aaaggttgag tctgtgaaaa ccaccgcctc tatgcctcgt 1560

ccatcaggta ttatatccgc accaaaggct gagcttgtaa aatccgccgc ttctgcagcc 1620

tctttgcctt gtacatcagg tattatatct tcaccaaagg ctgagcttgt aaaatccgcc 1680

gcttctgcag cctcttttcc tcgcccatca agtatgctat cagcaccaaa ggctgaccca 1740

gtaaagattg ttcctgctgc tgccactaac actaaatcgg ttggaccttt gaatttaagg 1800

catgcagtca atggaagccc aaaccacacg ataccttcat caccctttac taagccttta 1860

catatggctc ctctctccaa aggatctaca atccagagta attcagttcc tcctagtttt 1920

gcatcgtcaa ggttggtccc cacacagaga gctcctgcgg ctactgttgt cacgccacaa 1980

aagccaagtg tggtagcggc agctactgtt gtcacgccac aaaagccaag tgtgggagca 2040

gcagctactg ttgtaacgcc acaaaagcca agtgtgggag cagcagctaa tgttgtaacg 2100

ccacaaaagc caagtgtggg atcagcagct actgttgtaa cgccacaaaa gccaagtgtg 2160

ggagcagcag ttaccgtcac ttccaagccg gttggtgtac agaaagagca aactcaggga 2220

aacagagcaa gccccttggt tacagcaaca cttccgccaa ataaaaccat cccagcaaat 2280

tcagtgattg gcacagcaaa agcggtggct gcgaaagtgg agactcctcc tagccttatg 2340

cctaagaaaa atgaagtagt tggcagttgc accgataaaa gttcattgga taaaccacct 2400

gagaaagaaa gtactaccac ggtgtcacct ctagctgtag ctgcgactaa atcaaaaccc 2460

aaagatgaag caaccgtgac agggaccgga ctgaaggagt tgtag 2505

<210> 45
<211> 3969
<212> DNA
<213> Arabidopsis thaliana

<400> 45
atgggttata ccttgcaaca gatactgagg agcatctgct ccaacacgga ttggaactac 60

gccgtgttct ggaaacttaa tcaccactcg ccaatggttc ttactttgga ggatgtgtac 120

tgtgttaatc atgagcgcgg tttgatgccg gaaagcttgc atggagggcg ccatgctcat 180

gaccctcttg ggttagctgt ggctaagatg tcatatcatg tacactctct tggggaaggg 240

attgtaggac aagtagcaat ctctggacaa catcaatgga tcttctctga atatttgaat 300

gactctcatt cgacacttca ggttcacaac ggttgggaga gtcaaatttc tgctggaatt 360

aagacaattc ttatagtagc tgttggttct tgcggagttg tgcagcttgg ctctttgtgt 420
aaagttgaag aagacccggc tttggtgact catatcaggc atttattttt ggcacttacg 480
gatccactag cagaccatgc atcaaattta atgcaatgtg atattaacag tccatcggat 540
cggccaaaaa taccttccaa atgcttacat gaggcatccc ctgatttctc aggagaattt 600
gacaaagcta tggatatgga agggttaaat attgtatctc aaaacacaag taatagaagt 660
aacgaccttc catacaattt cactccaaca tattttcaca tggagaggac tgctcaagta 720
attggtgggc ttgaagcagt ccaaccttcc atgtttggaa gcaatgattg tgttacaagt 780
ggtttttcag ttggtgtggt tgatactaaa cacaagaatc aagtggatat aagtgatatg 840
agtaaggtga tttatgatga ggaaacaggt ggataccgat actcaagaga attagatccc 900
aatttccaac actactcgag gaatcatgtg cgtaatagtg gaggcacatc tgctttagct 960
atggagagtg ataggctaaa agcaggttca tcatatccac aacttgattc aactgtactt 1020
actgcgttga aaacagataa agattattct cgtcgaaatg aggttttcca accatctgag 1080
agccaaggaa gtatatttgt gaaagataca gaacataggc aggaggaaaa aagtgagtca 1140
agtcagttgg atgctttaac tgcatctttg tgttcttttt ctggcagtga gctgttagag 1200
gcattagggc cagcgttcag taaaacaagc actgattatg gggagctagc aaagtttgaa 1260
tctgctgcag ctataagacg aacaaatgat atgagccata gtcacctgac atttgaatcc 1320
agctccgaga atcttctaga tgccgttgtt gctagtatga gtaatggtga tggtaatgtc 1380
aggcgtgaaa tatcttcaag caggtcaaca cagtcattgc ttacaactgc tgaaatggca 1440
caggcagaac cttttggtca taataagcaa aatattgtta gcacagttga tagtgtgatt 1500
agccagccgc ctctagcaga tggcttatcc caacagaatc catcaaatat ctgcggagca 1560
ttttcttcca ttgggttttc atcaacatgt ctcagttcat ccagcgacca gtttccgacg 1620
tccctggaaa ttcccaagaa gaacaaaaag agagctaaac ctggtgaaag ttctcggcct 1680
cgtccaaggg acaggcaact tattcaggat cgtatcaaag aactaaggga gcttgtgcct 1740
aatggatcta agtgcagtat tgattccttg ctagagtgca cgatcaagca catgctcttc 1800
ctgcagagtg tctctcagca tgctgacaag ctcactaaaa gtgcaagttc aaagatgcaa 1860
cacaaggata ccggcaccct aggaatatca agcactgaac aaggttcgag ctgggcagtg 1920
gagattggag gccatctgca agtgtgctca atcatggtgg agaatctgga caaagaagga 1980
gtgatgctta ttgagatgct atgcgaagaa tgtagccact ttctcgagat agcgaacgtg 2040
ataaggagct tggaactcat catcctcaga ggcaccactg agaaacaagg cgagaaaaca 2100
tggatatgtt ttgtagtgga gggacaaaac aacaaagtaa tgcacaggat ggacatcctg 2160
tggtctcttg tgcaaatatt tcaacccaag gctacaaaca gtctgcatct ttatcgacaa 2220
tctcaaattc tttacatgaa tgctttcgcc aatgtgcata gtcttcgggt accttctcac 2280
catcttcgag atttctcagc gtcactctct ctggctcctc caaatttaaa gaaaatcatc 2340
aagcaatgct cgacgcccaa gcttctggag tctgctttag ccgccatgat caagacgagc 2400
ctaaaccaag actgtcgctt aatgaaccaa ttcatcactg cctgcacttc ctttaaacgt 2460

cttgacctcg cagtttccac catgacccag atgcaggaac ctaatgtttt cgtctacaac 2520

gcgttgttta aaggcttcgt tacttgttct cacccgattc gatctctgga attgtatgtt 2580

cgtatgctca gggactcggt ttctccatca agctacacgt actcttcact agtaaaggcg 2640

tcttctttcg cttctaggtt tggggaatca ctccaggcgc acatctggaa atttggattt 2700

ggtttccatg ttaaaattca gacgactctt attgattttt attcagccac tggtagaatc 2760

agggaagcca ggaaagtgtt tgatgaaatg cctgaaagag atgatattgc ttggaccaca 2820

atggtttctg cttatcgtcg ggttttggat atggactctg cgaattcttt agctaaccaa 2880

atgtcggaga agaatgaggc tacgtcgaac tgtttgatta atggatatat gggattaggc 2940

aatctggaac aagcagagtc attgtttaat cagatgcctg tgaaggacat aatctcatgg 3000

accactatga tcaagggtta ctcgcagaat aaaagatata gagaagcaat tgcagtgttc 3060

tacaaaatga tggaggaggg catcattcct gatgaggtta ctatgtcaac tgttatttca 3120

gcttgtgccc atctcggcgt gctggaaata ggtaaggagg ttcatatgta cacgttacag 3180

aacggttttg ttcttgatgt ctacattggt tctgcactgg tagatatgta ttccaaatgt 3240

ggtagcttag agcgggcgct tctggtgttc ttcaatttgc ccaaaaagaa tctattttgt 3300

tggaattcga tcattgaagg actggcggct catggttttg cacaagaagc actgaaaatg 3360

tttgccaaga tggagatgga gtcggtgaaa cctaacgcag tcacttttgt gagtgttttt 3420

actgcgtgta ctcacgcagg tcttgttgac gaaggtcgga ggatatatcg cagcatgatt 3480

gatgactatt ccattgtctc taatgttgaa cattacggag gcatggttca tctattcagc 3540

aaagctgggt tgatctatga ggctcttgaa ttgattggaa atatggaatt tgaaccaaat 3600

gcggttatct gggggccctt gcttgatggg tgcagaattc acaagaatct cgtgatagct 3660

gaaatagcgt ttaacaaact gatggttttg gagccgatga atagtgggta ttatttcctt 3720

ttagttagca tgtatgcaga acaaaacagg tggagagatg ttgcagagat taggggaagg 3780

atgagagagt tgggtataga aaagatatgt cctgggacaa gttcgattcg gatagataaa 3840

cgagaccatc tgtttgctgc agctgataag tctcactcag cttcagatga ggtttgcttg 3900

ctgcttgatg agatatatga tcagatggga ttagctggat atgtgcagga aactgagaat 3960

gtatattaa 3969

<210> 46
<211> 1746
<212> DNA
<213> Arabidopsis thaliana

<400> 46
atgggattct tcgatcttag cattccgtac aatgagccgc cacgatcagg tggtaaggaa 60

atcgccggcg ggaaaacctt acgattaaag ctcgccacga aagccatgga gctaggctat 120

gttgggatcg cacataaccg ttcgatcaaa ggcgtaatgt ctgacaaaga ctcttgtacg 180

atccctcttc tcactcttgg gtctctaatc aaagtcgctc cgcggttagc ttcttctgtc 240

ggattccatc gcgatttact cggtgttccg cgaactactc cgtttcggca gtacacgcgt 300

ctcacagttc atgtggagag taatgctcag tgtcagagtt tgaattctgg gaatccgatt 360

ctaaagagtt atgatattat tgctgttagg ccgatgaatc agaacgcttt cgattatgcc 420

tgtgagaaag ctgaggttga tcttatttcg atagatttta cggacaagat gttattccga 480

ttgaagcatc ccatggttaa agctgctatt cagcgaggga tttactttga gattaagtac 540

tctgatatcc ttatggatgc acaaacgagg agacaagtta tatcaaatgc taagttactg 600

gtggattgga ctagggggaa gaatctaatt atatcaagtg gtgctccttc agtcacagaa 660

cttagaggtc caaatgatgt cataaatctc atgttcttac ttggactctc tgctgaaaga 720

gctagagctg ccatttcaaa aaattgtagg aatatgatag ccaaggtttt aaagaaaaaa 780

cggtttcaca aagaagctgt cagggttgaa ttactttctg ctggtgatac ttttagcctc 840

gaacagcctc tgtctgaaga ttgcatgaaa tgggatcgcc tttcgagcgg tgaaggtgac 900

atgcttttgg atgatcttgc aaaggctttt gatgccacaa atgttgtggc gcacaaatcc 960

tcgaaggcga ttgatttcac ctctgttctt gatggcttgc caaaacatgg tttccgggtt 1020

aaggatattg taggaactga atcagtgact cagccttctg cagctaaggt gattgacact 1080

caggtgcaca gtagtaatca agtttctgaa ctacgtatgg ccacagcttc atctgatgat 1140

aaccttcggg aaattgaaac cataagccaa attgacatgc tgatgtctga agatgacaat 1200

aaggtggaac ctactacaaa tgtcctcaaa gaagaagcat ttgccctaag gaaatgcagt 1260

gccagccatg gccaggggat tttggtgcaa aatcagacgg ctactccctt tacactgaca 1320

agatgtacaa agtcagaagc agcgtcggat gttagcatga atattgagtc gacttccgaa 1380

ggtggatcaa tgtcaccgtc aaaaagcgat catgggatcc cacaaagtcc tgttgaagtg 1440

aataacatgg gaaatgctgc ttttgaagaa gaagcctcag tggacgaaaa cagcaaagaa 1500

agagctacta ctggtcatgc tagtaatgat gagatgcata tcactgagtc tggacaccac 1560

gcatccattg atgatgagaa gcatatccct gagcctgaac acctcacatc cattgctgat 1620

gagatgaaaa ttgattgttc ttcggaagca aatcacgacg agtacatgga ggtcacaatg 1680

gaagaccaga tgcatgaaac agtccagatg cggttgtgca agaccatgac gaagcatcaa 1740

gactag 1746

<210> 47
<211> 612
<212> DNA
<213> Arabidopsis thaliana

<400> 47
atggcaaaag gtcgaaagcc gacgacaatg aaccggagcg atcgatacct tggaagctac 60

acttacggtg acagtcacgg aaactccgtt accgacgaat tagagctcgg tgaggaagac 120

atctggtcac cggccgtcat tcacgacgac accaccgaga atgaggaatc ctacggcacg 180

tggaacttac gcgctacctt gggaaaaaac gggcgcgtgg gaggattgtc gctggctttc 240

gagggctctt tggttgctcc gccgtcgtct tcgccgatga tagtgcagaa gattcacggc 300

ggaggaggtg agggagagga agaccggaga aaattggcgt cttcggcgcc ggtaaacgta 360

ccggactgga gtaagatata ccgagttgac tcggttgagt caatacacga gttagacgac 420

gaggatgacg aggatgagga atccgggatg atgccgccgc atgagtacct tgctaagagt 480

caagcacggc ggagtagaaa gatcggaggt ggtggtgcgt cggtgtttga cggcgtcgga 540

aggactctca aaggcagaga actaaggcgc gttcgtgacg cgatttggag ccaaacaggg 600

ttctacggct aa 612

<210> 48
<211> 531
<212> DNA
<213> Arabidopsis thaliana

<400> 48
atggatcaac aacaacaagg tgataagaat ctgacagtgt tcgtaggacc ctggggagga 60

aatggaggaa ccacttggga tgatgggatt tatgatggtg tccgtgagat cagacttgtt 120

tatgaccatt gcattgactc catctcggtg atctacgata agaatggtaa acccgcaaag 180

tcagagaagc atggaggtgt gggaggcaac aaaacatcag agataaagct gcaataccca 240

gaggagtatc tgactggcgt gagtggctac tactgtccaa tggttaacag tggcactcct 300

gtaatcagat caatgacctt caagagcaat aaacaagtgt atggacctta tggagttgaa 360

cagggaacac ccttcacttt ctcagtcaat gggggacgca ttgttggtat gaacggtagg 420

agtggctggt accttgactc catcggcttc catctatcac gccctaaatc aaccaagatg 480

atcaacaagc tccgaaagaa gattcactgg ctcacaagga tagtagcatg a 531

<210> 49
<211> 384
<212> DNA
<213> Arabidopsis thaliana

<400> 49
atgactaata taggaaaatg catgcaggga tatctcgacg aacaattcat ggagttagaa 60

gagctccaag atgatgcaaa ccctaatttt gttgaagaag tttccgcatt atacttcaaa 120

gattcagctc ggttaatcaa taacattgac caagctttgg aaagaggatc atttgatttc 180

aatcggctgg atagttacat gcatcagttt aagggaagca gcacgagcat tggggcaagt 240

aaagtgaaag ctgaatgcac tacgtttagg gaatactgca gagctggaaa tgcggaagga 300

tgcttgagga ctttccagca actgaagaaa gaacactcaa cgttgagaaa gaagcttgaa 360

cattatttcc aggcgagcca ataa 384

<210> 50
<211> 1575
<212> DNA
<213> Arabidopsis thaliana

<400> 50
atgcctctgt ttgagctttt caggctcacc aaagctaagc ttgaatctgc tcaagacagg 60

aacccttctc cacctgtaga tgaagttgtg gagctggtgt gggaaaatgg tcagatatca 120

actcaaagtc agtcaagtag atcgaggaac attcctccac cacaagcaaa ctcttctaga 180

gctagagaga ttggaaatgg ctcaaagacg actatggtgg acgagatccc tatgtcagtg 240

ccatcactaa tgacgggttt gagtcaagac gatgactttg ttccatggtt gaatcatcat 300

ccctcccttg atggatattg ctctgatttc ttgcgtgatg tgtcgtctcc tgttactgtc 360

aacgagcaag agagtgatat ggcggtaaac caaactgctt tcccgttgtt tcagagaaga 420

aaggatggca atgaatcagc tcctgctgct tcttcgtcgc agtataacgg tttccaatcg 480

cattctctgt atggaagtga tagagctaga gatcttccta gccaacaaac caatccggat 540

cggtttactc agacgcagga accactaatt actagtaaca agcctagttt ggtcaacttt 600

tcacatttct tacgccctgc aacttttgcg aagactacta ataataacct tcatgacact 660

aaagaaaaga gtcctcaaag cccgccaaat gtgtttcaga ccagagttct tggagctaaa 720

gactctgaag ataaggttct taacgagtct gttgcttctg ctacgcctaa agataaccaa 780

aaggcttgcc taatatcaga ggactcatgt agaaaagacc aagagagtga aaaagcagtt 840

gtatgttctt ctgttggctc gggtaatagt ctcgatggcc catccgaaag tccttcactt 900

tctttaaaga gaaagcattc gaatattcaa gacattgact gtcatagtga agatgtggaa 960

gaagaatcag gagatggaag aaaggaagca ggtccatctc gaacgggttt gggttcaaag 1020

agaagccgct ctgcagaagt gcataatctg tctgaaagga gacggcgtga taggatcaac 1080

gagaagatgc gtgccctgca agaactcatt ccaaactgta acaaggtgga caaagcttcg 1140

atgctagatg aagccatcga gtatctcaag tcactccaac ttcaagtgca gatcatgtca 1200

atggcgtctg gttactatct gccaccggcg gttatgttcc caccgggtat ggggcattac 1260

ccggcagcag ctgctgcaat ggcaatgggt atgggaatgc cttatgcaat gggcttgcct 1320

gatttgagcc gtggtggttc atcggttaac cacggaccac agttccaagt ctcggggatg 1380

caacaacaac cagtggcgat gggtattcca cgtgtctctg gtggtggtat ctttgccggt 1440

tcttcgacga ttggcaatgg ctcgactaga gatttatctg gttctaaaga tcaaacaacg 1500

acgaataaca acagtaactt gaaaccaata aagagaaaac aggggtcttc tgatcagttt 1560

tgtggatcgt cgtga 1575

<210> 51
<211> 657
<212> DNA
<213> Arabidopsis thaliana

<400> 51
atggagaatg attgcacggt gaatattgtc tctctggaga aggatcgcga tgtttcggag 60

gcgtcggctg aatctcagag cgagtcgact ctttcgaact cgctcgattc cggtgttacg 120

gctgagacct ctcgttctga tgctgattcc aaactggatg aatgtactgc ttggacgaat 180

gagaaacaca actcatatct tgattattta gagagctcgt ttgttaggca attatactcc 240

ttgcttggag gtgggactca gagactttct agaactcgtg atgtgcagtc taactctcat 300

aaatcagctg atcagtttac cgtcctacaa aatggttgct ggcagaaggt taactttgga 360

aagaaacaat cttgtttgga gacttcatct gagtttcgtt ttcacagaaa ttcattgaga 420

aataagcctg aaaattccaa cggaaattac accatgggaa ctactgtcca aggagatgtg 480

ttatgtcatg acgaaaccaa acactcagag gcgtcagggc agaatttcag agaagaagaa 540

gaagaagaag agaagggaga ggtgagcaaa aaacgagaaa gagaagcaaa taacgatgat 600

agttcattga aggaggatca ggttgtgccg gtaaggatgg tgaagcccag aacgtga 657

<210> 52
<211> 459
<212> DNA
<213> Arabidopsis thaliana

<400> 52
atggctgaaa aagtaaagtc tggtcaagtt tttaacctat tatgcatatt ctcgatcttt 60

ttcttcctct ttgtgttatc agtgaatgtt tcggctgatg tcgattctga gagagcggtg 120

ccatctgaag ataaaacgac gactgtttgg ctaactaaaa tcaaacggtc cggtaaaaat 180

tattgggcta aagttagaga gactttggat cgtggacagt cccacttctt tcctccgaac 240

acatatttta ccggaaagaa tgatgcgccg atgggagccg gtgaaaatat gaaagaggcg 300

gcgacgagga gctttgagca tagcaaagcg acggtggagg aagctgctag atcagcggca 360

gaagtggtga gtgatacggc ggaagctgtg aaagaaaagg tgaagaggag cgtttccggt 420

ggagtgacgc agccgtcgga gggatctgag gagctataa 459

<210> 53
<211> 1017
<212> DNA
<213> Arabidopsis thaliana

<400> 53
atggcggctc cgcatttcac acaactcaaa attacactaa accctctcat gtatcccttt 60

ctcgtcttat ctctactaac tctcgccctc ttctcattcg tctccgccat cttctttctc 120

ctcaaagctt cccgcagcag agctgctttg tacagccaga aactcttatc cgaatccgaa 180

accaaactcc aaccagaatc gtctctatcg gagatttccg acgaagccca gtaccaaacc 240

catgaaaatg aaccgaccca tttgacgaat tcgcgactct atgagttact gctctccgat 300

aagaaggagg atgattcgga ttgggaagga gatcatgtga aaaagaagaa gaagaagaag 360

aagaatcgag gtaagaagaa gaaatcagac ataagaggag atgaatccgg cggcgaaaag 420

cagctcggtg agggagaaga tgggcttgtt ttgaatccga ggacagactc gatttcgata 480

tcggaaaaca aaccggagtt tgtttgttta tatcctttta catcgacgag cagtgctacg 540

cagaggaaga ttaagcagca atacgatcag cttgttaaat gcaataatgc caaaggattg 600

acactagctc aggttgggga gtttgctaat tgtttgatag aagccaaaaa tgaactacaa 660

cacaagtcag aagtaatcaa gcgcaagttt tcaataacaa aagcccttct ctttaaggct 720

gatagatctt cctttgaccg acttcgtcaa cagatctata agctggagat ggaacaaaaa 780

agagtagaag aagatgcact tgtatataat tggctccagc aacagcttaa actctcacct 840

gcatacaaaa aggttcttga aataagcgct tccatggaac tcaaagacaa atcgagcaca 900

gagttagaca atccagatga tgaattttca gacatttcct tcgaagagct attggaacag 960

gaaaagaaag actcgttttg gtcagcattt ctctccatct ctcctcaagc ttattag 1017

<210> 54
<211> 1302
<212> DNA
<213> Arabidopsis thaliana

<400> 54
atgagtagtt cggagagagt accgtgcgat ttctgcggcg agcgtacggc ggttttgttt 60

tgtagagccg atacggcgaa gctgtgtttg ccttgtgatc agcaagttca cacggcgaat 120

ctgttgtcga ggaagcacgt gcgatctcag atctgcgata attgcggtaa cgagccagtc 180

tctgttcggt gtttcaccga taatctgatt ttgtgtcagg agtgtgattg ggatgttcac 240

ggaagttgtt cagtttccga tgctcatgtt cgatccgccg tggaaggttt ttccggttgt 300

ccatcggcgt tggagcttgc tgctttatgg ggacttgatt tggagcaagg gaggaaagat 360

gaagagaatc aagttccgat gatggcgatg atgatggata atttcgggat gcagttggat 420

tcttgggttt tgggatctaa tgaattgatt gttcccagcg atacgacgtt taagaagcgt 480

ggatcttgtg gatctagttg tgggaggtat aagcaggtat tgtgtaagca gcttgaggag 540

ttgcttaaga gtggtgttgt cggtggtgat ggcgatgatg gtgatcgtga ccgtgattgt 600

gaccgtgagg gtgcttgtga tggagatgga gatggagaag caggagaggg gcttatggtt 660

ccggagatgt cagagagatt gaaatggtca agagatgttg aggagatcaa tggtggcgga 720

ggaggaggag ttaaccagca gtggaatgct actactacta atcctagtgg tggccagagt 780

tctcagatat gggattttaa cttgggacag tcacggggac ctgaggatac gagtcgagtg 840

gaagctgcat atgtagggaa aggtgctgct tcttcattca caatcaacaa ttttgttgac 900

catatgaatg aaacttgttc cactaatgtg aaaggtgtca aagagattaa aaaggatgac 960

tacaagcgat caacttcagg ccaggtacaa ccaacaaaat ctgagagcaa caatcgtcca 1020

attacctttg gctctgagaa aggttcgaac tcctccagtg acttgcattt cacagagcat 1080

attgctggaa ctagttgtaa gaccacaaga ctagttgcaa ctaaggctga tctggagcgg 1140

ctggctcaga acagaggaga tgcaatgcag cgttacaagg aaaagaggaa gacacggaga 1200

tatgataaga ccataaggta tgaatcgagg aaggcaagag ctgacactag gttgcgtgtc 1260

agaggcagat ttgtgaaagc tagtgaagct ccttaccctt aa 1302

<210> 55
<211> 1929
<212> DNA
<213> Arabidopsis thaliana

<400> 55
atgcctaatt tctcagttaa cgttccccaa ctctcatctc tttacagtac aaaaacgccc 60

aaagtgagaa tgaatctatg tgccgatcag gtgttcgata aaaagcttct gtggagagat 120

atgtcaacga agatgaaatt tccttctttt tctgctgcgg aattacctga tttgaggaaa 180

agtaacaaga ggaggggatc tcttaggatg atcaagtgca gagccgccgg agctgacggt 240

ggacgcgtgg ctgttgggga tgatgtgttt tcggttacta cttcttctaa gtatgaagtt 300

gactatctgg gtcaaagtac taagggagat ttgaatctca agcttgaccc tcttcagtca 360

tttggagatg ggcaggctac attggagggt cccattgagg aggtagcgag aacagaggct 420

caagcggctg aaaatttgat tagagagttg ggtatccaag gccctttctc tgcacagcac 480

tctcctcggg gtatattttg tagtcgtaca ttgaatcttc ggtccattag tgcaattgga 540

tatgatatgg attacacttt gatgcactac aatgtcatgg cttgggaagg aaaggcttat 600

gactattgca tggaaaatct aaagagcatg ggtttccctg ttgatggact tgcttttgat 660

ccggaactgg ttatcagggg tctcatgatt gacaaagaga aaggtaattt agttaaggcc 720

gatagatttg ggtatgtgaa gagagccatg cacggtacaa agatgttatc aaataaagct 780

gtcagtgaga tctatggaag ggagttagtt gacctgcgga accagagtcg atgggagttt 840

ctcaatacat ttttttcagt ttcagaggct ctggcttatg cacagatggt tgatagattg 900

gatgatggat ttatttcggc agatcttggc actcttgatt ataaaggact gtataaggct 960

gttgcaaaag ctctcttcag agcacatgtt gaaggacaac ttaagagtga gataatgtcc 1020

aagccggaac tatttgtcga gccagaccca gaactacctt tagctctttt agatcaaaag 1080

gaggctggta agaagctctt gcttatcaca aactcggatt atcactacac agacaaaatg 1140

atgaagcatt catttaacaa attccttccc aatgacatgg actggcgaga tctttttgac 1200

atggtgatag tttctgcgag gaaaccagag ttcttccaga tgtcgcaccc tctatatgag 1260

gttgtgactg gagagggttt gatgcgtcca tgcttcaagg ctgaaacagg aggtttgtac 1320

tcaggaggaa gtgctcaaat gatagagagt tcactcaacg ttcatggaga tgagattttg 1380

tatgttggtg accacatcta cactgatgtc agcgtatcca aagtccatct caggtggcga 1440

actgcgctga tttgccgtga actggaagaa gagtatatgg ctctaattgg cagtcgtggt 1500

caccgagaag agctaataga gcttataaat caaaaagagg ttgttgggga tctctttaac 1560

caacttcggc ttgctcttca aagacgaagc aaaggccgtc ctgctcagac tctcgctgct 1620

accaacttgg atgatcaaga actgacagag accatgcaaa agcttcttat tgtaatgcaa 1680

agactagatg acaagattgg tctaatgctg gaaacagatg gcgagctctt taacaaaagg 1740

tggggcttcc tctcacgcgc gggtttgtgg gataaaagcc acttgatgag acaaatcgaa 1800

aagtatgcgg atatatacac atcaagagtc tccaacttcc tcaactacac acccttcatg 1860

tatttccgct cacaagagca gtcactggct cacgattctc cgcttccaga tgcgggtata 1920

gaaaactag 1929

<210> 56
<211> 321
<212> DNA
<213> Arabidopsis thaliana

<400> 56
atgtgggatg aaactgtagc cggacctaaa ccggagcatg gccttggccg cctccgcaat 60

aagatcacca cccaacccct tgacatcaaa ggagaaggga gcagtagtaa aactgtggcg 120

gcggtggccg ggagtcctgg aactccgacg acgccaggat cggcgcgtaa ggaaaacgtg 180

tggagaagtg tgtttcatcc aggaagtaac atcgccacta gaggaatggg cacaaacctc 240

ttcgacaagc cttctcaccc aaactctccc accgtctacg attggctata cagcgacgac 300

actaggagca agcaccgttg a 321

<210> 57
<211> 2106
<212> DNA
<213> Arabidopsis thaliana

<400> 57
atggagattc cactctcgcg ttaccagagc ataagattag acgagattcg agactcttct 60

tccaatccca aggttctcac tttcccgcga aaattctcgt tacgaggaag aagatggaag 120

aacccatttg gaagactcag ttgttcttct gtagttcaag gtctgaaacc aaaaccaaag 180

ctgaaaccag aaccaattag aatcgaggtt aaggaatcga aagatcagat tttggatgat 240

acccagatca gtaaatctgg tgtaacgatt tgtagtcaga tagagaagtt ggttttgtgt 300

aatagattca gagaagcttt tgaattgttt gagattctgg agattcgctg tagttttaag 360

gttggtgtta gtacttatga tgctttagtg gaagcttgta ttcgtttgaa atcgattcgg 420

tgtgttaaaa gggtttatgg gtttatgatg agtaatgggt ttgagccgga gcagtatatg 480

atgaacagaa tcttgttgat gcatgtcaag tgtgggatga ttattgatgc acgtaggttg 540

tttgatgaaa tccctgagag aaatttgtat tcttattact cgattatctc tgggtttgtt 600

aattttggga attatgttga agcttttgag ttgtttaaga tgatgtggga ggagctttct 660

gattgtgaga ctcatacgtt tgcggtgatg ctacgggcct cggctggctt agggtctatt 720

tatgtgggga aacagttaca cgtttgtgcg ttgaagttag gagttgttga taataccttt 780

gtctcgtgtg gattgattga tatgtatagc aagtgtgggg atattgaaga tgctcgatgt 840

gcttttgagt gtatgcccga gaaaactact gttgcttgga acaacgttat tgcgggttat 900

gcgcttcatg gttatagtga ggaagctctg tgtttgttgt atgacatgcg agactcaggt 960

gtgtctattg atcagttcac actttcgata atgataagaa tttctacaaa gcttgcaaag 1020

cttgagctta ctaagcaggc acacgctagt ttaattcgaa acggttttga atcggaaatc 1080

gttgcaaaca cagctcttgt agacttttat agcaaatggg gtagagtaga tactgctaga 1140

tatgtttttg ataaattgcc gagaaaaaat ataatctcat ggaacgcttt gatgggtgga 1200

tatgcaaatc atggtagagg aactgatgct gttaagttgt ttgagaaaat gattgcagca 1260

aacgtcgctc caaaccatgt cacatttctt gcagttctat cagcttgtgc ctattcaggt 1320

ttatctgagc aaggttggga gatttttcta tcgatgagtg aggttcatgg gatcaaacca 1380

agggcgatgc attatgcctg catgattgag ctgttgggta gagacggttt attagatgaa 1440

gccattgcgt ttatccgaag agctcctttg aaaaccacgg tgaacatgtg ggcagcactc 1500

ttgaatgcct gtaggatgca ggaaaactta gagcttggaa gagtggttgc tgaaaaactc 1560

tatggaatgg gacccgagaa gctcggaaac tacgttgtga tgtataatat gtacaacagt 1620

atgggaaaaa ctgcagaagc tgcaggggtt ttggagacat tggagagcaa aggattaagc 1680

atgatgccgg cttgtacttg ggttgaggtt ggagatcaga ctcacagctt tctttcagga 1740

gataggtttg attcttacaa tgagacggtg aaaaggcaga tataccaaaa agtggatgaa 1800

ctaatggaag agatttccga gtatgggtac tcagaggagg agcaacacct tcttccagac 1860

gtagatgaaa aggaagaaga gcgagtaggg cgatatcaca gcgagaaact agccatagct 1920

tacggattgg tgaatacgcc ggaatggaat ccattgcaga ttactcagaa ccataggata 1980

tgcaaaaatt gccacaaggt ggttgagttc atatctttgg ttacaggacg agagatggta 2040

gtgagagacg cgagccggtt ccatcatttt aaagaaggga agtgttcttg tggaggttat 2100

tggtga 2106

<210> 58
<211> 1500
<212> DNA
<213> Arabidopsis thaliana

<400> 58
atgaaaactt gtttgatctt cttcctctac acaacaattc tccaatacta tttccacttc 60

tctgtgtcat cattatcaac acctcttctc ctccatctct cccactctct ctcaacctca 120

aaacactctt catctcctct ccaccttctc aaatcatcct cctcccgttc ctccgcccgc 180

ttccgccgcc accaccacaa acaacaacaa caacaacttt cactccctat ctcctccggc 240

agcgattatc tcatctccct ctccgtcggc tcctcctcct cagccgtctc cttgtacttg 300

gacaccggaa gcgacctcgt ttggttccct tgccgtcctt tcacttgcat cctctgtgaa 360

tccaaaccac tccctccttc tcctccttca tctctctcct cctccgccac caccgtctcc 420

tgctcctccc cttcttgctc cgccgctcac tcttcccttc cctcctccga cctctgcgct 480

atctccaact gtcctcttga tttcatcgaa accggagatt gcaacacttc ttcttaccct 540

tgtcctcctt tctactacgc ttacggtgac ggctctctcg tcgcaaaact ctactccgac 600

tcactctctc tcccttccgt ctccgtctct aacttcacct tcggctgcgc tcacaccact 660

ctcgctgaac ctatcggcgt cgctggattc ggccgtggac gtctctctct tcccgctcag 720

ctcgctgttc actctcctca tctcggtaat agcttctctt attgtctcgt ctctcactct 780

tttgactcgg accgagtccg ccgtccgagt ccgctcatcc tcggtcgctt cgtcgataaa 840

aaagagaaac gtgtcggaac caccgatgat catgatgacg gtgatgatga gaagaagaag 900

aaaaatgagt tcgtcttcac agagatgctt gaaaacccaa agcatcccta cttctactct 960

gtttcactcc aaggaatctc aatcggaaaa cggaatattc cagctccggc gatgctcaga 1020

agaattgaca aaaacggtgg cggaggagtt gttgttgact cagggacaac gttcacgatg 1080

cttccggcga aattctacaa ttcggtggtt gaagaattcg atagtcgggt cgggcgggtt 1140

cacgaacggg ctgatcgggt cgaaccgagt tcgggtatga gtccttgtta ctatttaaac 1200

cagacggtta aagttccagc tctggttttg cattttgccg ggaacagatc cagtgtgacg 1260

ctccccagga gaaattattt ttacgaattt atggacggtg gagatggtaa agaagagaag 1320

aggaaaattg gatgtttgat gttgatgaac ggtggagatg aatcagaact tcgaggtggt 1380

actggggcta ttctggggaa ttaccagcaa caagggtttg aggtggttta tgatctgttg 1440

aacagaagag ttgggtttgc taagaggaag tgcgcatctt tatgggattc gcttaaataa 1500

<210> 59
<211> 1284
<212> DNA
<213> Arabidopsis thaliana

<400> 59
atggcattaa cccttttgtc tcacgaatta tctgacctct gtatcggtaa gccaccttta 60

cggtgtctct ccgtcgccac agccaccgta gctgacgcca tcgccgctct caaatcctct 120

gacgaaccgt tcctcaccgt atggagctgt aatcacgatg agaaaacaga tgataatgat 180

aagtgtgagt gtttgggtaa gatctgtatg gctgatgtaa tctgttacct atccaaattc 240

gacaacaatg ttttgtctct ttcctctgct ttcgacgcat ctgtctctgt tcttcttccc 300

aaatctcgtg ccctcgtcgt ccatgttcaa tcttcttgca gtttgattga agctattgat 360

ctgataatca aaggagcaca gaatctgatt gttccgattc atacgaaatc aatcacaaag 420

agaagacaac aacaaaaact tctgaaacga aacgtcgtcg tttcactcac caacgcaact 480

tcaacaaccc acaaaaacag ccgagaattc tgctggatca cacaagaaga cattattcga 540

ttccttctcg attccattag cgttttctct ccattaccgt cgctttctat ctccgatctt 600

ggagttatca atagtacaca cactatcctc gccgttgatt actactcctc agctgcttcc 660

gccgtctctg ccatctctcg tgccatcttg gacaatgtct ctgtcgcggt ggttggtaaa 720

ggatgtgatc aagaagatcc atgtatggtt ttgataggcg agatttcacc gatgacactc 780

gcttgctgcg atgaaactgc cgtagcagcg gttgctacac tctctgccgg agatttaatg 840

tcctatatcg acggtagtgg tccgccggag agtctagttg gagtagttag gaatcgtttg 900

gaagataaag ggatggttgg attaatctca ctcattgatt ctttgtcgtt gtcgtcgggg 960

tcttcctcgg atgaagaatc tccggcgggg aagacgagaa tgacttcttc gtatgggagg 1020

tcggtgagta gcgcggcgag gatggctagg aaatcggtgg cgatagtgtg taatcggaag 1080

agttctttaa tggcggtgat gatacaagct attgctcata gagtgagtta tgtgtgggtg 1140

attgatgaag atggttgttt gattggttgt acaatcggaa tcagtaatta tgtaaatagg 1200

ttagttagac cacgtatttt ggctgcaata tgggtttcac gtctaaacct aaagaaagca 1260

acacttgatg agtctcacgt ctaa 1284

<210> 60
<211> 1098
<212> DNA
<213> Arabidopsis thaliana

<400> 60
atggcatctg cattttgctc actttgtccc actcccacct ccttattctc ttcccacgcg 60

cttataccca ctctacagtg gcgttcgagt tcgagttcga ggtctcctcc gctacatatt 120

tcccgcgttt tatcagttga aactgttcct ttaagcccat cattcacctg gaacgatgtt 180

tttgagaaca gtcgaaaaga atacgtgcct cagaactcct ccgatctcac cggatttctc 240

gagaaagtcg accgctgtaa tcgtggatta gagaagttag gtgagttcat tccatttgtt 300

atagaggaac aaatcgttgg ttatattcac aagggattta caaagtactt gagggacttt 360

aatgatatct ttacattttc acaatatggt ggccatgtaa cgcttaacat gatgcttgac 420

aagcctgaag aaagaaccag agcagttgca catgtgatca aaatattggg taacaaagga 480

atcatccctg ggatacgaaa tgagctatat cctgtgaagc catcgtttaa tgctcctgcc 540

tttttttcta tagagcgtgc tgctgctcct tattttggat tgaagggtta cgcaattcat 600

gtgaatgggt atgtagaaag agatggacaa aaatttctat ggataggtaa aagaagtcta 660

gcaaaatcca cttatccagg aaaacttgat catctggttg ctggaggatt gcctcacggg 720

attagcgttt gtgagaatct agtaaaggaa tgcgaagagg aagctgggat ttccaaagtc 780

ttggctgata gggcgattgc ggtcggtgtt gtttcctaca tggatatcga tcggtactgt 840

ttcacacgtg atgtgctgtt ttgttatgat ttggaactcc ctcaagattt tgttcccaca 900

aatcaagatg gagaagttga cagcttcagg ttgattccag tcgctcaagt tgctaatgtg 960

gttcggaaga ctagtttttt taaggacagt tgttccctgg tcattattga cttcttgttt 1020

cggcacgggt taatcagacc agagtcaccg ggttacttgg atctataccg acgcctgagg 1080

aatggagatt gctcataa 1098

<210> 61
<211> 2268
<212> DNA
<213> Arabidopsis thaliana

<400> 61
atggaaatct acaccatgaa aacgaatttt cttgtactgg ctttgtcttt gtgtatcctt 60

ctttcaagct tccatgaggt ttcttgtcag gatgatggta gtggtttgag taatttggat 120

ctaatagaac gtgattatca agatagtgtc aatgctcttc aaggcaagga cgatgaagat 180

cagtctgcaa agatacagag tgaaaaccag aataacacta cagtgactga taagaacact 240

atttctctat ctctatcaga tgaatctgag gttggatctg ttagtgatga aagcgttgga 300

cgttcgagtc tgttggatca aatcaaactt gaattcgaag ctcatcacaa tagtattaac 360

caagctggat ctgatggtgt caaggctgaa tccaaggatg atgatgaaga attatctgct 420

catagacaga aaatgttgga agaaatcgaa catgagtttg aagctgcttc agatagtctg 480

aaacaactaa agactgatga tgtaaacgaa ggaaatgatg aagaacattc tgcaaagagg 540

caaagtttgt tggaagagat cgaacgtgag tttgaagctg ctacaaaaga acttgaacaa 600

ctaaaggtta atgacttcac cggggacaaa gatgacgaag aacactctgc aaagagaaaa 660

agtatgcttg aagctattga acgcgagttt gaagctgcta tggaaggcat tgaagcactt 720

aaggtttctg attccacagg aagcggagat gatgaagaac aatctgcaaa gagactaagt 780

atgcttgaag agatcgaacg ggaatttgaa ggtcttgaac aactaagggc tagcgattca 840

accgcggaca ataacgaaga agaacacgct gcaaagggac aaagtttgtt agaagagatc 900

gaacgagagt tcgaagctgc tacagagagc cttaagcaac ttcaagttga tgattctact 960

gaagacaaag aacactttac agctgcaaag aggcaaagtc tgctggaaga gattgaacgt 1020

gaatttgaag ctgcaacaaa agatcttaaa caactaaatg atttcactga aggcagtgct 1080

gatgatgaac aatctgcaaa gagaaacaaa atgttggaag atatcgaacg cgaatttgaa 1140

gctgctacaa taggtcttga acaactaaag gctaatgatt tctctgaagg caataataat 1200

gaagaacaat ctgcaaagag aaagagtatg cttgaagaga tcgaacgcga gttcgaagct 1260

gctattggag gtcttaaaca gatcaaagtt gatgattcca gaaatcttga agaagaatct 1320

gctaagagaa agataatttt ggaagagatg gaacgtgaat ttgaagaagc acacagtggt 1380

attaatgcaa aggctgacaa agaagaatct gcaaagaaac agagtggctc tgctatacca 1440

gaggttcttg gactaggaca gtcaggtggt tgtagctgtt ctaaacaaga cgaagattcc 1500

tcgattgtta taccaacaaa atatagcata gaagatatcc tctctgaaga atctgcagtc 1560

cagggaacag agacttctag tctcaccgcg tctttgactc aactcgttga gaatcacagg 1620

aaagaaaagg aatctctact cggacacaga gttctcactt ctccttctat agcttcttcc 1680

acaagcgaat catctgctac atcagagact gtagaaaccc taagggctaa actgaatgag 1740

cttcgcggct taaccgctcg tgagcttgtg acacgtaaag atttcggtca gattctcatt 1800

acggctgcga gttttgaaga gctaagttca gctccaatca gttacatttc taggttagct 1860

aaatacagaa acgtcatcaa agaaggactt gaagcttctg agagagttca catcgcgcag 1920

gtacgagcaa aaatgctcaa agaagttgcc acggagaagc aaaccgccgt ggacactcat 1980

ttcgcaaccg ctaaaaagct tgctcaagaa ggagacgcgt tgttcgttaa aatcttcgca 2040

atcaagaaac tgttggcgaa acttgaagca gagaaagaat ctgttgatgg aaagtttaag 2100

gagactgtga aagaactttc tcatcttctg gctgatgctt ctgaggctta cgaagagtat 2160

catggcgcgg tgaggaaggc gaaagacgag caagcggctg aggaatttgc gaaagaggcg 2220

acgcaaagtg cagagatcat ttgggttaag tttcttagtt ctctttag 2268

<210> 62
<211> 501
<212> DNA
<213> Arabidopsis thaliana

<400> 62
atggcagcgc gtgcccatga tgttgcggca ttgagtatca aaggaagttc cgcaatcctt 60

aacttccctg agctcgcgga ttttctgcca agaccagtct cgctcagcca acaggatatc 120

caggccgcag ccgccgaagc cgctcttatg gatttcaaaa ctgtaccatt ccatcttcag 180

gatgactcaa cgccgttgca aactaggtgt gatactgaga agatcgaaaa gtggtcatcc 240

tcatcgtcct cagcctcatc ctcatcctca tcttcgtcct cgtcctcatc atctatgctt 300

tcgggggagc taggagatat tgtggagttg ccgagtcttg aaaacaatgt aaaatacgat 360

tgtgcgctgt atgactcgtt ggaggggctg gtgtcgatgc ccccatggtt agatgctacc 420

gaaaatgatt ttaggtatgg agatgattcg gtactgttgg acccatgtct caaagaaagc 480

tttttgtgga attatgagta a 501

<210> 63
<211> 465
<212> DNA
<213> Arabidopsis thaliana

<400> 63
atgtcgctag acgcgaatac ttgttctatc gtcttctttc tattcttcac tgtctctttc 60

gccgtctccg gccaaaagcc aaccgcttac gacgccgtca aactctataa cttaccacca 120

ggaatcctcc caaaaggagt ggttgactat gagcttaacc caaaaacagg caacttcaaa 180

gtctacttca atgacacgtg tgaattcacc atccaatcct accagctcaa gtacaaatca 240

actatctccg gcgttatatc acccggtcat gtcaagaatc tgaaaggagt tagtgttaag 300

gttcttttct tctgggttaa tattgctgaa gtgtctcttg acggcgccga tctcgacttc 360

tccgtcggaa tcgcgtcggc gagttttccg gctgctaatt ttgaagagag tcctcagtgt 420

ggttgtgggt ttgattgtaa taatgggctt ttattttctt cttga 465

<210> 64
<211> 891
<212> DNA
<213> Arabidopsis thaliana

<400> 64
atggctcaaa aggttgaagc aaaaggaggg aaaggaggca accaatggga cgatggatcc 60

gatcatgacg ccgtgaccaa gattcaggtt gcagtaggcg gaatgggaat tcaatacatt 120

cagttcgatt acgtcaagaa cggacaaacc gaacaaactc ctcttcgtgg tatcaaaggc 180

agtaccattc caactgatcc gtttgtgatt aaccatcctg aggagcatct agtttctatt 240

gaaatttggt ataaacctga tggtcttatt caagggctta ggttcatatc caacaaaaag 300

acttctcgtt tcattggata cgatcgtggt actagatcat ttctccaagt tcaagacaag 360

aagatcattg gctttcatgg gtctgccgga gacaatctta attctcttgg agcttacttt 420

gctccgttga ctatcccatt gactcctgcc aagccgctac cggcacttgg tagtgatgat 480

ggaacagcat gggatgatgg tgcttacgtt ggggttaaga aggtgtacgt aggacaagcc 540

caagatggta tatcggctgt taagtttgta tacgacaaaa gccctgagga ggtcacagga 600

gaagaacatg gaaagagtac tctactcgga ttcgaagagt tcgtacttga ctatccaagt 660

gaatacatca tcgcagtcga aggcacctac gataaaatct ttgggagtga tggctcagtc 720

ataactatgc ttaggttcaa gactaataag caaacgtccc ctccctttgg acttgaagct 780

ggcactgcct tcgaactcaa agaggaaggc cacaaaatcg ttggcttcca tggaagagcc 840

gatgctttgc tccacaaaat tggagttcat gtccgtcccg tttccaactg a 891

<210> 65
<211> 1071
<212> DNA
<213> Arabidopsis thaliana

<400>  65
atgtcttcac ttgcagattt aatcaatctc gatctctccg attccactga ccagatcatc      60

gccgagtaca tatggattgg tggatcgggc ttggatatga gaagcaaagc aaggactttg     120

cctggaccag tgacggatcc atcgcagtta ccgaaatgga actacgacgg ttcaagcacc     180

ggccaagctc cgggcgatga cagtgaagtc atcatctacc ctcaagctat cttcaaagac     240

cccttcagaa gaggcaacaa catccttgtg atgtgtgacg catatacacc ggcaggagag     300

ccgattccga cgaacaaaag gcatgcggcg gctaagatct ttgaagaccc tagtgttgtc     360

gccgaagaaa catggtacgg aattgaacaa gagtatacct tgttgcaaaa ggatattaag     420

tggccggtag gttggccggt cggcggtttc ccaggtcctc agggaccgta ctactgtgga     480

gttggagcag acaaagcctt tggaagagac atcgttgatt ctcattacaa agcttgtctt     540

tacgccggaa tcaatgtcag tgggactaac ggcgaagtta tgcctggcca gtgggagttc     600

caagtcggtc ccaccgttgg aatcgctgcc gccgatcagg tctgggttgc tcgttacatt     660

cttgagagga tcacagaatt ggctggagtt gttctgtctc tagaccctaa accaattccg     720

ggagattgga atggtgcagg ggcacacaca aattacagta cgaagtcgat gagagaagat     780

ggagggtacg aggtgataaa gaaagcaata gagaagcttg gattgcgtca caaggaacac     840

attgctgctt atggtgaagg caacgagcgt cgtctcaccg gaaaacatga aaccgccgat     900

atcaacactt tcttatgggg tgtggcaaac cgtggggcat cgattagggt tgggcgtgac     960

actgagcagg ctggaaaagg atactttgaa gatcgtaggc cagcttcgaa catggatcct    1020

tacactgtga cctccatgat tgctgaatcc acaatccttt ggaaaccatg a             1071


<210>  66
<211>  990
<212>  DNA
<213>  Arabidopsis thaliana

<400>  66
atgactgatt ctgcttacag agtagacacc atttctagac tcgcccaatg gcgaatccac      60

aatctttctt cctccactta ccgcaagtcc gatcctttca agatgggtct ttggaattgg     120

cacttgtctg tggagaaaag caagatgcta ttaaatgtta agttgtatcc agaggtatca     180

aaccttacca gagaaaatcc accggttgct tcctttgctc ttcgtgttgt ctcttctact     240

ggtgagagaa aggctctatc tcatccagaa gtaatagata agcggattaa gacaaacgaa     300

gattttattt ggactattga agttccctta actgggaaaa tcatcatcga cgtcgagttt     360

cttgacttga aggttttgtc tcaagatagt ggagaacttt actctatctg ggccaacggt     420

tcaactgaga atcaatcgca agtaactgcg gtaacatccc ttggacgtat gttgacagaa     480

agcatttaca ccgacataac gatcaatgcc tctgatggaa gcattggagc tcaccgagca     540

gttctcgctg cccgttcacc tgttttccgc agcatgtttt tacacgacct gaaagagaaa     600

gaactatcag aaataaacgt actcgacatg ccacttgatg cttgccaagc ttttctcagt     660

tatatctacg gcaatatcca aaacgaagac tttcttatac acagattggc actcctccaa     720

gcagctgaga aatatgatat tgctgattta aaagaggcgt gccacttgag tcttctagac 780

gatatcgaca caaagaatgt gcttgagagg ctacagaatg cttatctcta tcaattacct 840

gaactgaagg ctagctgcat gagatatctt gtgaagtttg gcaaaatatt tgagatccga 900

gacgagttca acatattcat gcaatgcgca gacagagatt tgatttctga aatcttccac 960

gaagtcctca gtacctggaa aggattttag 990

<210> 67
<211> 1113
<212> DNA
<213> Arabidopsis thaliana

<400> 67
atgtcggcga tcatgaaaag tctctgtttc tccttcctta tcctcgcttc atttgcaact 60

ttcttctccg ttgctgatgc atggaggttt aacgtcggag gtaacggcgc ttgggttaca 120

aatcctcaag aaaactacaa tacttgggct gaaagaaacc gtttccaagt caatgactct 180

ctctatttta agtacgcgaa gggatcagac tctgttcaac aagtgatgaa agcagatttt 240

gatggatgca acgttagaaa tccgatcaag aacttcgaaa atggtgaatc tgtggttact 300

cttgatcgat ctggtgcttt ttatttcata agcggtaatc aagatcactg tcaaaaggga 360

cagaaattga tcgtcgttgt cctcgccgtc agaaatcaac cttcggctcc ggctcattcc 420

cctgttcctt cagtttctcc tactcaacct cctaaatctc attcccctgt ttctcccgtt 480

gctccagcgt ctgctccttc aaaatctcag ccacctagat cctctgtttc tccagcacaa 540

ccacctaaat cttcttcccc tatttctcac acaccagctc tctcaccgtc gcatgctaca 600

tctcactctc cagctactcc atctccgtca ccaaaatctc cctcccctgt ttctcactca 660

ccatctcact ctccggcaca taccccatct cactctccgg cacatacccc atctcactct 720

ccggcacatg ccccatctca ctccccggcg catgctccat ctcactctcc ggcgcatgct 780

ccatctcact ccccggcgca ttctccatct cactcaccgg cgactccaaa atccccatct 840

ccttcttctt ctccagctca gtctccggcc actccatctc cgatgacacc acaatcccca 900

tcccctgttt cttccccatc acctgatcag tctgctgctc cctctgacca gtctacaccc 960

ttggcacctt ctccttccga aacaactccg accgccgata acatcactgc gccggctcct 1020

agtcccagga caaactcagc aagtggttta gccgttactt cggttatgtc tacactattt 1080

agtgcgactt ttacctttct gatgtttgct taa 1113

<210> 68
<211> 588
<212> DNA
<213> Arabidopsis thaliana

<400> 68
atggctttga agacagtttt cgtagctttt atgattctcc ttgccatcta ttcgcaaacg 60

acgtttgggg acgatgtgaa gtgcgagaat ctggatgaaa acacgtgtgc cttcgcggtc 120

tcgtccactg gaaaacgttg cgttttggag aagagcatga agaggagcgg gatcgaggtg 180

tacacatgtc gatcatcgga gatagaagct aacaaggtca caaacattat tgaatcggac 240

gagtgcatta aagcgtgtgg tctagaccgg aaagctttag gtatatcttc ggacgcattg 300

ttggaatctc agttcacaca taaactctgc tcggttaaat gcttaaacca atgtcctaac 360

gtagtcgatc tctacttcaa ccttgctgct ggtgaaggag tgtatttacc aaagctatgt 420

gaatcacaag aagggaagtc aagaagagca atgtcggaaa ttaggagctc gggaattgca 480

atggacactc ttgcaccggt tggaccagtc atgttgggcg agatagcacc tgagccggct 540

acttcaatgg acaacatgcc ttacgtgccg gcaccttcac cgtattaa 588

<210> 69
<211> 2006
<212> DNA
<213> Arabidopsis thaliana

<400> 69
atgtcaaagg aagagccttg tgttacaagc aagactggat cgctggtcta cgttctggtt 60

tcggaggttt tacaggattt agcaccgaca acatatgttt tcttcgcctc tgcgcttcct 120

gttattgcct ttggcgagca acttagccac gacacagaga gatcgttgag cacagtggaa 180

acgttagcat caacagcgtt atgtggagtg atacactcgt tattgggagg acaaccattg 240

ttgatacttg gagttgcaga accaactgtc ttaatgtaca aatacttgta cgacttcgct 300

aaaggaagac ctgaattggg caaacaactc tacttagctt gggttgcttg ggtttgtgtg 360

tggacggctt tgttactatt cctaatggcg atattcaaca tggcttatat catcaaccgg 420

ttcacgagga tcgctggtga gctgtttggt atgttgatcg ctgttctatt tctccaacaa 480

accataaagg gaatggtgag tgaatttagg attccaaaag gtgaagactc aaaacttgaa 540

aagtatcagt ttgagtggct ctacacaaac ggacttcttg gccttatttt cacagtcggt 600

cttgtctaca ccgctttgaa gagcagaaaa gcaaggtctt ggccatacgg aacaggatgt 660

tgccgaagct tcgttgcaga ctacggagtt ccgttgatgg ttgtggtttg gacagcattg 720

tctttcagta cgccatcaaa actaccctct ggtgtcccga gaagactcgt tagtcctctt 780

ccatgggact ctgtttcttt aacacattgg actgtcatca aggacatggg taaagtctct 840

cccggttaca tatttgcagc gtttataccc gcattgatga tcgcaggcct ctacttcttt 900

gaccacagcg ttgtctcgca gctcgcgcag cagaaggagt ttaacctcaa gaacccttct 960

gcatatcact acgacattct cttgttaggt ttcatggtat tgatctgtgg aatgctcggt 1020

ctaccgcctt ccaacggagt cctcccgcag tctcctatgc ataccaaaag cctagctgtt 1080

ttcaaacgac agttaatgcg gaggaagatg gtgatgacag ccaaagaaag catcagacag 1140

aaagcaacgt cctctcaagt gtacgaggat atggaacaag tcttcataga aatggacaaa 1200

agcccacttg ctgagacaca cacaacactg ataaatgagc tgcaagatct gaaagaggca 1260

gtgatgaaga agagtgacga cgacggggat accggcgaag agagtggttt cgatccagag 1320

aagcacgttg acgcttactt gcctgttcga gtcaacgagc agagagtgag caacctgttg 1380

caatcattgc tagtgatagg tgcagtgttt gctctaccgg tcattaagct cataccgact 1440

tcacttctat ggggatattt tgcttacatg gccattgata gcctcccaga caatcaattc 1500

ttcgaacgaa cagtacttct cttcgtccca ccaacccgga gattcaaggt cttggaagga 1560
gcgcatgcat cgttcgtgga gaaagttccg cataagtcaa tcgctgcatt cacgctattt 1620
cagatactct actttgggct ttgctacgga gtgacgtgga ttccagtggc cggaatcatg 1680
tttccggttc ttttcttcct tttagtagcc atcagacagt accttctccc taagctcttt 1740
aaaccagcct atctccggga actcgatgcg gcgggtatga ggagatccct ggaactccta 1800
gaaacccgct tgaactgtct ttcaggtcga ataactcggc gagaggggtc caagagtgtg 1860
atgctgagat tctagacgag ttaacaacga gcagaggcga gctcaaagtc cgtacactcg 1920
gtcataacga agacaaaggc caccagatat atcccaagga gatagtagaa gtaggggatg 1980
gggacatgag ttcttcgaga gagtga 2006